# EUROPEAN PATENT APPLICATION

(11) **EP 2 511 377 A1**
(43) Date of publication of application: **17.10.2012**
(21) Application number: 11003157.2
(22) Date of filing: 14.04.2011
(51) Int. Cl.: C12P 7/40, C12N 9/88, C12N 9/90, C12N 9/10, C12N 9/02, C12N 9/00, C12N 9/06

(54) **Means and methods for producing crotonic acid**

(71) Applicant: B.R.A.I.N. Biotechnology Research And Information Network AG, 64673 Zwingenberg (DE)
(72) Inventor: Koch, Daniel, 64342 Seeheim-Jugenheim (DE); Meurer, Guido, 64342 Seeheim-Jugenheim (DE)
(74) Representative: Vossius & Partner

(57) **Abstract**

This invention relates to a recombinant host cell with elevated expression or activity of the following enzymes as compared to the parent host cell it is derived from: (a) 4-hydroxybutyryl-CoA dehydratase; and (b) (ba) crotonate CoA-transferase; and/or (bb) at least one, preferably all of the enzymes of at least one of the following groups (i) to (iv): (i) 2-ketoacid decarboxylase; (ii) 2-ketoglutarate dehydrogenase, succinyl-CoA synthetase and succinic semialdehyde dehydrogenase; (iii) 2-ketoglutarate dehydrogenase and succinyl-CoA reductase; and (iv) glutamate dehydrogenase, glutamate decarboxylase and γ-amino-N-butyrate transaminase. Provided is also a method for producing a compound selected from the group of crotonic acid, crotonyl-CoA, butyryl-CoA, butyric acid, butyraldehyde and butanol. Furthermore, disclosed are kits suitable for practising the methods of the invention as well as for generating the host cell according to the invention.

SEQUENCE LISTING

<110> B.R.A.I.N. AG

<120> Means and methods for producing crotonic acid, butanol and
related compounds

<130> S2973 EP

<160> 58

<170> PatentIn version 3.5

<210> 1
<211> 2802
<212> DNA
<213> Escherichia coli

<400> 1
atgcagaaca gcgctttgaa agcctggttg gactcttctt acctctctgg cgcaaaccag 60

agctggatag aacagctcta tgaagacttc ttaaccgatc ctgactcggt tgacgctaac 120

tggcgttcga cgttccagca gttacctggt acgggagtca aaccggatca attccactct 180

caaacgcgtg aatatttccg ccgcctggcg aaagacgctt cacgttactc ttcaacgatc 240

tccgaccctg acaccaatgt gaagcaggtt aaagtcctgc agctcattaa cgcataccgc 300

ttccgtggtc accagcatgc gaatctcgat ccgctgggac tgtggcagca agataaagtg 360

gccgatctgg atccgtcttt ccacgatctg accgaagcag acttccagga gaccttcaac 420

gtcggttcat ttgccagcgg caaagaaacc atgaaactcg gcgagctgct ggaagccctc 480

aagcaaacct actgcggccc gattggtgcc gagtatatgc acattaccag caccgaagaa 540

aaacgctgga tccaacagcg tatcgagtct ggtcgcgcga ctttcaatag cgaagagaaa 600

aaacgcttct taagcgaact gaccgccgct gaaggtcttg aacgttacct cggcgcaaaa 660

ttccctggcg caaaacgctt ctcgctggaa ggcggtgacg cgttaatccc gatgcttaaa 720

gagatgatcc gccacgctgg caacagcggc acccgcgaag tggttctcgg gatggcgcac 780

cgtggtcgtc tgaacgtgct ggtgaacgtg ctgggtaaaa aaccgcaaga cttgttcgac 840

gagttcgccg gtaaacataa agaacacctc ggcacgggtg acgtgaaata ccacatgggc 900

ttctcgtctg acttccagac cgatggcggc ctggtgcacc tggcgctggc gtttaacccg 960

tctcaccttg agattgtaag cccggtagtt atcggttctg ttcgtgcccg tctggacaga 1020

cttgatgagc cgagcagcaa caaagtgctg ccaatcacca tccacggtga cgccgcagtg 1080

accgggcagg gcgtggttca ggaaaccctg aacatgtcga aagcgcgtgg ttatgaagtt 1140

ggcggtacgg tacgtatcgt tatcaacaac caggttggtt tcaccacctc taatccgctg 1200

gatgcccgtt ctacgccgta ctgtactgat atcggtaaga tggttcaggc cccgattttc 1260

cacgttaacg cggacgatcc ggaagccgtt gcctttgtga cccgtctggc gctcgatttc 1320

cgtaacacct ttaaacgtga tgtcttcatc gacctggtgt gctaccgccg tcacggccac 1380

aacgaagccg acgagccgag cgcaacccag ccgctgatgt atcagaaaat caaaaaacat 1440

ccgacaccgc gcaaaatcta cgctgacaag ctggagcagg aaaaagtggc gacgctggaa 1500

gatgccaccg agatggttaa cctgtaccgc gatgcgctgg atgctggcga ttgcgtagtg 1560
gcagagtggc gtccgatgaa catgcactct ttcacctggt cgccgtacct caaccacgaa 1620
tgggacgaag agtacccgaa caaagttgag atgaagcgcc tgcaggagct ggcgaaacgc 1680
atcagcacgg tgccggaagc agttgaaatg cagtctcgcg ttgccaagat ttatggcgat 1740
cgccaggcga tggctgccgg tgagaaactg ttcgactggg gcggtgcgga aaacctcgct 1800
tacgccacgc tggttgatga aggcattccg gttcgcctgt cgggtgaaga ctccggtcgc 1860
ggtaccttct tccaccgcca cgcggtgatc cacaaccagt ctaacggttc cacttacacg 1920
ccgctgcaac atatccataa cgggcagggc gcgttccgtg tctgggactc cgtactgtct 1980
gaagaagcag tgctggcgtt tgaatatggt tatgccaccg cagaaccacg cactctgacc 2040
atctgggaag cgcagttcgg tgacttcgcc aacggtgcgc aggtggttat cgaccagttc 2100
atctcctctg gcgaacagaa atggggccgg atgtgtggtc tggtgatgtt gctgccgcac 2160
ggttacgaag ggcaggggcc ggagcactcc tccgcgcgtc tggaacgtta tctgcaactt 2220
tgtgctgagc aaaacatgca ggtttgcgta ccgtctaccc cggcacaggt ttaccacatg 2280
ctgcgtcgtc aggcgctgcg cgggatgcgt cgtccgctgg tcgtgatgtc gccgaaatcc 2340
ctgctgcgtc atccgctggc ggtttccagc ctcgaagaac tggcgaacgg caccttcctg 2400
ccagccatcg gtgaaatcga cgagcttgat ccgaagggcg tgaagcgcgt agtgatgtgt 2460
tctggtaagg tttattacga cctgctggaa cagcgtcgta agaacaatca acacgatgtc 2520
gccattgtgc gtatcgagca actctacccg ttcccgcata aagcgatgca ggaagtgttg 2580
cagcagtttg ctcacgtcaa ggattttgtc tggtgccagg aagagccgct caaccagggc 2640
gcatggtact gcagccagca tcatttccgt gaagtgattc cgtttggggc ttctctgcgt 2700
tatgcaggcc gcccggcctc cgcctctccg gcggtagggt atatgtccgt tcaccagaaa 2760
cagcaacaag atctggttaa tgacgcgctg aacgtcgaat aa 2802

<210> 2
<211> 933
<212> PRT
<213> Escherichia coli

<400> 2

Met Gln Asn Ser Ala Leu Lys Ala Trp Leu Asp Ser Ser Tyr Leu Ser
1               5                   10                  15

Gly Ala Asn Gln Ser Trp Ile Glu Gln Leu Tyr Glu Asp Phe Leu Thr
            20                  25                  30

Asp Pro Asp Ser Val Asp Ala Asn Trp Arg Ser Thr Phe Gln Gln Leu
        35                  40                  45

Pro Gly Thr Gly Val Lys Pro Asp Gln Phe His Ser Gln Thr Arg Glu
    50                  55                  60

Tyr Phe Arg Arg Leu Ala Lys Asp Ala Ser Arg Tyr Ser Ser Thr Ile
65                  70                  75                  80

Ser Asp Pro Asp Thr Asn Val Lys Gln Val Lys Val Leu Gln Leu Ile
                85                  90                  95

Asn Ala Tyr Arg Phe Arg Gly His Gln His Ala Asn Leu Asp Pro Leu
            100                 105                 110

Gly Leu Trp Gln Gln Asp Lys Val Ala Asp Leu Asp Pro Ser Phe His
        115                 120                 125

Asp Leu Thr Glu Ala Asp Phe Gln Glu Thr Phe Asn Val Gly Ser Phe
    130                 135                 140

Ala Ser Gly Lys Glu Thr Met Lys Leu Gly Glu Leu Leu Glu Ala Leu
145                 150                 155                 160

Lys Gln Thr Tyr Cys Gly Pro Ile Gly Ala Glu Tyr Met His Ile Thr
                165                 170                 175

Ser Thr Glu Glu Lys Arg Trp Ile Gln Gln Arg Ile Glu Ser Gly Arg
            180                 185                 190

Ala Thr Phe Asn Ser Glu Glu Lys Lys Arg Phe Leu Ser Glu Leu Thr
        195                 200                 205

Ala Ala Glu Gly Leu Glu Arg Tyr Leu Gly Ala Lys Phe Pro Gly Ala
    210                 215                 220

Lys Arg Phe Ser Leu Glu Gly Gly Asp Ala Leu Ile Pro Met Leu Lys
225                 230                 235                 240

Glu Met Ile Arg His Ala Gly Asn Ser Gly Thr Arg Glu Val Val Leu
                245                 250                 255

Gly Met Ala His Arg Gly Arg Leu Asn Val Leu Val Asn Val Leu Gly
            260                 265                 270

Lys Lys Pro Gln Asp Leu Phe Asp Glu Phe Ala Gly Lys His Lys Glu
        275                 280                 285

His Leu Gly Thr Gly Asp Val Lys Tyr His Met Gly Phe Ser Ser Asp
    290                 295                 300

Phe Gln Thr Asp Gly Gly Leu Val His Leu Ala Leu Ala Phe Asn Pro
305                 310                 315                 320

Ser His Leu Glu Ile Val Ser Pro Val Val Ile Gly Ser Val Arg Ala
                325                 330                 335

Arg Leu Asp Arg Leu Asp Glu Pro Ser Ser Asn Lys Val Leu Pro Ile
            340                 345                 350

Thr Ile His Gly Asp Ala Ala Val Thr Gly Gln Gly Val Val Gln Glu
        355                 360                 365

Thr Leu Asn Met Ser Lys Ala Arg Gly Tyr Glu Val Gly Gly Thr Val
    370                 375                 380

Arg Ile Val Ile Asn Asn Gln Val Gly Phe Thr Thr Ser Asn Pro Leu
385                 390                 395                 400

Asp Ala Arg Ser Thr Pro Tyr Cys Thr Asp Ile Gly Lys Met Val Gln
                405                 410                 415

Ala Pro Ile Phe His Val Asn Ala Asp Asp Pro Glu Ala Val Ala Phe
            420                 425                 430

Val Thr Arg Leu Ala Leu Asp Phe Arg Asn Thr Phe Lys Arg Asp Val
        435                 440                 445

Phe Ile Asp Leu Val Cys Tyr Arg Arg His Gly His Asn Glu Ala Asp
    450                 455                 460

Glu Pro Ser Ala Thr Gln Pro Leu Met Tyr Gln Lys Ile Lys Lys His
465                 470                 475                 480

Pro Thr Pro Arg Lys Ile Tyr Ala Asp Lys Leu Glu Gln Glu Lys Val
                485                 490                 495

Ala Thr Leu Glu Asp Ala Thr Glu Met Val Asn Leu Tyr Arg Asp Ala
            500                 505                 510

Leu Asp Ala Gly Asp Cys Val Val Ala Glu Trp Arg Pro Met Asn Met
        515                 520                 525

His Ser Phe Thr Trp Ser Pro Tyr Leu Asn His Glu Trp Asp Glu Glu
    530                 535                 540

Tyr Pro Asn Lys Val Glu Met Lys Arg Leu Gln Glu Leu Ala Lys Arg
545                 550                 555                 560

Ile Ser Thr Val Pro Glu Ala Val Glu Met Gln Ser Arg Val Ala Lys
                565                 570                 575

Ile Tyr Gly Asp Arg Gln Ala Met Ala Ala Gly Glu Lys Leu Phe Asp
            580                 585                 590

Trp Gly Gly Ala Glu Asn Leu Ala Tyr Ala Thr Leu Val Asp Glu Gly
        595                 600                 605

Ile Pro Val Arg Leu Ser Gly Glu Asp Ser Gly Arg Gly Thr Phe Phe
    610                 615                 620

His Arg His Ala Val Ile His Asn Gln Ser Asn Gly Ser Thr Tyr Thr
625                 630                 635                 640

Pro Leu Gln His Ile His Asn Gly Gln Gly Ala Phe Arg Val Trp Asp
                645                 650                 655

Ser Val Leu Ser Glu Glu Ala Val Leu Ala Phe Glu Tyr Gly Tyr Ala
            660                 665                 670

Thr Ala Glu Pro Arg Thr Leu Thr Ile Trp Glu Ala Gln Phe Gly Asp
        675                 680                 685

Phe Ala Asn Gly Ala Gln Val Val Ile Asp Gln Phe Ile Ser Ser Gly
    690                 695                 700

Glu Gln Lys Trp Gly Arg Met Cys Gly Leu Val Met Leu Leu Pro His
705                 710                 715                 720

Gly Tyr Glu Gly Gln Gly Pro Glu His Ser Ser Ala Arg Leu Glu Arg
                725                 730                 735

Tyr Leu Gln Leu Cys Ala Glu Gln Asn Met Gln Val Cys Val Pro Ser
            740                 745                 750

Thr Pro Ala Gln Val Tyr His Met Leu Arg Arg Gln Ala Leu Arg Gly
        755                 760                 765

Met Arg Arg Pro Leu Val Val Met Ser Pro Lys Ser Leu Leu Arg His
    770                 775                 780

Pro Leu Ala Val Ser Ser Leu Glu Glu Leu Ala Asn Gly Thr Phe Leu
785                 790                 795                 800

Pro Ala Ile Gly Glu Ile Asp Glu Leu Asp Pro Lys Gly Val Lys Arg
                805                 810                 815

Val Val Met Cys Ser Gly Lys Val Tyr Tyr Asp Leu Leu Glu Gln Arg
            820                 825                 830

Arg Lys Asn Asn Gln His Asp Val Ala Ile Val Arg Ile Glu Gln Leu
        835                 840                 845

Tyr Pro Phe Pro His Lys Ala Met Gln Glu Val Leu Gln Gln Phe Ala
    850                 855                 860

His Val Lys Asp Phe Val Trp Cys Gln Glu Glu Pro Leu Asn Gln Gly
865                 870                 875                 880

Ala Trp Tyr Cys Ser Gln His His Phe Arg Glu Val Ile Pro Phe Gly
                885                 890                 895

Ala Ser Leu Arg Tyr Ala Gly Arg Pro Ala Ser Ala Ser Pro Ala Val
            900                 905                 910

Gly Tyr Met Ser Val His Gln Lys Gln Gln Gln Asp Leu Val Asn Asp
        915                 920                 925

Ala Leu Asn Val Glu
    930

<210> 3
<211> 1218
<212> DNA
<213> Escherichia coli

<400> 3
atgagtagcg tagatattct ggtccctgac ctgcctgaat ccgtagccga tgccaccgtc 60

gcaacctggc ataaaaaacc cggcgacgca gtcgtacgtg atgaagtgct ggtagaaatc 120

gaaactgaca aagtggtact ggaagtaccg gcatcagcag acggcattct ggatgcggtt 180

ctggaagatg aaggtacaac ggtaacgtct cgtcagatcc ttggtcgcct gcgtgaaggc 240

aacagcgccg gtaaagaaac cagcgccaaa tctgaagaga aagcgtccac tccggcgcaa 300

cgccagcagg cgtctctgga agagcaaaac aacgatgcgt taagcccggc gatccgtcgc 360

ctgctggctg aacacaatct cgacgccagc gccattaaag gcaccggtgt gggtggtcgt 420

ctgactcgtg aagatgtgga aaaacatctg gcgaaagccc cggcgaaaga gtctgctccg 480

gcagcggctg ctccggcggc gcaaccggct ctggctgcac gtagtgaaaa acgtgtcccg 540

atgactcgcc tgcgtaagcg tgtggcagag cgtctgctgg aagcgaaaaa ctccaccgcc 600

atgctgacca cgttcaacga agtcaacatg aagccgatta tggatctgcg taagcagtac 660

ggtgaagcgt ttgaaaaacg ccacggcatc cgtctgggct ttatgtcctt ctacgtgaaa 720

gcggtggttg aagccctgaa acgttacccg gaagtgaacg cttctatcga cggcgatgac 780

gtggtttacc acaactattt cgacgtcagc atggcggttt ctacgccgcg cggcctggtg 840

acgccggttc tgcgtgatgt cgataccctc ggcatggcag acatcgagaa gaaaatcaaa 900

gagctggcag tcaaaggccg tgacggcaag ctgaccgttg aagatctgac cggtggtaac 960

ttcaccatca ccaacggtgg tgtgttcggt tccctgatgt ctacgccgat catcaacccg 1020

ccgcagagcg caattctggg tatgcacgct atcaaagatc gtccgatggc ggtgaatggt 1080

caggttgaga tcctgccgat gatgtacctg gcgctgtcct acgatcaccg tctgatcgat 1140

ggtcgcgaat ccgtgggctt cctggtaacg atcaaagagt tgctggaaga tccgacgcgt 1200

ctgctgctgg acgtgtag 1218

<210> 4
<211> 405

<212>  PRT
<213>  Escherichia coli

<400>  4

Met Ser Ser Val Asp Ile Leu Val Pro Asp Leu Pro Glu Ser Val Ala
1               5                   10                  15

Asp Ala Thr Val Ala Thr Trp His Lys Lys Pro Gly Asp Ala Val Val
            20                  25                  30

Arg Asp Glu Val Leu Val Glu Ile Glu Thr Asp Lys Val Val Leu Glu
        35                  40                  45

Val Pro Ala Ser Ala Asp Gly Ile Leu Asp Ala Val Leu Glu Asp Glu
    50                  55                  60

Gly Thr Thr Val Thr Ser Arg Gln Ile Leu Gly Arg Leu Arg Glu Gly
65                  70                  75                  80

Asn Ser Ala Gly Lys Glu Thr Ser Ala Lys Ser Glu Glu Lys Ala Ser
                85                  90                  95

Thr Pro Ala Gln Arg Gln Gln Ala Ser Leu Glu Glu Gln Asn Asn Asp
            100                 105                 110

Ala Leu Ser Pro Ala Ile Arg Arg Leu Leu Ala Glu His Asn Leu Asp
        115                 120                 125

Ala Ser Ala Ile Lys Gly Thr Gly Val Gly Gly Arg Leu Thr Arg Glu
    130                 135                 140

Asp Val Glu Lys His Leu Ala Lys Ala Pro Ala Lys Glu Ser Ala Pro
145                 150                 155                 160

Ala Ala Ala Ala Pro Ala Ala Gln Pro Ala Leu Ala Ala Arg Ser Glu
                165                 170                 175

Lys Arg Val Pro Met Thr Arg Leu Arg Lys Arg Val Ala Glu Arg Leu
            180                 185                 190

Leu Glu Ala Lys Asn Ser Thr Ala Met Leu Thr Thr Phe Asn Glu Val
        195                 200                 205

Asn Met Lys Pro Ile Met Asp Leu Arg Lys Gln Tyr Gly Glu Ala Phe
    210                 215                 220

Glu Lys Arg His Gly Ile Arg Leu Gly Phe Met Ser Phe Tyr Val Lys
225                 230                 235                 240

Ala Val Val Glu Ala Leu Lys Arg Tyr Pro Glu Val Asn Ala Ser Ile
                245                 250                 255

Asp Gly Asp Asp Val Val Tyr His Asn Tyr Phe Asp Val Ser Met Ala
            260                 265                 270

Val Ser Thr Pro Arg Gly Leu Val Thr Pro Val Leu Arg Asp Val Asp
        275                 280                 285

Thr Leu Gly Met Ala Asp Ile Glu Lys Lys Ile Lys Glu Leu Ala Val
    290                 295                 300

Lys Gly Arg Asp Gly Lys Leu Thr Val Glu Asp Leu Thr Gly Gly Asn
305                 310                 315                 320

Phe Thr Ile Thr Asn Gly Gly Val Phe Gly Ser Leu Met Ser Thr Pro
                325                 330                 335

Ile Ile Asn Pro Pro Gln Ser Ala Ile Leu Gly Met His Ala Ile Lys
            340                 345                 350

Asp Arg Pro Met Ala Val Asn Gly Gln Val Glu Ile Leu Pro Met Met
        355                 360                 365

Tyr Leu Ala Leu Ser Tyr Asp His Arg Leu Ile Asp Gly Arg Glu Ser
    370                 375                 380

Val Gly Phe Leu Val Thr Ile Lys Glu Leu Leu Glu Asp Pro Thr Arg
385                 390                 395                 400

Leu Leu Leu Asp Val
                405

<210> 5
<211> 1425
<212> DNA
<213> Escherichia coli

<400> 5
atgagtactg aaatcaaaac tcaggtcgtg gtacttgggg caggccccgc aggttactcc 60

gctgccttcc gttgcgctga tttaggtctg gaaaccgtaa tcgtagaacg ttacaacacc 120

cttggcggtg tttgcctgaa cgtcggctgt atcccttcta aagcactgct gcacgtagca 180

aaagttatcg aagaagccaa agcgctggct gaacacggta tcgtcttcgg cgaaccgaaa 240

accgatatcg acaagattcg tacctggaaa gagaaagtga tcaatcagct gaccggtggt 300

ctggctggta tggcgaaagg ccgcaaagtc aaagtggtca acggtctggg taaattcacc 360

ggggctaaca ccctggaagt tgaaggtgag aacggcaaaa ccgtgatcaa cttcgacaac 420

gcgatcattg cagcgggttc tcgcccgatc caactgccgt ttattccgca tgaagatccg 480

cgtatctggg actccactga cgcgctggaa ctgaaagaag taccagaacg cctgctggta 540

atgggtggcg gtatcatcgg tctggaaatg ggcaccgttt accacgcgct gggttcacag 600

attgacgtgg ttgaaatgtt cgaccaggtt atcccggcag ctgacaaaga catcgttaaa 660

gtcttcacca agcgtatcag caagaaattc aacctgatgc tggaaaccaa agttaccgcc 720

gttgaagcga aagaagacgg catttatgtg acgatggaag gcaaaaaagc acccgctgaa 780

ccgcagcgtt acgacgccgt gctggtagcg attggtcgtg tgccgaacgg taaaaacctc 840

gacgcaggca aagcaggcgt ggaagttgac gaccgtggtt tcatccgcgt tgacaaacag 900

ctgcgtacca acgtaccgca catctttgct atcggcgata tcgtcggtca accgatgctg 960

gcacacaaag gtgttcacga aggtcacgtt gccgctgaag ttatcgccgg taagaaacac 1020

tacttcgatc cgaaagttat cccgtccatc gcctataccg aaccagaagt tgcatgggtg 1080

ggtctgactg agaaagaagc gaaagagaaa ggcatcagct atgaaaccgc caccttcccg 1140

tgggctgctt ctggtcgtgc tatcgcttcc gactgcgcag acggtatgac caagctgatt 1200

ttcgacaaag aatctcaccg tgtgatcggt ggtgcgattg tcggtactaa cggcggcgag 1260

ctgctgggtg aaatcggcct ggcaatcgaa atgggttgtg atgctgaaga catcgcactg 1320

accatccacg cgcacccgac tctgcacgag tctgtgggcc tggcggcaga agtgttcgaa 1380

ggtagcatta ccgacctgcc gaacccgaaa gcgaagaaga agtaa 1425

<210> 6
<211> 474
<212> PRT
<213> Escherichia coli

<400> 6

<210>  7
<211>  1167
<212>  DNA
<213>  Escherichia coli

<400>  7
atgaacttac atgaatatca ggcaaaacaa ctttttgccc gctatggctt accagcaccg      60

gtgggttatg cctgtactac tccgcgcgaa gcagaagaag ccgcttcaaa aatcggtgcc     120

ggtccgtggg tagtgaaatg tcaggttcac gctggtggcc gcggtaaagc gggcggtgtg     180

aaagttgtaa acagcaaaga agacatccgt gcttttgcag aaaactggct gggcaagcgt     240

ctggtaacgt atcaaacaga tgccaatggc caaccggtta accagattct ggttgaagca     300

gcgaccgata tcgctaaaga gctgtatctc ggtgccgttg ttgaccgtag ttcccgtcgt     360

gtggtcttta tggcctccac cgaaggcggc gtggaaatcg aaaaagtggc ggaagaaact     420

ccgcacctga tccataaagt tgcgcttgat ccgctgactg gcccgatgcc gtatcaggga     480

cgcgagctgg cgttcaaact gggtctggaa ggtaaactgg ttcagcagtt caccaaaatc     540

ttcatgggcc tggcgaccat tttcctggag cgcgacctgg cgttgatcga aatcaacccg     600

ctggtcatca ccaaacaggg cgatctgatt tgcctcgacg gcaaactggg cgctgacggc     660

aacgcactgt tccgccagcc tgatctgcgc gaaatgcgtg accagtcgca ggaagatccg     720

cgtgaagcac aggctgcaca gtgggaactg aactacgttg cgctggacgg taacatcggt     780

tgtatggtta acggcgcagg tctggcgatg ggtacgatgg acatcgttaa actgcacggc     840

ggcgaaccgg ctaacttcct tgacgttggc ggcggcgcaa ccaaagaacg tgtaaccgaa     900

gcgttcaaaa tcatcctctc tgacgacaaa gtgaaagccg ttctggttaa catcttcggc     960

ggtatcgttc gttgcgacct gatcgctgac ggtatcatcg gcgcggtagc agaagtgggt    1020

gttaacgtac cggtcgtggt acgtctggaa ggtaacaacg ccgaactcgg cgcgaagaaa    1080

ctggctgaca gcggcctgaa tattattgca gcaaaaggtc tgacggatgc agctcagcag    1140

gttgttgccg cagtggaggg gaaataa                                        1167

<210>  8
<211>  388

<212>  PRT
<213>  Escherichia coli

<400>  8

Met Asn Leu His Glu Tyr Gln Ala Lys Gln Leu Phe Ala Arg Tyr Gly
1               5                   10                  15

Leu Pro Ala Pro Val Gly Tyr Ala Cys Thr Thr Pro Arg Glu Ala Glu
            20                  25                  30

Glu Ala Ala Ser Lys Ile Gly Ala Gly Pro Trp Val Val Lys Cys Gln
        35                  40                  45

Val His Ala Gly Gly Arg Gly Lys Ala Gly Gly Val Lys Val Val Asn
    50                  55                  60

Ser Lys Glu Asp Ile Arg Ala Phe Ala Glu Asn Trp Leu Gly Lys Arg
65                  70                  75                  80

Leu Val Thr Tyr Gln Thr Asp Ala Asn Gly Gln Pro Val Asn Gln Ile
                85                  90                  95

Leu Val Glu Ala Ala Thr Asp Ile Ala Lys Glu Leu Tyr Leu Gly Ala
            100                 105                 110

Val Val Asp Arg Ser Ser Arg Arg Val Val Phe Met Ala Ser Thr Glu
        115                 120                 125

Gly Gly Val Glu Ile Glu Lys Val Ala Glu Glu Thr Pro His Leu Ile
    130                 135                 140

His Lys Val Ala Leu Asp Pro Leu Thr Gly Pro Met Pro Tyr Gln Gly
145                 150                 155                 160

Arg Glu Leu Ala Phe Lys Leu Gly Leu Glu Gly Lys Leu Val Gln Gln
                165                 170                 175

Phe Thr Lys Ile Phe Met Gly Leu Ala Thr Ile Phe Leu Glu Arg Asp
            180                 185                 190

Leu Ala Leu Ile Glu Ile Asn Pro Leu Val Ile Thr Lys Gln Gly Asp
        195                 200                 205

Leu Ile Cys Leu Asp Gly Lys Leu Gly Ala Asp Gly Asn Ala Leu Phe
    210                 215                 220

Arg Gln Pro Asp Leu Arg Glu Met Arg Asp Gln Ser Gln Glu Asp Pro
225                 230                 235                 240

Arg Glu Ala Gln Ala Ala Gln Trp Glu Leu Asn Tyr Val Ala Leu Asp
                245                 250                 255

Gly Asn Ile Gly Cys Met Val Asn Gly Ala Gly Leu Ala Met Gly Thr
            260                 265                 270

Met Asp Ile Val Lys Leu His Gly Gly Glu Pro Ala Asn Phe Leu Asp
        275                 280                 285

Val Gly Gly Gly Ala Thr Lys Glu Arg Val Thr Glu Ala Phe Lys Ile
    290                 295                 300

Ile Leu Ser Asp Asp Lys Val Lys Ala Val Leu Val Asn Ile Phe Gly
305                 310                 315                 320

Gly Ile Val Arg Cys Asp Leu Ile Ala Asp Gly Ile Ile Gly Ala Val
                325                 330                 335

Ala Glu Val Gly Val Asn Val Pro Val Val Val Arg Leu Glu Gly Asn
            340                 345                 350

Asn Ala Glu Leu Gly Ala Lys Lys Leu Ala Asp Ser Gly Leu Asn Ile
        355                 360                 365

Ile Ala Ala Lys Gly Leu Thr Asp Ala Ala Gln Gln Val Val Ala Ala
    370                 375                 380

Val Glu Gly Lys
385

<210>  9
<211>  870
<212>  DNA
<213>  Escherichia coli

<400>  9
atgtccattt taatcgataa aaacaccaag gttatctgcc agggctttac cggtagccag      60

gggactttcc actcagaaca ggccattgca tacggcacta aaatggttgg cggcgtaacc     120

ccaggtaaag gcggcaccac ccacctcggc ctgccggtgt tcaacaccgt gcgtgaagcc     180

gttgctgcca ctggcgctac cgcttctgtt atctacgtac cagcaccgtt ctgcaaagac     240

tccattctgg aagccatcga cgcaggcatc aaactgatta tcaccatcac tgaaggcatc     300

ccgacgctgg atatgctgac cgtgaaagtg aagctggatg aagcaggcgt tcgtatgatc     360

ggcccgaact gcccaggcgt tatcactccg ggtgaatgca aaatcggtat ccagcctggt     420

cacattcaca aaccgggtaa agtgggtatc gtttcccgtt ccggtacact gacctatgaa     480

gcggttaaac agaccacgga ttacggtttc ggtcagtcga cctgtgtcgg tatcggcggt     540

gacccgatcc cgggctctaa ctttatcgac attctcgaaa tgttcgaaaa agatccgcag     600

accgaagcga tcgtgatgat cggtgagatc ggcggtagcg ctgaagaaga agcagctgcg     660

tacatcaaag agcacgttac caagccagtt gtgggttaca tcgctggtgt gactgcgccg     720

aaaggcaaac gtatgggcca cgcgggtgcc atcattgccg gtgggaaagg gactgcggat     780

gagaaattcg ctgctctgga agccgcaggc gtgaaaaccg ttcgcagcct ggcggatatc 840

ggtgaagcac tgaaaactgt tctgaaataa 870

<210> 10
<211> 289
<212> PRT
<213> Escherichia coli

<400> 10

Met Ser Ile Leu Ile Asp Lys Asn Thr Lys Val Ile Cys Gln Gly Phe
1               5                   10                  15

Thr Gly Ser Gln Gly Thr Phe His Ser Glu Gln Ala Ile Ala Tyr Gly
            20                  25                  30

Thr Lys Met Val Gly Gly Val Thr Pro Gly Lys Gly Gly Thr Thr His
        35                  40                  45

Leu Gly Leu Pro Val Phe Asn Thr Val Arg Glu Ala Val Ala Ala Thr
    50                  55                  60

Gly Ala Thr Ala Ser Val Ile Tyr Val Pro Ala Pro Phe Cys Lys Asp
65                  70                  75                  80

Ser Ile Leu Glu Ala Ile Asp Ala Gly Ile Lys Leu Ile Ile Thr Ile
                85                  90                  95

Thr Glu Gly Ile Pro Thr Leu Asp Met Leu Thr Val Lys Val Lys Leu
            100                 105                 110

Asp Glu Ala Gly Val Arg Met Ile Gly Pro Asn Cys Pro Gly Val Ile
        115                 120                 125

Thr Pro Gly Glu Cys Lys Ile Gly Ile Gln Pro Gly His Ile His Lys
    130                 135                 140

Pro Gly Lys Val Gly Ile Val Ser Arg Ser Gly Thr Leu Thr Tyr Glu
145                 150                 155                 160

Ala Val Lys Gln Thr Thr Asp Tyr Gly Phe Gly Gln Ser Thr Cys Val
                165                 170                 175

Gly Ile Gly Gly Asp Pro Ile Pro Gly Ser Asn Phe Ile Asp Ile Leu
            180                 185                 190

Glu Met Phe Glu Lys Asp Pro Gln Thr Glu Ala Ile Val Met Ile Gly
        195                 200                 205

Glu Ile Gly Gly Ser Ala Glu Glu Glu Ala Ala Ala Tyr Ile Lys Glu
    210                 215                 220

His Val Thr Lys Pro Val Val Gly Tyr Ile Ala Gly Val Thr Ala Pro
225                 230                 235                 240

Lys Gly Lys Arg Met Gly His Ala Gly Ala Ile Ile Ala Gly Gly Lys
                245                 250                 255

Gly Thr Ala Asp Glu Lys Phe Ala Ala Leu Glu Ala Ala Gly Val Lys
            260                 265                 270

Thr Val Arg Ser Leu Ala Asp Ile Gly Glu Ala Leu Lys Thr Val Leu
        275                 280                 285

Lys

<210>  11
<211>  1362
<212>  DNA
<213>  Clostridium kluyveri

<400>  11
atgagtaatg aagtatctat aaaagaatta attgaaaagg caaaggtggc acaaaaaaaa      60

ttggaagcct atagtcaaga acaagttgat gtactagtaa aagcactagg aaaagtggtt     120

tatgataatg cagaaatgtt tgcaaaagaa gcagttgaag aaacagaaat gggtgtttat     180

gaagataaag tagctaaatg tcatttgaaa tcaggagcta tttggaatca tataaaagac     240

aagaaaactg taggcataat aaaagaagaa cctgaaaggg cacttgttta tgttgctaag     300

ccaaagggag ttgtggcagc tactacgcct ataactaatc cagtggtaac tcctatgtgt     360

aatgcaatgg ctgctataaa gggcagaaat acaataatag tagcaccaca tcctaaagca     420

aagaaagttt cagctcatac tgtagaactt atgaatgctg agcttaaaaa attgggagca     480

ccagaaaata tcatacagat agtagaagca ccatcaagag aagctgctaa ggaacttatg     540

gaaagtgctg atgtagttat tgctacaggc ggtgctggaa gagttaaagc tgcttactcc     600

agtggaagac cagcttatgg cgttggacct ggaaattcac aggtaatagt tgataaggga     660

tacgattata acaaagctgc acaggatata ataacaggaa gaaaatatga caatggaatt     720

atatgttctt cagagcaatc agttatagct cctgctgaag attatgataa ggtaatagca     780

gcttttgtag aaaatggggc attctatgta gaagatgagg aaacagtaga aaagtttaga     840

tcaactttat ttaaagatgg aaaaataaac agcaagatta taggtaaatc cgtccaaatt     900

attgcggatc ttgcaggagt aaaagtacca gaaggtacta aggttatagt acttaagggt     960

aaaggtgcag gagaaaaaga tgtactttgt aaagaaaaaa tgtgtccagt tttagtagca    1020

ttgaaatatg atacttttga agaagcagtt gaaatagcta tggctaatta tatgtatgaa    1080

ggagctggtc atacagcagg catacattct gacaatgacg agaacataag atatgcagga    1140

actgtattac ctataagcag attagttgta aatcagcctg caactactgc tggaggaagt    1200

ttcaataatg gatttaaccc tactactaca ctaggctgcg gatcatgggg cagaaacagt    1260

atttcagaaa atcttactta cgagcatctt ataaatgttt caagaatagg gtatttcaat     1320

aaagaagcaa aagttcctag ctatgaggaa atatggggat aa                        1362

<210>  12
<211>  472
<212>  PRT
<213>  Clostridium kluyveri

<400>  12

Met Ser Asn Glu Val Ser Ile Lys Glu Leu Ile Glu Lys Ala Lys Ala
1               5                   10                  15

Ala Gln Lys Lys Leu Glu Ala Tyr Ser Gln Glu Gln Val Asp Val Leu
            20                  25                  30

Val Lys Ala Leu Gly Lys Val Val Tyr Asp Asn Ala Glu Met Phe Ala
        35                  40                  45

Lys Glu Ala Val Glu Glu Thr Glu Met Gly Val Tyr Glu Asp Lys Val
    50                  55                  60

Ala Lys Cys His Leu Lys Ser Gly Ala Ile Trp Asn His Ile Lys Asp
65                  70                  75                  80

Lys Lys Thr Val Gly Ile Ile Lys Glu Glu Pro Glu Arg Ala Leu Val
                85                  90                  95

Tyr Val Ala Lys Pro Lys Gly Val Val Ala Ala Thr Thr Pro Ile Thr
            100                 105                 110

Asn Pro Val Val Thr Pro Met Cys Asn Ala Met Ala Ala Ile Lys Gly
        115                 120                 125

Arg Asn Thr Ile Ile Val Ala Pro His Pro Lys Ala Lys Lys Val Ser
    130                 135                 140

Ala His Thr Val Glu Leu Met Asn Ala Glu Leu Lys Lys Leu Gly Ala
145                 150                 155                 160

Pro Glu Asn Ile Ile Gln Ile Val Glu Ala Pro Ser Arg Glu Ala Ala
                165                 170                 175

Lys Glu Leu Met Glu Ser Ala Asp Val Val Ile Ala Thr Gly Gly Ala
            180                 185                 190

Gly Arg Val Lys Ala Ala Tyr Ser Ser Gly Arg Pro Ala Tyr Gly Val
        195                 200                 205

Gly Pro Gly Asn Ser Gln Val Ile Val Asp Lys Gly Tyr Asp Tyr Asn
    210                 215                 220

Lys Ala Ala Gln Asp Ile Ile Thr Gly Arg Lys Tyr Asp Asn Gly Ile
225                 230                 235                 240

Ile Cys Ser Ser Glu Gln Ser Val Ile Ala Pro Ala Glu Asp Tyr Asp
                245                 250                 255

Lys Val Ile Ala Ala Phe Val Glu Asn Gly Ala Phe Tyr Val Glu Asp
            260                 265                 270

Glu Glu Thr Val Glu Lys Phe Arg Ser Thr Leu Phe Lys Asp Gly Lys
        275                 280                 285

Ile Asn Ser Lys Ile Ile Gly Lys Ser Val Gln Ile Ile Ala Asp Leu
    290                 295                 300

Ala Gly Val Lys Val Pro Glu Gly Thr Lys Val Ile Val Leu Lys Gly
305                 310                 315                 320

Lys Gly Ala Gly Glu Lys Asp Val Leu Cys Lys Glu Lys Met Cys Pro
                325                 330                 335

Val Leu Val Ala Leu Lys Tyr Asp Thr Phe Glu Glu Ala Val Glu Ile
            340                 345                 350

Ala Met Ala Asn Tyr Met Tyr Glu Gly Ala Gly His Thr Ala Gly Ile
        355                 360                 365

His Ser Asp Asn Asp Glu Asn Ile Arg Tyr Ala Arg Thr Val Leu Pro
    370                 375                 380

Ile Ser Arg Leu Val Val Asn Gln Pro Ala Thr Thr Ala Gly Gly Thr
385                 390                 395                 400

Val Leu Pro Ile Ser Arg Leu Val Val Asn Gln Pro Ala Thr Thr Ala
                405                 410                 415

Gly Gly Ser Phe Asn Asn Gly Phe Asn Pro Thr Thr Thr Leu Gly Cys
            420                 425                 430

Gly Ser Trp Gly Arg Asn Ser Ile Ser Glu Asn Leu Thr Tyr Glu His
        435                 440                 445

Leu Ile Asn Val Ser Arg Ile Gly Tyr Phe Asn Lys Glu Ala Lys Val
    450                 455                 460

Pro Ser Tyr Glu Glu Ile Trp Gly
465                 470

<210>  13
<211>  1074
<212>  DNA

<213>  Metallosphaera sedula

<400>  13
atgaggagaa cgctaaaggc cgcaattctc ggggccacag gtctggttgg aatagaatac      60

gtgagaatgt tagctgatca tccctacatc aagccaacat atcttgcagg aaagggttcc     120

gtgggaaagc cctatggcga aatagttaga tggcaaacag ttgggaacgt gcccaaagag     180

gtagccaacc aagaggtaaa accaacggat ccgaagctaa tggatgacgt tgacataatt     240

ttctctccgc taccgcaggg cgcagcggga cctgtcgagg agcagttcgc aaagctaggc     300

ttcaacgtga tcagcaattc tccggatcac aggtttgata tggacgttcc catgataata     360

ccggaggtca atccgcatac cgtgacctta attgacgagc aaaggaagag gagggactgg     420

aagggattca tagttaccac tcccttatgc acggcccagg gagctgcaat acccctaacg     480

cccatttatc agaacttcaa gatgagcggt gtaatgataa ccacaatgca atccttgtca     540

ggtgctggct atccgggaat cgcttccctg gatattgtgg acaacgctct cccctgggga      600

gatgggtacg acgccaagac cgtgaaggag ataacaagaa tactatcaga ggttaagagg     660

aacgttcagg agcccggcgt caacgagata accctggatg ctactaccca tagaattgca     720

acaattcacg gtcactacga ggtagcatac gtaaccttca aggaggacac agacgttaga     780

aaggtaatgg agtcgatgga gtccttcaag ggagaacccc aggacctgaa gttaccaaca     840

gcaccagaga agccaatcat agttacgaca caggacgcta gaccacaggt attcttcgat     900

aggtgggctg gaaatccgcc cggaatgagc gttgttgtgg gaaggctaaa gcaggttaac     960

ccaaggacca taaggttcgt ttcgctaatc cataacacag ttaggggagc agctggaggc    1020

ggtgtgctga cagctgagct cttagtggag aagggctaca tagacaagag atga          1074

<210>  14
<211>  357
<212>  PRT
<213>  Metallosphaera sedula

<400>  14

Met Arg Arg Thr Leu Lys Ala Ala Ile Leu Gly Ala Thr Gly Leu Val
1               5                   10                  15

Gly Ile Glu Tyr Val Arg Met Leu Ala Asp His Pro Tyr Ile Lys Pro
            20                  25                  30

Thr Tyr Leu Ala Gly Lys Gly Ser Val Gly Lys Pro Tyr Gly Glu Ile
        35                  40                  45

Val Arg Trp Gln Thr Val Gly Asn Val Pro Lys Glu Val Ala Asn Gln
    50                  55                  60

Glu Val Lys Pro Thr Asp Pro Lys Leu Met Asp Asp Val Asp Ile Ile
65                  70                  75                  80

Phe Ser Pro Leu Pro Gln Gly Ala Ala Gly Pro Val Glu Glu Gln Phe

85                  90                  95

Ala Lys Leu Gly Phe Asn Val Ile Ser Asn Ser Pro Asp His Arg Phe
            100                 105                 110

Asp Met Asp Val Pro Met Ile Ile Pro Glu Val Asn Pro His Thr Val
        115                 120                 125

Thr Leu Ile Asp Glu Gln Arg Lys Arg Arg Asp Trp Lys Gly Phe Ile
    130                 135                 140

Val Thr Thr Pro Leu Cys Thr Ala Gln Gly Ala Ala Ile Pro Leu Thr
145                 150                 155                 160

Pro Ile Tyr Gln Asn Phe Lys Met Ser Gly Val Met Ile Thr Thr Met
                165                 170                 175

Gln Ser Leu Ser Gly Ala Gly Tyr Pro Gly Ile Ala Ser Leu Asp Ile
            180                 185                 190

Val Asp Asn Ala Leu Pro Leu Gly Asp Gly Tyr Asp Ala Lys Thr Val
        195                 200                 205

Lys Glu Ile Thr Arg Ile Leu Ser Glu Val Lys Arg Asn Val Gln Glu
    210                 215                 220

Pro Gly Val Asn Glu Ile Thr Leu Asp Ala Thr Thr His Arg Ile Ala
225                 230                 235                 240

Thr Ile His Gly His Tyr Glu Val Ala Tyr Val Thr Phe Lys Glu Asp
                245                 250                 255

Thr Asp Val Arg Lys Val Met Glu Ser Met Glu Ser Phe Lys Gly Glu
            260                 265                 270

Pro Gln Asp Leu Lys Leu Pro Thr Ala Pro Glu Lys Pro Ile Ile Val
        275                 280                 285

Thr Thr Gln Asp Ala Arg Pro Gln Val Phe Phe Asp Arg Trp Ala Gly
    290                 295                 300

Asn Pro Pro Gly Met Ser Val Val Val Gly Arg Leu Lys Gln Val Asn
305                 310                 315                 320

Pro Arg Thr Ile Arg Phe Val Ser Leu Ile His Asn Thr Val Arg Gly
                325                 330                 335

Ala Ala Gly Gly Gly Val Leu Thr Ala Glu Leu Leu Val Glu Lys Gly
            340                 345                 350

Tyr Ile Asp Lys Arg

        355

<210> 15
<211> 3696
<212> DNA
<213> Mycobacterium tuberculosis

<400> 15
gtggccaaca taagttcacc attcgggcaa aacgaatggc tggtcgaaga gatgtaccgc 60

aagttccgcg acgacccctc ctcggtcgat cccagctggc acgagttcct ggttgactac 120

agccccgaac ccacctccca accagctgcc gaaccaaccc gggttacctc gccactcgtt 180

gccgagcggg ccgctgcggc cgccccgcag gcacccccca agccggccga caccgcggcc 240

gcgggcaacg gcgtggtcgc cgcactggcc gccaaaactg ccgttccccc gccagccgaa 300

ggtgacgagg tagcggtgct gcgcggcgcc gccgcggccg tcgtcaagaa catgtccgcg 360

tcgttggagg tgccgacggc gaccagcgtc cgggcggtcc cggccaagct actgatcgac 420

aaccggatcg tcatcaacaa ccagttgaag cggacccgcg gcggcaagat ctcgttcacg 480

catttgctgg gctacgccct ggtgcaggcg gtgaagaaat tcccgaacat gaaccggcac 540

tacaccgaag tcgacggcaa gcccaccgcg gtcacgccgg cgcacaccaa tctcggcctg 600

gcgatcgacc tgcaaggcaa ggacgggaag cgttccctgg tggtggccgg catcaagcgg 660

tgcgagacca tgcgattcgc gcagttcgtc acggcctacg aagacatcgt acgccgggcc 720

cgcgacggca agctgaccac tgaagacttt gccggcgtga cgatttcgct gaccaatccc 780

ggaaccatcg gcaccgtgca ttcggtgccg cggctgatgc ccggccaggg cgccatcatc 840

ggcgtgggcg ccatggaata ccccgccgag tttcaaggcg ccagcgagga acgcatcgcc 900

gagctgggca tcggcaaatt gatcactttg acctccacct acgaccaccg catcatccag 960

ggcgcggaat cgggcgactt cctgcgcacc atccacgagt tgctgctctc ggatggcttc 1020

tgggacgagg tcttccgcga actgagcatc ccatatctgc cggtgcgctg gagcaccgac 1080

aaccccgact cgatcgtcga caagaacgct cgcgtcatga acttgatcgc ggcctaccgc 1140

aaccgcggcc atctgatggc cgataccgac ccgctgcggt tggacaaagc tcggttccgc 1200

agtcaccccg acctcgaagt gctgacccac ggcctgacgc tgtgggatct cgatcgggtg 1260

ttcaaggtcg acggctttgc cggtgcgcag tacaagaaac tgcgcgacgt gctgggcttg 1320

ctgcgcgatg cctactgccg ccacatcggc gtggagtacg cccatatcct cgaccccgaa 1380

caaaaggagt ggctcgaaca acgggtcgag accaagcacg tcaaacccac tgtggcccaa 1440

cagaaataca tcctcagcaa gctcaacgcc gccgaggcct ttgaaacgtt cctacagacc 1500

aagtacgtcg gccagaagcg gttctcgctg gaaggcgccg aaagcgtgat cccgatgatg 1560

gacgcggcga tcgaccagtg cgctgagcac ggcctcgacg aggtggtcat cgggatgccg 1620

caccggggcc ggctcaacgt gctggccaac atcgtcggca agccgtactc gcagatcttc 1680

accgagttcg agggcaacct gaatccgtcg caggcgcacg gctccggtga cgtcaagtac 1740

cacctgggcg ccaccgggct gtacctgcag atgttcggcg acaacgacat tcaggtgtcg 1800

ctgaccgcca acccgtcgca tctggaggcc gtcgacccgg tgctggaggg attggtgcgg 1860
gccaagcagg atctgctcga ccacggaagc atcgacagcg acggccaacg ggcgttctcg 1920
gtggtgccgc tgatgttgca tggcgatgcc gcgttcgccg gtcagggtgt ggtcgccgag 1980
acgctgaacc tggcgaatct gccgggctac cgcgtcggcg gcaccatcca catcatcgtc 2040
aacaaccaga tcggcttcac caccgcgccc gagtattcca ggtccagcga gtactgcacc 2100
gacgtcgcaa agatgatcgg ggcaccgatc tttcacgtca acggcgacga cccggaggcg 2160
tgtgtctggg tggcgcggtt ggcggtggac ttccgacaac ggttcaagaa ggacgtcgtc 2220
atcgacatgc tgtgctaccg ccgccgcggg cacaacgagg gtgacgaccc gtcgatgacc 2280
aacccctacg tgtacgacgt cgtcgacacc aagcgcgggg cccgcaaaag ctacaccgaa 2340
gccctgatcg gacgtggcga catctcgatg aaggaggccg aggacgcgct gcgcgactac 2400
cagggccagc tggaacgggt gttcaacgaa gtgcgcgagc tggagaagca cggtgtgcag 2460
ccgagcgagt cggtcgagtc cgaccagatg attcccgcgg ggctggccac tgcggtggac 2520
aagtcgctgc tggcccggat cggcgatgcg ttcctcgcct tgccgaacgg cttcaccgcg 2580
cacccgcgag tccaaccggt gctggagaag cgccgggaga tggcctatga aggcaagatc 2640
gactgggcct ttggcgagct gctggcgctg ggctcgctgg tggccgaagg caagctggtg 2700
cgcttgtcgg ggcaggacag ccgccgcggc accttctccc agcggcattc ggttctcatc 2760
gaccgccaca ctggcgagga gttcacacca ctgcagctgc tggcgaccaa ctccgacggc 2820
agcccgaccg gcggaaagtt cctggtctac gactcgccac tgtcggagta cgccgccgtc 2880
ggcttcgagt acggctacac tgtgggcaat ccggacgccg tggtgctctg ggaggcgcag 2940
ttcggcgact tcgtcaacgg cgcgcagtcg atcatcgacg agttcatcag ctccggtgag 3000
gccaagtggg gccaattgtc caacgtcgtg ctgctgttac cgcacgggca cgaggggcag 3060
ggacccgacc acacttctgc ccggatcgaa cgcttcttgc agttgtgggc ggaaggttcg 3120
atgaccatcg cgatgccgtc gactccgtcg aactacttcc acctgctacg ccggcatgcc 3180
ctggacggca tccaacgccc gctgatcgtg ttcacgccca agtcgatgtt gcgtcacaag 3240
gccgccgtca gcgaaatcaa ggacttcacc gagatcaagt tccgctcagt gctggaggaa 3300
cccacctatg aggacggcat cggagaccgc aacaaggtca gccggatcct gctgaccagt 3360
ggcaagctgt attacgagct ggccgcccgc aaggccaagg acaaccgcaa tgacctcgcg 3420
atcgtgcggc ttgaacagct cgccccgctg cccaggcgtc gactgcgtga aacgctggac 3480
cgctacgaga acgtcaagga gttcttctgg gtccaagagg aaccggccaa ccagggtgcg 3540
tggccgcgat tcgggctcga actacccgag ctgctgcctg acaagttggc cgggatcaag 3600
cgaatctcgc gccgggcgat gtcagccccg tcgtcaggct cgtcgaaggt gcacgccgtc 3660
gaacagcagg agatcctcga cgaggcgttc ggctga 3696

<210> 16
<211> 1231

<212>  PRT
<213>  Mycobacterium tuberculosis

<400>  16

Met Ala Asn Ile Ser Ser Pro Phe Gly Gln Asn Glu Trp Leu Val Glu
1               5                   10                  15

Glu Met Tyr Arg Lys Phe Arg Asp Asp Pro Ser Ser Val Asp Pro Ser
            20                  25                  30

Trp His Glu Phe Leu Val Asp Tyr Ser Pro Glu Pro Thr Ser Gln Pro
        35                  40                  45

Ala Ala Glu Pro Thr Arg Val Thr Ser Pro Leu Val Ala Glu Arg Ala
    50                  55                  60

Ala Ala Ala Ala Pro Gln Ala Pro Pro Lys Pro Ala Asp Thr Ala Ala
65                  70                  75                  80

Ala Gly Asn Gly Val Val Ala Ala Leu Ala Ala Lys Thr Ala Val Pro
                85                  90                  95

Pro Pro Ala Glu Gly Asp Glu Val Ala Val Leu Arg Gly Ala Ala Ala
            100                 105                 110

Ala Val Val Lys Asn Met Ser Ala Ser Leu Glu Val Pro Thr Ala Thr
        115                 120                 125

Ser Val Arg Ala Val Pro Ala Lys Leu Leu Ile Asp Asn Arg Ile Val
    130                 135                 140

Ile Asn Asn Gln Leu Lys Arg Thr Arg Gly Gly Lys Ile Ser Phe Thr
145                 150                 155                 160

His Leu Leu Gly Tyr Ala Leu Val Gln Ala Val Lys Lys Phe Pro Asn
                165                 170                 175

Met Asn Arg His Tyr Thr Glu Val Asp Gly Lys Pro Thr Ala Val Thr
            180                 185                 190

Pro Ala His Thr Asn Leu Gly Leu Ala Ile Asp Leu Gln Gly Lys Asp
        195                 200                 205

Gly Lys Arg Ser Leu Val Val Ala Gly Ile Lys Arg Cys Glu Thr Met
    210                 215                 220

Arg Phe Ala Gln Phe Val Thr Ala Tyr Glu Asp Ile Val Arg Arg Ala
225                 230                 235                 240

Arg Asp Gly Lys Leu Thr Thr Glu Asp Phe Ala Gly Val Thr Ile Ser
                245                 250                 255

Leu Thr Asn Pro Gly Thr Ile Gly Thr Val His Ser Val Pro Arg Leu
            260                 265                 270

Met Pro Gly Gln Gly Ala Ile Ile Gly Val Gly Ala Met Glu Tyr Pro
        275                 280                 285

Ala Glu Phe Gln Gly Ala Ser Glu Glu Arg Ile Ala Glu Leu Gly Ile
    290                 295                 300

Gly Lys Leu Ile Thr Leu Thr Ser Thr Tyr Asp His Arg Ile Ile Gln
305                 310                 315                 320

Gly Ala Glu Ser Gly Asp Phe Leu Arg Thr Ile His Glu Leu Leu Leu
                325                 330                 335

Ser Asp Gly Phe Trp Asp Glu Val Phe Arg Glu Leu Ser Ile Pro Tyr
            340                 345                 350

Leu Pro Val Arg Trp Ser Thr Asp Asn Pro Asp Ser Ile Val Asp Lys
        355                 360                 365

Asn Ala Arg Val Met Asn Leu Ile Ala Ala Tyr Arg Asn Arg Gly His
    370                 375                 380

Leu Met Ala Asp Thr Asp Pro Leu Arg Leu Asp Lys Ala Arg Phe Arg
385                 390                 395                 400

Ser His Pro Asp Leu Glu Val Leu Thr His Gly Leu Thr Leu Trp Asp
                405                 410                 415

Leu Asp Arg Val Phe Lys Val Asp Gly Phe Ala Gly Ala Gln Tyr Lys
            420                 425                 430

Lys Leu Arg Asp Val Leu Gly Leu Leu Arg Asp Ala Tyr Cys Arg His
        435                 440                 445

Ile Gly Val Glu Tyr Ala His Ile Leu Asp Pro Glu Gln Lys Glu Trp
    450                 455                 460

Leu Glu Gln Arg Val Glu Thr Lys His Val Lys Pro Thr Val Ala Gln
465                 470                 475                 480

Gln Lys Tyr Ile Leu Ser Lys Leu Asn Ala Ala Glu Ala Phe Glu Thr
                485                 490                 495

Phe Leu Gln Thr Lys Tyr Val Gly Gln Lys Arg Phe Ser Leu Glu Gly
            500                 505                 510

Ala Glu Ser Val Ile Pro Met Met Asp Ala Ala Ile Asp Gln Cys Ala
        515                 520                 525

Glu His Gly Leu Asp Glu Val Val Ile Gly Met Pro His Arg Gly Arg
    530                 535                 540

Leu Asn Val Leu Ala Asn Ile Val Gly Lys Pro Tyr Ser Gln Ile Phe
545                 550                 555                 560

Thr Glu Phe Glu Gly Asn Leu Asn Pro Ser Gln Ala His Gly Ser Gly
                565                 570                 575

Asp Val Lys Tyr His Leu Gly Ala Thr Gly Leu Tyr Leu Gln Met Phe
            580                 585                 590

Gly Asp Asn Asp Ile Gln Val Ser Leu Thr Ala Asn Pro Ser His Leu
        595                 600                 605

Glu Ala Val Asp Pro Val Leu Glu Gly Leu Val Arg Ala Lys Gln Asp
    610                 615                 620

Leu Leu Asp His Gly Ser Ile Asp Ser Asp Gly Gln Arg Ala Phe Ser
625                 630                 635                 640

Val Val Pro Leu Met Leu His Gly Asp Ala Ala Phe Ala Gly Gln Gly
                645                 650                 655

Val Val Ala Glu Thr Leu Asn Leu Ala Asn Leu Pro Gly Tyr Arg Val
            660                 665                 670

Gly Gly Thr Ile His Ile Ile Val Asn Asn Gln Ile Gly Phe Thr Thr
        675                 680                 685

Ala Pro Glu Tyr Ser Arg Ser Ser Glu Tyr Cys Thr Asp Val Ala Lys
    690                 695                 700

Met Ile Gly Ala Pro Ile Phe His Val Asn Gly Asp Asp Pro Glu Ala
705                 710                 715                 720

Cys Val Trp Val Ala Arg Leu Ala Val Asp Phe Arg Gln Arg Phe Lys
                725                 730                 735

Lys Asp Val Val Ile Asp Met Leu Cys Tyr Arg Arg Arg Gly His Asn
            740                 745                 750

Glu Gly Asp Asp Pro Ser Met Thr Asn Pro Tyr Val Tyr Asp Val Val
        755                 760                 765

Asp Thr Lys Arg Gly Ala Arg Lys Ser Tyr Thr Glu Ala Leu Ile Gly
    770                 775                 780

Arg Gly Asp Ile Ser Met Lys Glu Ala Glu Asp Ala Leu Arg Asp Tyr
785                 790                 795                 800

Gln Gly Gln Leu Glu Arg Val Phe Asn Glu Val Arg Glu Leu Glu Lys
                805                 810                 815

His Gly Val Gln Pro Ser Glu Ser Val Glu Ser Asp Gln Met Ile Pro
            820                 825                 830

Ala Gly Leu Ala Thr Ala Val Asp Lys Ser Leu Leu Ala Arg Ile Gly
        835                 840                 845

Asp Ala Phe Leu Ala Leu Pro Asn Gly Phe Thr Ala His Pro Arg Val
    850                 855                 860

Gln Pro Val Leu Glu Lys Arg Arg Glu Met Ala Tyr Glu Gly Lys Ile
865                 870                 875                 880

Asp Trp Ala Phe Gly Glu Leu Leu Ala Leu Gly Ser Leu Val Ala Glu
                885                 890                 895

Gly Lys Leu Val Arg Leu Ser Gly Gln Asp Ser Arg Arg Gly Thr Phe
            900                 905                 910

Ser Gln Arg His Ser Val Leu Ile Asp Arg His Thr Gly Glu Glu Phe
        915                 920                 925

Thr Pro Leu Gln Leu Leu Ala Thr Asn Ser Asp Gly Ser Pro Thr Gly
    930                 935                 940

Gly Lys Phe Leu Val Tyr Asp Ser Pro Leu Ser Glu Tyr Ala Ala Val
945                 950                 955                 960

Gly Phe Glu Tyr Gly Tyr Thr Val Gly Asn Pro Asp Ala Val Val Leu
                965                 970                 975

Trp Glu Ala Gln Phe Gly Asp Phe Val Asn Gly Ala Gln Ser Ile Ile
            980                 985                 990

Asp Glu Phe Ile Ser Ser Gly Glu Ala Lys Trp Gly Gln Leu Ser Asn
        995                 1000                1005

Val Val Leu Leu Leu Pro His Gly His Glu Gly Gln Gly Pro Asp
    1010                1015                1020

His Thr Ser Ala Arg Ile Glu Arg Phe Leu Gln Leu Trp Ala Glu
    1025                1030                1035

Gly Ser Met Thr Ile Ala Met Pro Ser Thr Pro Ser Asn Tyr Phe
    1040                1045                1050

His Leu Leu Arg Arg His Ala Leu Asp Gly Ile Gln Arg Pro Leu
    1055                1060                1065

Ile Val Phe Thr Pro Lys Ser Met Leu Arg His Lys Ala Ala Val
    1070                1075                1080

Ser Glu Ile Lys Asp Phe Thr Glu Ile Lys Phe Arg Ser Val Leu
    1085                1090                1095

Glu Glu Pro Thr Tyr Glu Asp Gly Ile Gly Asp Arg Asn Lys Val
    1100                1105                1110

Ser Arg Ile Leu Leu Thr Ser Gly Lys Leu Tyr Tyr Glu Leu Ala
    1115                1120                1125

Ala Arg Lys Ala Lys Asp Asn Arg Asn Asp Leu Ala Ile Val Arg
    1130                1135                1140

Leu Glu Gln Leu Ala Pro Leu Pro Arg Arg Arg Leu Arg Glu Thr
    1145                1150                1155

Leu Asp Arg Tyr Glu Asn Val Lys Glu Phe Phe Trp Val Gln Glu
    1160                1165                1170

Glu Pro Ala Asn Gln Gly Ala Trp Pro Arg Phe Gly Leu Glu Leu
    1175                1180                1185

Pro Glu Leu Leu Pro Asp Lys Leu Ala Gly Ile Lys Arg Ile Ser
    1190                1195                1200

Arg Arg Ala Met Ser Ala Pro Ser Ser Gly Ser Ser Lys Val His
    1205                1210                1215

Ala Val Glu Gln Gln Glu Ile Leu Asp Glu Ala Phe Gly
    1220                1225                1230

<210>  17
<211>  3666
<212>  DNA
<213>  Corynebacterium glutamicum

<400>  17
gtgagcagcg ctagtacttt cggccagaat gcgtggctgg tagacgagat gttccagcag      60

ttccagaagg accccaagtc cgtggacaag gaatggagag aactctttga ggcgcagggg     120

ggaccaaata ctacccccgc tacaacagaa gcacagcctt cagcgcccaa ggagtctgcg     180

aaaccagcac caaaggctgc ccctgcagcc aaggcagcac cgcgcgtaga aaccaagccg     240

gccgacaaga ccgcccctaa ggccaaggag tcctcagtgc cacagcaacc taagcttccg     300

gagccaggac aaaccccaat caggggtatt ttcaagtcca tcgcgaagaa catggatatc     360

tccctggaaa tcccaaccgc aacctcggtt cgcgatatgc cagctcgcct catgttcgaa     420

aaccgcgcga tggtcaacga tcagctcaag cgcacccgcg gtggcaagat ctccttcacc     480

cacatcattg gctacgccat ggtgaaggca gtcatggctc acccggacat gaacaactcc     540

tacgacgtca tcgacggcaa gccaaccctg atcgtgcctg agcacatcaa cctgggcctt 600
gctatcgacc ttcctcagaa ggacggctcc cgcgcacttg tcgtagcagc catcaaggaa 660
accgagaaga tgaacttctc cgagttcctc gcagcctacg aagacatcgt ggcacgctcc 720
cgcaagggca agctcaccat ggatgactac cagggcgtta ccgtttcctt gaccaaccca 780
ggtggcatcg gtacccgcca ctctgttcca cgtctaacca agggccaggg caccatcatc 840
ggtgtcggtt ccatggatta cccagcagag ttccagggcg cttcagaaga ccgccttgca 900
gagctcggcg ttggcaaact tgtcaccatc acctccacct acgatcaccg cgtgatccag 960
ggtgctgtgt ccggtgaatt cctgcgcacc atgtctcgcc tgctcaccga tgattccttc 1020
tgggatgaga tcttcgacgc aatgaacgtt ccttacaccc caatgcgttg ggcacaggac 1080
gttccaaaca ccggtgttga taagaacacc cgcgtcatgc agctcattga ggcataccgc 1140
tcccgtggac acctcatcgc tgacaccaac ccactttcat gggttcagcc tggcatgcca 1200
gttccagacc accgcgacct cgacatcgag acccacaacc tgaccatctg ggatctggac 1260
cgtaccttca acgtcggtgg cttcggcggc aaggagacca tgaccctgcg cgaggtactg 1320
tcccgcctcc gcgctgcgta caccctcaag gtcggctccg aatacaccca catcctggac 1380
cgcgacgagc gcacctggct gcaggaccgc ctcgaggccg gaatgccaaa gccaacccag 1440
gcagagcaga agtacatcct gcagaagctg aacgccgcgg aggctttcga gaacttcctg 1500
cagaccaagt acgtcggcca gaagcgcttc tccctcgaag gtgcagaagc acttatccca 1560
ctgatggact ccgccatcga caccgccgca ggccaaggcc tcgacgaagt tgtcatcggt 1620
atgccacacc gtggtcgcct caacgtgctg ttcaacatcg tgggcaagcc actggcatcc 1680
atcttcaacg agtttgaagg ccaaatggag cagggccaga tcggtggctc cggtgacgtg 1740
aagtaccacc tcggttccga aggccagcac ctgcagatgt tcggcgacgg cgagatcaag 1800
gtctccctga ctgctaaccc gtcccacctg gaagctgtta acccagtgat ggaaggtatc 1860
gtccgcgcaa agcaggacta cctggacaag ggcgtagacg gcaagactgt tgtgccactg 1920
ctgctccacg gtgacgctgc attcgcaggc ctgggcatcg tgccagaaac catcaacctg 1980
gctaagctgc gtggctacga cgtcggcggc accatccaca tcgtggtgaa caaccagatc 2040
ggcttcacca ccaccccaga ctccagccgc tccatgcact acgcaaccga ctacgccaag 2100
gcattcggct gcccagtctt ccacgtcaac ggcgacgacc cagaggcagt tgtctgggtt 2160
ggccagctgg ccaccgagta ccgtcgtcgc ttcggcaagg acgtcttcat cgacctcgtc 2220
tgctaccgcc tccgcggcca caacgaagct gatgatcctt ccatgaccca gccaaagatg 2280
tatgagctca tcaccggccg cgagaccgtt cgtgctcagt acaccgaaga cctgctcgga 2340
cgtggagacc tctccaacga agatgcagaa gcagtcgtcc gcgacttcca cgaccagatg 2400
gaatctgtgt tcaacgaagt caaggaaggc ggcaagaagc aggctgaggc acagaccggc 2460
atcaccggct cccagaagct tccacacggc cttgagacca acatctcccg tgaagagctc 2520
ctggaactgg gacaggcttt cgccaacacc ccagaaggct tcaactacca cccacgtgtg 2580

gctcccgttg ctaagaagcg cgtctcctct gtcaccgaag gtggcatcga ctgggcatgg 2640

ggcgagctcc tcgccttcgg ttccctggct aactccggcc gcttggttcg ccttgcaggt 2700

gaagattccc gccgcggtac cttcacccag cgccacgcag ttgccatcga cccagcgacc 2760

gctgaagagt tcaacccact ccacgagctt gcacagtcca agggcaacaa cggtaagttc 2820

ctggtctaca actccgcact gaccgagtac gcaggcatgg gcttcgagta cggctactcc 2880

gtaggaaacg aagactccat cgttgcatgg gaagcacagt tcggcgactt cgccaacggc 2940

gctcagacca tcatcgatga gtacgtctcc tcaggcgaag ctaagtgggg ccagacctcc 3000

aagctgatcc ttctgctgcc tcacggctac gaaggccagg gcccagacca ctcttccgca 3060

cgtatcgagc gcttcctgca gctgtgcgct gagggttcca tgactgttgc tcagccatcc 3120

accccagcaa accacttcca cctactgcgt cgtcacgctc tgtccgacct gaagcgtcca 3180

ctggttatct tcaccccgaa gtccatgctg cgtaacaagg ctgctgcctc cgcaccagaa 3240

gacttcactg aggtcaccaa gttccagtcc gtgatcaacg atccaaacgt tgcagatgca 3300

gccaaggtga agaaggtcat gctggtctcc ggcaagctgt actacgaatt ggcaaagcgc 3360

aaggagaagg acggacgcga cgacatcgcg atcgttcgta tcgaaatgct ccacccaatt 3420

ccgttcaacc gcatctccga ggctcttgcc ggctacccta acgctgagga agtcctcttc 3480

gttcaggatg agccagcaaa ccagggccca tggccgttct accaggagca cctcccagag 3540

ctgatcccga acatgccaaa gatgcgccgc gtttcccgcc gcgctcagtc ctccaccgca 3600

actggtgttg ccaaggtgca ccagctggag gagaagcagc ttatcgacga ggctttcgag 3660

gcttaa 3666

<210> 18
<211> 1221
<212> PRT
<213> Corynebacterium glutamicum

<400> 18

Met Ser Ser Ala Ser Thr Phe Gly Gln Asn Ala Trp Leu Val Asp Glu
1               5                   10                  15

Met Phe Gln Gln Phe Gln Lys Asp Pro Lys Ser Val Asp Lys Glu Trp
            20                  25                  30

Arg Glu Leu Phe Glu Ala Gln Gly Gly Pro Asn Thr Thr Pro Ala Thr
        35                  40                  45

Thr Glu Ala Gln Pro Ser Ala Pro Lys Glu Ser Ala Lys Pro Ala Pro
    50                  55                  60

Lys Ala Ala Pro Ala Ala Lys Ala Ala Pro Arg Val Glu Thr Lys Pro
65                  70                  75                  80

Ala Asp Lys Thr Ala Pro Lys Ala Lys Glu Ser Ser Val Pro Gln Gln

                85                  90                  95

Pro Lys Leu Pro Glu Pro Gly Gln Thr Pro Ile Arg Gly Ile Phe Lys
            100                 105                 110

Ser Ile Ala Lys Asn Met Asp Ile Ser Leu Glu Ile Pro Thr Ala Thr
        115                 120                 125

Ser Val Arg Asp Met Pro Ala Arg Leu Met Phe Glu Asn Arg Ala Met
    130                 135                 140

Val Asn Asp Gln Leu Lys Arg Thr Arg Gly Gly Lys Ile Ser Phe Thr
145                 150                 155                 160

His Ile Ile Gly Tyr Ala Met Val Lys Ala Val Met Ala His Pro Asp
                165                 170                 175

Met Asn Asn Ser Tyr Asp Val Ile Asp Gly Lys Pro Thr Leu Ile Val
            180                 185                 190

Pro Glu His Ile Asn Leu Gly Leu Ala Ile Asp Leu Pro Gln Lys Asp
        195                 200                 205

Gly Ser Arg Ala Leu Val Val Ala Ala Ile Lys Glu Thr Glu Lys Met
    210                 215                 220

Asn Phe Ser Glu Phe Leu Ala Ala Tyr Glu Asp Ile Val Ala Arg Ser
225                 230                 235                 240

Arg Lys Gly Lys Leu Thr Met Asp Asp Tyr Gln Gly Val Thr Val Ser
                245                 250                 255

Leu Thr Asn Pro Gly Gly Ile Gly Thr Arg His Ser Val Pro Arg Leu
            260                 265                 270

Thr Lys Gly Gln Gly Thr Ile Ile Gly Val Gly Ser Met Asp Tyr Pro
        275                 280                 285

Ala Glu Phe Gln Gly Ala Ser Glu Asp Arg Leu Ala Glu Leu Gly Val
    290                 295                 300

Gly Lys Leu Val Thr Ile Thr Ser Thr Tyr Asp His Arg Val Ile Gln
305                 310                 315                 320

Gly Ala Val Ser Gly Glu Phe Leu Arg Thr Met Ser Arg Leu Leu Thr
                325                 330                 335

Asp Asp Ser Phe Trp Asp Glu Ile Phe Asp Ala Met Asn Val Pro Tyr
            340                 345                 350

Thr Pro Met Arg Trp Ala Gln Asp Val Pro Asn Thr Gly Val Asp Lys

355                 360                 365

Asn Thr Arg Val Met Gln Leu Ile Glu Ala Tyr Arg Ser Arg Gly His
    370                 375                 380

Leu Ile Ala Asp Thr Asn Pro Leu Ser Trp Val Gln Pro Gly Met Pro
385                 390                 395                 400

Val Pro Asp His Arg Asp Leu Asp Ile Glu Thr His Asn Leu Thr Ile
                405                 410                 415

Trp Asp Leu Asp Arg Thr Phe Asn Val Gly Gly Phe Gly Gly Lys Glu
            420                 425                 430

Thr Met Thr Leu Arg Glu Val Leu Ser Arg Leu Arg Ala Ala Tyr Thr
        435                 440                 445

Leu Lys Val Gly Ser Glu Tyr Thr His Ile Leu Asp Arg Asp Glu Arg
    450                 455                 460

Thr Trp Leu Gln Asp Arg Leu Glu Ala Gly Met Pro Lys Pro Thr Gln
465                 470                 475                 480

Ala Glu Gln Lys Tyr Ile Leu Gln Lys Leu Asn Ala Ala Glu Ala Phe
                485                 490                 495

Glu Asn Phe Leu Gln Thr Lys Tyr Val Gly Gln Lys Arg Phe Ser Leu
            500                 505                 510

Glu Gly Ala Glu Ala Leu Ile Pro Leu Met Asp Ser Ala Ile Asp Thr
        515                 520                 525

Ala Ala Gly Gln Gly Leu Asp Glu Val Val Ile Gly Met Pro His Arg
    530                 535                 540

Gly Arg Leu Asn Val Leu Phe Asn Ile Val Gly Lys Pro Leu Ala Ser
545                 550                 555                 560

Ile Phe Asn Glu Phe Glu Gly Gln Met Glu Gln Gly Gln Ile Gly Gly
                565                 570                 575

Ser Gly Asp Val Lys Tyr His Leu Gly Ser Glu Gly Gln His Leu Gln
            580                 585                 590

Met Phe Gly Asp Gly Glu Ile Lys Val Ser Leu Thr Ala Asn Pro Ser
        595                 600                 605

His Leu Glu Ala Val Asn Pro Val Met Glu Gly Ile Val Arg Ala Lys
    610                 615                 620

Gln Asp Tyr Leu Asp Lys Gly Val Asp Gly Lys Thr Val Val Pro Leu

625                 630                 635                 640

Leu Leu His Gly Asp Ala Ala Phe Ala Gly Leu Gly Ile Val Pro Glu
                645                 650                 655

Thr Ile Asn Leu Ala Lys Leu Arg Gly Tyr Asp Val Gly Gly Thr Ile
            660                 665                 670

His Ile Val Val Asn Asn Gln Ile Gly Phe Thr Thr Thr Pro Asp Ser
        675                 680                 685

Ser Arg Ser Met His Tyr Ala Thr Asp Tyr Ala Lys Ala Phe Gly Cys
    690                 695                 700

Pro Val Phe His Val Asn Gly Asp Asp Pro Glu Ala Val Val Trp Val
705                 710                 715                 720

Gly Gln Leu Ala Thr Glu Tyr Arg Arg Arg Phe Gly Lys Asp Val Phe
                725                 730                 735

Ile Asp Leu Val Cys Tyr Arg Leu Arg Gly His Asn Glu Ala Asp Asp
            740                 745                 750

Pro Ser Met Thr Gln Pro Lys Met Tyr Glu Leu Ile Thr Gly Arg Glu
        755                 760                 765

Thr Val Arg Ala Gln Tyr Thr Glu Asp Leu Leu Gly Arg Gly Asp Leu
    770                 775                 780

Ser Asn Glu Asp Ala Glu Ala Val Val Arg Asp Phe His Asp Gln Met
785                 790                 795                 800

Glu Ser Val Phe Asn Glu Val Lys Glu Gly Gly Lys Lys Gln Ala Glu
                805                 810                 815

Ala Gln Thr Gly Ile Thr Gly Ser Gln Lys Leu Pro His Gly Leu Glu
            820                 825                 830

Thr Asn Ile Ser Arg Glu Glu Leu Leu Glu Leu Gly Gln Ala Phe Ala
        835                 840                 845

Asn Thr Pro Glu Gly Phe Asn Tyr His Pro Arg Val Ala Pro Val Ala
    850                 855                 860

Lys Lys Arg Val Ser Ser Val Thr Glu Gly Gly Ile Asp Trp Ala Trp
865                 870                 875                 880

Gly Glu Leu Leu Ala Phe Gly Ser Leu Ala Asn Ser Gly Arg Leu Val
                885                 890                 895

Arg Leu Ala Gly Glu Asp Ser Arg Arg Gly Thr Phe Thr Gln Arg His

900                 905                 910

Ala Val Ala Ile Asp Pro Ala Thr Ala Glu Glu Phe Asn Pro Leu His
        915                 920                 925

Glu Leu Ala Gln Ser Lys Gly Asn Asn Gly Lys Phe Leu Val Tyr Asn
    930                 935                 940

Ser Ala Leu Thr Glu Tyr Ala Gly Met Gly Phe Glu Tyr Gly Tyr Ser
945                 950                 955                 960

Val Gly Asn Glu Asp Ser Ile Val Ala Trp Glu Ala Gln Phe Gly Asp
                965                 970                 975

Phe Ala Asn Gly Ala Gln Thr Ile Ile Asp Glu Tyr Val Ser Ser Gly
            980                 985                 990

Glu Ala Lys Trp Gly Gln Thr Ser  Lys Leu Ile Leu Leu  Leu Pro His
        995                 1000                1005

Gly Tyr  Glu Gly Gln Gly Pro  Asp His Ser Ser Ala  Arg Ile Glu
    1010                1015                1020

Arg Phe  Leu Gln Leu Cys Ala  Glu Gly Ser Met Thr  Val Ala Gln
    1025                1030                1035

Pro Ser  Thr Pro Ala Asn His  Phe His Leu Leu Arg  Arg His Ala
    1040                1045                1050

Leu Ser  Asp Leu Lys Arg Pro  Leu Val Ile Phe Thr  Pro Lys Ser
    1055                1060                1065

Met Leu  Arg Asn Lys Ala Ala  Ala Ser Ala Pro Glu  Asp Phe Thr
    1070                1075                1080

Glu Val  Thr Lys Phe Gln Ser  Val Ile Asn Asp Pro  Asn Val Ala
    1085                1090                1095

Asp Ala  Ala Lys Val Lys Lys  Val Met Leu Val Ser  Gly Lys Leu
    1100                1105                1110

Tyr Tyr  Glu Leu Ala Lys Arg  Lys Glu Lys Asp Gly  Arg Asp Asp
    1115                1120                1125

Ile Ala  Ile Val Arg Ile Glu  Met Leu His Pro Ile  Pro Phe Asn
    1130                1135                1140

Arg Ile  Ser Glu Ala Leu Ala  Gly Tyr Pro Asn Ala  Glu Glu Val
    1145                1150                1155

Leu Phe  Val Gln Asp Glu Pro  Ala Asn Gln Gly Pro  Trp Pro Phe

1160                    1165                    1170

Tyr Gln Glu His Leu Pro Glu Leu Ile Pro Asn Met Pro Lys Met
    1175                    1180                    1185

Arg Arg Val Ser Arg Arg Ala Gln Ser Ser Thr Ala Thr Gly Val
    1190                    1195                    1200

Ala Lys Val His Gln Leu Glu Glu Lys Gln Leu Ile Asp Glu Ala
    1205                    1210                    1215

Phe Glu Ala
    1220

<210>  19
<211>  1647
<212>  DNA
<213>  Lactococcus lactis

<400>  19
atgtatacag taggagatta cctattagac cgattacacg agttaggaat tgaagaaatt      60

tttggagtcc ctggagacta taacttacaa tttttagatc aaattatttc ccgcaaggat     120

atgaaatggg tcggaaatgc taatgaatta aatgcttctt atatggctga tggctatgct     180

cgtactaaaa aagctgccgc atttcttaca acctttggag taggtgaatt gagtgcagtt     240

aatggattag caggaagtta cgccgaaaat ttaccagtag tagaaatagt gggatcacct     300

acatcaaaag tccaaaatga aggaaaattt gttcatcata cgctggctga cggtgatttt     360

aaacacttta tgaaaatgca cgaacctgtt acagcagctc gaactttact gacagcagaa     420

aatgcaaccg ttgaaattga ccgagtactt tctgcactac taaaagaaag aaaacctgtc     480

tatatcaact taccagttga tgttgctgct gcaaaagcag agaaaccctc actccctttg     540

aaaaaagaaa atccaacttc aaatacaagt gaccaagaga ttttgaataa aattcaagaa     600

agcttgaaaa atgccaaaaa accaatcgtg attacaggac atgaaataat tagctttggc     660

ttagaaaata cagtcactca atttatttca aagacaaaac tccctattac gacattaaac     720

tttggaaaaa gttcagttga tgaaactctc ccttcatttt taggaatcta taatggtaaa     780

ctctcagagc ctaatcttaa agaattcgtg gaatcagccg acttcatcct gatgcttgga     840

gttaaactca cagactcttc aacaggagca tttacccatc atttaaatga aaataaaatg     900

atttcactga acatagacga aggaaaaata tttaacgaaa gcatccaaaa ttttgatttt     960

gaatccctca tctcctctct cttagaccta agcggaatag aatacaaagg aaaatatatc    1020

gataaaaagc aagaagactt tgttccatca aatgcgcttt tatcacaaga ccgcctatgg    1080

caagcagttg aaaacctaac tcaaagcaat gaaacaatcg ttgctgaaca agggacatca    1140

ttctttggcg cttcatcaat ttttcttaaa ccaaagagtc attttattgg tcaaccctta    1200

tggggatcaa ttggatatac attcccagca gcattaggaa gccaaattgc agataaagaa    1260

agcagacacc ttttatttat tggtgatggt tcacttcaac ttacagtgca agaattagga    1320

ttagcaatca gagaaaaaat taatccaatt tgctttatta tcaataatga tggttataca 1380

gtcgaaagag aaattcatgg accaaatcaa agctacaatg atattccaat gtggaattac 1440

tcaaaattac cagaatcatt tggagcaaca gaagaacgag tagtctcgaa aatcgttaga 1500

actgaaaatg aatttgtgtc tgtcatgaaa gaagctcaag cagatccaaa tagaatgtac 1560

tggattgagt tagttttggc aaaagaagat gcaccaaaag tactgaaaaa aatgggtaaa 1620

ctatttgctg aacaaaataa atcataa 1647

<210> 20
<211> 548
<212> PRT
<213> Lactococcus lactis

<400> 20

Met Tyr Thr Val Gly Asp Tyr Leu Leu Asp Arg Leu His Glu Leu Gly
1               5                   10                  15

Ile Glu Glu Ile Phe Gly Val Pro Gly Asp Tyr Asn Leu Gln Phe Leu
            20                  25                  30

Asp Gln Ile Ile Ser Arg Lys Asp Met Lys Trp Val Gly Asn Ala Asn
        35                  40                  45

Glu Leu Asn Ala Ser Tyr Met Ala Asp Gly Tyr Ala Arg Thr Lys Lys
    50                  55                  60

Ala Ala Ala Phe Leu Thr Thr Phe Gly Val Gly Glu Leu Ser Ala Val
65                  70                  75                  80

Asn Gly Leu Ala Gly Ser Tyr Ala Glu Asn Leu Pro Val Val Glu Ile
                85                  90                  95

Val Gly Ser Pro Thr Ser Lys Val Gln Asn Glu Gly Lys Phe Val His
            100                 105                 110

His Thr Leu Ala Asp Gly Asp Phe Lys His Phe Met Lys Met His Glu
        115                 120                 125

Pro Val Thr Ala Ala Arg Thr Leu Leu Thr Ala Glu Asn Ala Thr Val
    130                 135                 140

Glu Ile Asp Arg Val Leu Ser Ala Leu Leu Lys Glu Arg Lys Pro Val
145                 150                 155                 160

Tyr Ile Asn Leu Pro Val Asp Val Ala Ala Ala Lys Ala Glu Lys Pro
                165                 170                 175

Ser Leu Pro Leu Lys Lys Glu Asn Pro Thr Ser Asn Thr Ser Asp Gln
            180                 185                 190

Glu Ile Leu Asn Lys Ile Gln Glu Ser Leu Lys Asn Ala Lys Lys Pro
        195                 200                 205

Ile Val Ile Thr Gly His Glu Ile Ile Ser Phe Gly Leu Glu Asn Thr
    210                 215                 220

Val Thr Gln Phe Ile Ser Lys Thr Lys Leu Pro Ile Thr Thr Leu Asn
225                 230                 235                 240

Phe Gly Lys Ser Ser Val Asp Glu Thr Leu Pro Ser Phe Leu Gly Ile
                245                 250                 255

Tyr Asn Gly Lys Leu Ser Glu Pro Asn Leu Lys Glu Phe Val Glu Ser
            260                 265                 270

Ala Asp Phe Ile Leu Met Leu Gly Val Lys Leu Thr Asp Ser Ser Thr
        275                 280                 285

Gly Ala Phe Thr His His Leu Asn Glu Asn Lys Met Ile Ser Leu Asn
    290                 295                 300

Ile Asp Glu Gly Lys Ile Phe Asn Glu Ser Ile Gln Asn Phe Asp Phe
305                 310                 315                 320

Glu Ser Leu Ile Ser Ser Leu Leu Asp Leu Ser Gly Ile Glu Tyr Lys
                325                 330                 335

Gly Lys Tyr Ile Asp Lys Lys Gln Glu Asp Phe Val Pro Ser Asn Ala
            340                 345                 350

Leu Leu Ser Gln Asp Arg Leu Trp Gln Ala Val Glu Asn Leu Thr Gln
        355                 360                 365

Ser Asn Glu Thr Ile Val Ala Glu Gln Gly Thr Ser Phe Phe Gly Ala
    370                 375                 380

Ser Ser Ile Phe Leu Lys Pro Lys Ser His Phe Ile Gly Gln Pro Leu
385                 390                 395                 400

Trp Gly Ser Ile Gly Tyr Thr Phe Pro Ala Ala Leu Gly Ser Gln Ile
                405                 410                 415

Ala Asp Lys Glu Ser Arg His Leu Leu Phe Ile Gly Asp Gly Ser Leu
            420                 425                 430

Gln Leu Thr Val Gln Glu Leu Gly Leu Ala Ile Arg Glu Lys Ile Asn
        435                 440                 445

Pro Ile Cys Phe Ile Ile Asn Asn Asp Gly Tyr Thr Val Glu Arg Glu
    450                 455                 460

Ile His Gly Pro Asn Gln Ser Tyr Asn Asp Ile Pro Met Trp Asn Tyr
465                 470                 475                 480

Ser Lys Leu Pro Glu Ser Phe Gly Ala Thr Glu Glu Arg Val Val Ser
                485                 490                 495

Lys Ile Val Arg Thr Glu Asn Glu Phe Val Ser Val Met Lys Glu Ala
            500                 505                 510

Gln Ala Asp Pro Asn Arg Met Tyr Trp Ile Glu Leu Val Leu Ala Lys
        515                 520                 525

Glu Asp Ala Pro Lys Val Leu Lys Lys Met Gly Lys Leu Phe Ala Glu
    530                 535                 540

Gln Asn Lys Ser
545

<210>  21
<211>  1344
<212>  DNA
<213>  Corynebacterium glutamicum

<400>  21
atgacagttg atgagcaggt ctctaactat tacgacatgc ttctgaagcg caatgctggc      60

gagcctgaat ttcaccaggc agtggcagag gttttggaat ctttgaagat cgtcctggaa     120

aaggaccctc attacgctga ttacggtctc atccagcgcc tgtgcgagcc tgagcgtcag     180

ctcatcttcc gtgtgccttg ggttgatgac cagggccagg tccacgtcaa ccgtggtttc     240

cgcgtgcagt tcaactctgc acttggacca tacaagggcg gcctgcgctt ccacccatct     300

gtaaacctgg gcattgtgaa gttcctgggc tttgagcaga tctttaaaaa ctccctaacc     360

ggcctgccaa tcggtggtgg caagggtgga tccgacttcg accctaaggg caagtccgat     420

ctggaaatca tgcgtttctg ccagtccttc atgaccgagc tacaccgcca catcggtgag     480

taccgcgacg ttcctgcagg tgacatcgga gttggtggcc gcgagatcgg ttacctgttt     540

ggccactacc gtcgcatggc taaccagcac gagtccggcg ttttgaccgg taagggcctg     600

acctggggtg gatccctggt ccgcaccgag gcaactggct acggctgcgt ttacttcgtg     660

agtgaaatga tcaaggctaa gggcgagagc atcagcggcc agaagatcat cgtttccggt     720

tccggcaacg tagcaaccta cgcgattgaa aaggctcagg aactcggcgc aaccgttatt     780

ggtttctccg attccagcgg ttgggttcat acccctaacg gcgttgacgt ggctaagctc     840

cgcgaaatca aggaagttcg tcgcgcacgc gtatccgtgt acgccgacga agttgaaggc     900

gcaacctacc acaccgacgg ttccatctgg gatctcaagt gcgatatcgc tcttccttgt     960

gcaactcaga acgagctcaa cggcgagaac gctaagactc ttgcagacaa cggctgccgt    1020

ttcgttgctg aaggcgcgaa catgccttcc acccctgagg ctgttgaggt cttccgtgag    1080

cgcgacatcc gcttcggacc aggcaaggca gctaacgctg gtggcgttgc aacctccgct    1140

ctggagatgc agcagaacgc ttcgcgcgat tcctggagct tcgagtacac cgacgagcgc 1200

ctccaggtga tcatgaagaa catcttcaag acctgtgcag agaccgcagc agagtatgga 1260

cacgagaacg attacgttgt cggcgctaac attgctggct tcaagaaggt agctgacgcg 1320

atgctggcac agggcgtcat ctaa 1344

<210> 22
<211> 447
<212> PRT
<213> Corynebacterium glutamicum

<400> 22

Met Thr Val Asp Glu Gln Val Ser Asn Tyr Tyr Asp Met Leu Leu Lys
1               5                   10                  15

Arg Asn Ala Gly Glu Pro Glu Phe His Gln Ala Val Ala Glu Val Leu
            20                  25                  30

Glu Ser Leu Lys Ile Val Leu Glu Lys Asp Pro His Tyr Ala Asp Tyr
        35                  40                  45

Gly Leu Ile Gln Arg Leu Cys Glu Pro Glu Arg Gln Leu Ile Phe Arg
    50                  55                  60

Val Pro Trp Val Asp Asp Gln Gly Gln Val His Val Asn Arg Gly Phe
65                  70                  75                  80

Arg Val Gln Phe Asn Ser Ala Leu Gly Pro Tyr Lys Gly Gly Leu Arg
                85                  90                  95

Phe His Pro Ser Val Asn Leu Gly Ile Val Lys Phe Leu Gly Phe Glu
            100                 105                 110

Gln Ile Phe Lys Asn Ser Leu Thr Gly Leu Pro Ile Gly Gly Gly Lys
        115                 120                 125

Gly Gly Ser Asp Phe Asp Pro Lys Gly Lys Ser Asp Leu Glu Ile Met
    130                 135                 140

Arg Phe Cys Gln Ser Phe Met Thr Glu Leu His Arg His Ile Gly Glu
145                 150                 155                 160

Tyr Arg Asp Val Pro Ala Gly Asp Ile Gly Val Gly Gly Arg Glu Ile
                165                 170                 175

Gly Tyr Leu Phe Gly His Tyr Arg Arg Met Ala Asn Gln His Glu Ser
            180                 185                 190

Gly Val Leu Thr Gly Lys Gly Leu Thr Trp Gly Gly Ser Leu Val Arg
        195                 200                 205

Thr Glu Ala Thr Gly Tyr Gly Cys Val Tyr Phe Val Ser Glu Met Ile
    210                 215                 220

Lys Ala Lys Gly Glu Ser Ile Ser Gly Gln Lys Ile Ile Val Ser Gly
225                 230                 235                 240

Ser Gly Asn Val Ala Thr Tyr Ala Ile Glu Lys Ala Gln Glu Leu Gly
                245                 250                 255

Ala Thr Val Ile Gly Phe Ser Asp Ser Ser Gly Trp Val His Thr Pro
            260                 265                 270

Asn Gly Val Asp Val Ala Lys Leu Arg Glu Ile Lys Glu Val Arg Arg
        275                 280                 285

Ala Arg Val Ser Val Tyr Ala Asp Glu Val Glu Gly Ala Thr Tyr His
    290                 295                 300

Thr Asp Gly Ser Ile Trp Asp Leu Lys Cys Asp Ile Ala Leu Pro Cys
305                 310                 315                 320

Ala Thr Gln Asn Glu Leu Asn Gly Glu Asn Ala Lys Thr Leu Ala Asp
                325                 330                 335

Asn Gly Cys Arg Phe Val Ala Glu Gly Ala Asn Met Pro Ser Thr Pro
            340                 345                 350

Glu Ala Val Glu Val Phe Arg Glu Arg Asp Ile Arg Phe Gly Pro Gly
        355                 360                 365

Lys Ala Ala Asn Ala Gly Gly Val Ala Thr Ser Ala Leu Glu Met Gln
    370                 375                 380

Gln Asn Ala Ser Arg Asp Ser Trp Ser Phe Glu Tyr Thr Asp Glu Arg
385                 390                 395                 400

Leu Gln Val Ile Met Lys Asn Ile Phe Lys Thr Cys Ala Glu Thr Ala
                405                 410                 415

Ala Glu Tyr Gly His Glu Asn Asp Tyr Val Val Gly Ala Asn Ile Ala
            420                 425                 430

Gly Phe Lys Lys Val Ala Asp Ala Met Leu Ala Gln Gly Val Ile
        435                 440                 445

<210>  23
<211>  1401
<212>  DNA
<213>  Escherichia coli

<400>  23
atggaccaga agctgttaac ggatttccgc tcagaactac tcgattcacg ttttggcgca        60

aaggccattt ctactatcgc ggagtcaaaa cgatttccgc tgcacgaaat gcgcgatgat 120

gtcgcatttc agattatcaa tgatgaatta tatcttgatg gcaacgctcg tcagaacctg 180

gccactttct gccagacctg ggacgacgaa aacgtccata aattgatgga tttgtcgatc 240

aataaaaact ggatcgacaa agaagaatat ccgcaatccg cagccatcga cctgcgttgc 300

gtaaatatgg ttgccgatct gtggcatgcg cctgcgccga aaaatggtca ggccgttggc 360

accaacacca ttggttcttc cgaggcctgt atgctcggcg ggatggcgat gaaatggcgt 420

tggcgcaagc gtatggaagc tgcaggcaaa ccaacggata aaccaaacct ggtgtgcggt 480

ccggtacaaa tctgctggca taaattcgcc cgctactggg atgtggagct gcgtgagatc 540

cctatgcgcc ccggtcagtt gtttatggac ccgaaacgca tgattgaagc ctgtgacgaa 600

aacaccatcg gcgtggtgcc gactttcggc gtgacctaca ccggtaacta tgagttccca 660

caaccgctgc acgatgcgct ggataaattc caggccgaca ccggtatcga catcgacatg 720

cacatcgacg ctgccagcgg tggcttcctg gcaccgttcg tcgccccgga tatcgtctgg 780

gacttccgcc tgccgcgtgt gaaatcgatc agtgcttcag gccataaatt cggtctggct 840

ccgctgggct gcggctgggt tatctggcgt gacgaagaag cgctgccgca ggaactggtg 900

ttcaacgttg actacctggg tggtcaaatt ggtacttttg ccatcaactt ctcccgcccg 960

gcgggtcagg taattgcaca gtactatgaa ttcctgcgcc tcggtcgtga aggctatacc 1020

aaagtacaga acgcctctta ccaggttgcc gcttatctgg cggatgaaat cgccaaactg 1080

gggccgtatg agttcatctg tacgggtcgc ccggacgaag gcatcccggc ggtttgcttc 1140

aaactgaaag atggtgaaga tccgggatac accctgtacg acctctctga acgtctgcgt 1200

ctgcgcggct ggcaggttcc ggccttcact ctcggcggtg aagccaccga catcgtggtg 1260

atgcgcatta tgtgtcgtcg cggcttcgaa atggactttg ctgaactgtt gctggaagac 1320

tacaaagcct ccctgaaata tctcagcgat cacccgaaac tgcagggtat tgcccagcag 1380

aacagcttta aacacacctg a 1401

<210> 24
<211> 466
<212> PRT
<213> Escherichia coli

<400> 24

Met Asp Gln Lys Leu Leu Thr Asp Phe Arg Ser Glu Leu Leu Asp Ser
1               5                   10                  15

Arg Phe Gly Ala Lys Ala Ile Ser Thr Ile Ala Glu Ser Lys Arg Phe
            20                  25                  30

Pro Leu His Glu Met Arg Asp Asp Val Ala Phe Gln Ile Ile Asn Asp
        35                  40                  45

Glu Leu Tyr Leu Asp Gly Asn Ala Arg Gln Asn Leu Ala Thr Phe Cys

50                  55                  60

Gln Thr Trp Asp Asp Glu Asn Val His Lys Leu Met Asp Leu Ser Ile
65                  70                  75                  80

Asn Lys Asn Trp Ile Asp Lys Glu Glu Tyr Pro Gln Ser Ala Ala Ile
                85                  90                  95

Asp Leu Arg Cys Val Asn Met Val Ala Asp Leu Trp His Ala Pro Ala
            100                 105                 110

Pro Lys Asn Gly Gln Ala Val Gly Thr Asn Thr Ile Gly Ser Ser Glu
        115                 120                 125

Ala Cys Met Leu Gly Gly Met Ala Met Lys Trp Arg Trp Arg Lys Arg
    130                 135                 140

Met Glu Ala Ala Gly Lys Pro Thr Asp Lys Pro Asn Leu Val Cys Gly
145                 150                 155                 160

Pro Val Gln Ile Cys Trp His Lys Phe Ala Arg Tyr Trp Asp Val Glu
                165                 170                 175

Leu Arg Glu Ile Pro Met Arg Pro Gly Gln Leu Phe Met Asp Pro Lys
            180                 185                 190

Arg Met Ile Glu Ala Cys Asp Glu Asn Thr Ile Gly Val Val Pro Thr
        195                 200                 205

Phe Gly Val Thr Tyr Thr Gly Asn Tyr Glu Phe Pro Gln Pro Leu His
    210                 215                 220

Asp Ala Leu Asp Lys Phe Gln Ala Asp Thr Gly Ile Asp Ile Asp Met
225                 230                 235                 240

His Ile Asp Ala Ala Ser Gly Gly Phe Leu Ala Pro Phe Val Ala Pro
                245                 250                 255

Asp Ile Val Trp Asp Phe Arg Leu Pro Arg Val Lys Ser Ile Ser Ala
            260                 265                 270

Ser Gly His Lys Phe Gly Leu Ala Pro Leu Gly Cys Gly Trp Val Ile
        275                 280                 285

Trp Arg Asp Glu Glu Ala Leu Pro Gln Glu Leu Val Phe Asn Val Asp
    290                 295                 300

Tyr Leu Gly Gly Gln Ile Gly Thr Phe Ala Ile Asn Phe Ser Arg Pro
305                 310                 315                 320

Ala Gly Gln Val Ile Ala Gln Tyr Tyr Glu Phe Leu Arg Leu Gly Arg

325                 330                 335

Glu Gly Tyr Thr Lys Val Gln Asn Ala Ser Tyr Gln Val Ala Ala Tyr
            340                 345                 350

Leu Ala Asp Glu Ile Ala Lys Leu Gly Pro Tyr Glu Phe Ile Cys Thr
        355                 360                 365

Gly Arg Pro Asp Glu Gly Ile Pro Ala Val Cys Phe Lys Leu Lys Asp
    370                 375                 380

Gly Glu Asp Pro Gly Tyr Thr Leu Tyr Asp Leu Ser Glu Arg Leu Arg
385                 390                 395                 400

Leu Arg Gly Trp Gln Val Pro Ala Phe Thr Leu Gly Gly Glu Ala Thr
                405                 410                 415

Asp Ile Val Val Met Arg Ile Met Cys Arg Arg Gly Phe Glu Met Asp
            420                 425                 430

Phe Ala Glu Leu Leu Leu Glu Asp Tyr Lys Ala Ser Leu Lys Tyr Leu
        435                 440                 445

Ser Asp His Pro Lys Leu Gln Gly Ile Ala Gln Gln Asn Ser Phe Lys
    450                 455                 460

His Thr
465

<210>  25
<211>  1401
<212>  DNA
<213>  Escherichia coli

<400>  25
atggataaga agcaagtaac ggatttaagg tcggaactac tcgattcacg ttttggtgcg      60

aagtctattt ccactatcgc agaatcaaaa cgttttccgc tgcacgaaat gcgcgacgat     120

gtcgcattcc agattatcaa tgacgaatta tatcttgatg gcaacgctcg tcagaacctg     180

gccactttct gccagacctg ggacgacgaa aatgtccaca aattgatgga tttatccatt     240

aacaaaaact ggatcgacaa agaagaatat ccgcaatccg cagccatcga cctgcgttgc     300

gtaaatatgg ttgccgatct gtggcatgcg cctgcgccga aaaatggtca ggccgttggc     360

accaacacca ttggttcttc cgaggcctgt atgctcggcg ggatggcgat gaaatggcgt     420

tggcgcaagc gtatggaagc tgcaggcaaa ccaacggata aaccaaacct ggtgtgcggt     480

ccggtacaaa tctgctggca taaattcgcc cgctactggg atgtggagct gcgtgagatc     540

cctatgcgcc ccggtcagtt gtttatggac ccgaaacgca tgattgaagc ctgtgacgaa     600

aacaccatcg gcgtggtgcc gactttcggc gtgacctaca ctggtaacta tgagttccca     660

caaccgctgc acgatgcgct ggataaattc caggccgata ccggtatcga catcgacatg     720

cacatcgacg ctgccagcgg tggcttcctg gcaccgttcg tcgccccgga tatcgtctgg 780

gacttccgcc tgccgcgtgt gaaatcgatc agtgcttcag gccataaatt cggtctggct 840

ccgctgggct gcggctgggt tatctggcgt gacgaagaag cgctgccgca ggaactggtg 900

ttcaacgttg actacctggg tggtcaaatt ggtacttttg ccatcaactt ctcccgcccg 960

gcgggtcagg taattgcaca gtactatgaa ttcctgcgcc tcggtcgtga aggctatacc 1020

aaagtacaga acgcctctta ccaggttgcc gcttatctgg cggatgaaat cgccaaactg 1080

gggccgtatg agttcatctg tacgggtcgc ccggacgaag gcatcccggc ggtttgcttc 1140

aaactgaaag atggtgaaga tccgggatac accctgtatg acctctctga acgtctgcgt 1200

ctgcgcggct ggcaggttcc ggccttcact ctcggcggtg aagccaccga catcgtggtg 1260

atgcgcatta tgtgtcgtcg cggcttcgaa atggactttg ctgaactgtt gctggaagac 1320

tacaaagcct ccctgaaata tctcagcgat cacccgaaac tgcagggtat tgcccaacag 1380

aacagcttta aacatacctg a 1401

<210> 26
<211> 466
<212> PRT
<213> Escherichia coli

<400> 26

Met Asp Lys Lys Gln Val Thr Asp Leu Arg Ser Glu Leu Leu Asp Ser
1               5                   10                  15

Arg Phe Gly Ala Lys Ser Ile Ser Thr Ile Ala Glu Ser Lys Arg Phe
            20                  25                  30

Pro Leu His Glu Met Arg Asp Asp Val Ala Phe Gln Ile Ile Asn Asp
        35                  40                  45

Glu Leu Tyr Leu Asp Gly Asn Ala Arg Gln Asn Leu Ala Thr Phe Cys
    50                  55                  60

Gln Thr Trp Asp Asp Glu Asn Val His Lys Leu Met Asp Leu Ser Ile
65                  70                  75                  80

Asn Lys Asn Trp Ile Asp Lys Glu Glu Tyr Pro Gln Ser Ala Ala Ile
                85                  90                  95

Asp Leu Arg Cys Val Asn Met Val Ala Asp Leu Trp His Ala Pro Ala
            100                 105                 110

Pro Lys Asn Gly Gln Ala Val Gly Thr Asn Thr Ile Gly Ser Ser Glu
        115                 120                 125

Ala Cys Met Leu Gly Gly Met Ala Met Lys Trp Arg Trp Arg Lys Arg
    130                 135                 140

Met Glu Ala Ala Gly Lys Pro Thr Asp Lys Pro Asn Leu Val Cys Gly
145                 150                 155                 160

Pro Val Gln Ile Cys Trp His Lys Phe Ala Arg Tyr Trp Asp Val Glu
                165                 170                 175

Leu Arg Glu Ile Pro Met Arg Pro Gly Gln Leu Phe Met Asp Pro Lys
            180                 185                 190

Arg Met Ile Glu Ala Cys Asp Glu Asn Thr Ile Gly Val Val Pro Thr
        195                 200                 205

Phe Gly Val Thr Tyr Thr Gly Asn Tyr Glu Phe Pro Gln Pro Leu His
    210                 215                 220

Asp Ala Leu Asp Lys Phe Gln Ala Asp Thr Gly Ile Asp Ile Asp Met
225                 230                 235                 240

His Ile Asp Ala Ala Ser Gly Gly Phe Leu Ala Pro Phe Val Ala Pro
                245                 250                 255

Asp Ile Val Trp Asp Phe Arg Leu Pro Arg Val Lys Ser Ile Ser Ala
            260                 265                 270

Ser Gly His Lys Phe Gly Leu Ala Pro Leu Gly Cys Gly Trp Val Ile
        275                 280                 285

Trp Arg Asp Glu Glu Ala Leu Pro Gln Glu Leu Val Phe Asn Val Asp
    290                 295                 300

Tyr Leu Gly Gly Gln Ile Gly Thr Phe Ala Ile Asn Phe Ser Arg Pro
305                 310                 315                 320

Ala Gly Gln Val Ile Ala Gln Tyr Tyr Glu Phe Leu Arg Leu Gly Arg
                325                 330                 335

Glu Gly Tyr Thr Lys Val Gln Asn Ala Ser Tyr Gln Val Ala Ala Tyr
            340                 345                 350

Leu Ala Asp Glu Ile Ala Lys Leu Gly Pro Tyr Glu Phe Ile Cys Thr
        355                 360                 365

Gly Arg Pro Asp Glu Gly Ile Pro Ala Val Cys Phe Lys Leu Lys Asp
    370                 375                 380

Gly Glu Asp Pro Gly Tyr Thr Leu Tyr Asp Leu Ser Glu Arg Leu Arg
385                 390                 395                 400

Leu Arg Gly Trp Gln Val Pro Ala Phe Thr Leu Gly Gly Glu Ala Thr
                405                 410                 415

Asp Ile Val Val Met Arg Ile Met Cys Arg Arg Gly Phe Glu Met Asp
            420                 425                 430

Phe Ala Glu Leu Leu Leu Glu Asp Tyr Lys Ala Ser Leu Lys Tyr Leu
        435                 440                 445

Ser Asp His Pro Lys Leu Gln Gly Ile Ala Gln Gln Asn Ser Phe Lys
    450                 455                 460

His Thr
465

<210>  27
<211>  1281
<212>  DNA
<213>  Escherichia coli

<400>  27
atgaacagca ataaagagtt aatgcagcgc cgcagtcagg cgattccccg tggcgttggg      60

caaattcacc cgattttcgc tgaccgcgcg gaaaactgcc gggtgtggga cgttgaaggc     120

cgtgagtatc ttgatttcgc gggcgggatt gcggtgctca ataccgggca cctgcatccg     180

aaggtggtgg ccgcggtgga agcgcagttg aaaaaactgt cgcacacctg cttccaggtg     240

ctggcttacg agccgtatct ggagctgtgc gagattatga atcagaaggt gccgggcgat     300

ttcgccaaga aaacgctgct ggttacgacc ggttccgaag cggtggaaaa cgcggtaaaa     360

atcgcccgcg ccgccaccaa acgtagcggc accatcgctt ttagcggcgc gtatcacggg     420

cgcacgcatt acacgctggc gctgaccggc aaggtgaatc cgtactctgc gggcatgggg     480

ctgatgccgg gtcatgttta tcgcgcgctt tatccttgcc cgctgcacgg cataagcgag     540

gatgacgcta tcgccagcat ccaccggatc ttcaaaaatg atgccgcgcc ggaagatatc     600

gccgccatcg tgattgagcc ggttcagggc gaaggcggtt tctacgcctc gtcgccagcc     660

tttatgcagc gtttacgcgc tctgtgtgac gagcacggga tcatgctgat tgccgatgaa     720

gtgcagagcg gcgcggggcg taccggcacg ctgtttgcga tggagcagat gggcgttgcg     780

ccggatctta ccacctttgc gaaatcgatc gcgggcggct tcccgctggc gggcgtcacc     840

gggcgcgcgg aagtaatgga tgccgtcgct ccaggcggtc tgggcggcac ctatgcgggt     900

aacccgattg cctgcgtggc tgcgctggaa gtgttgaagg tgtttgagca ggaaaatctg     960

ctgcaaaaag ccaacgatct ggggcagaag ttgaaagacg gattgctggc gatagccgaa    1020

aaacacccgg agatcggcga cgtacgcggg ctgggggcga tgatcgccat tgagctgttt    1080

gaagacggcg atcacaacaa gccggacgcc aaactcaccg ccgagatcgt ggctcgcgcc    1140

cgcgataaag gcctgattct tctctcctgc ggcccgtatt acaacgtgct gcgcatcctt    1200

gtaccgctca ccattgaaga cgctcagatc cgtcagggtc tggagatcat cagccagtgt    1260

tttgatgagg cgaagcagta g                                              1281

<210>  28
<211>  426
<212>  PRT
<213>  Escherichia coli

<400>  28

Met Asn Ser Asn Lys Glu Leu Met Gln Arg Arg Ser Gln Ala Ile Pro
1               5                   10                  15

Arg Gly Val Gly Gln Ile His Pro Ile Phe Ala Asp Arg Ala Glu Asn
            20                  25                  30

Cys Arg Val Trp Asp Val Glu Gly Arg Glu Tyr Leu Asp Phe Ala Gly
        35                  40                  45

Gly Ile Ala Val Leu Asn Thr Gly His Leu His Pro Lys Val Val Ala
    50                  55                  60

Ala Val Glu Ala Gln Leu Lys Lys Leu Ser His Thr Cys Phe Gln Val
65                  70                  75                  80

Leu Ala Tyr Glu Pro Tyr Leu Glu Leu Cys Glu Ile Met Asn Gln Lys
                85                  90                  95

Val Pro Gly Asp Phe Ala Lys Lys Thr Leu Leu Val Thr Thr Gly Ser
            100                 105                 110

Glu Ala Val Glu Asn Ala Val Lys Ile Ala Arg Ala Ala Thr Lys Arg
        115                 120                 125

Ser Gly Thr Ile Ala Phe Ser Gly Ala Tyr His Gly Arg Thr His Tyr
    130                 135                 140

Thr Leu Ala Leu Thr Gly Lys Val Asn Pro Tyr Ser Ala Gly Met Gly
145                 150                 155                 160

Leu Met Pro Gly His Val Tyr Arg Ala Leu Tyr Pro Cys Pro Leu His
                165                 170                 175

Gly Ile Ser Glu Asp Asp Ala Ile Ala Ser Ile His Arg Ile Phe Lys
            180                 185                 190

Asn Asp Ala Ala Pro Glu Asp Ile Ala Ala Ile Val Ile Glu Pro Val
        195                 200                 205

Gln Gly Glu Gly Gly Phe Tyr Ala Ser Ser Pro Ala Phe Met Gln Arg
    210                 215                 220

Leu Arg Ala Leu Cys Asp Glu His Gly Ile Met Leu Ile Ala Asp Glu
225                 230                 235                 240

Val Gln Ser Gly Ala Gly Arg Thr Gly Thr Leu Phe Ala Met Glu Gln

                245                 250                 255

Met Gly Val Ala Pro Asp Leu Thr Thr Phe Ala Lys Ser Ile Ala Gly
            260                 265                 270

Gly Phe Pro Leu Ala Gly Val Thr Gly Arg Ala Glu Val Met Asp Ala
        275                 280                 285

Val Ala Pro Gly Gly Leu Gly Gly Thr Tyr Ala Gly Asn Pro Ile Ala
    290                 295                 300

Cys Val Ala Ala Leu Glu Val Leu Lys Val Phe Glu Gln Glu Asn Leu
305                 310                 315                 320

Leu Gln Lys Ala Asn Asp Leu Gly Gln Lys Leu Lys Asp Gly Leu Leu
                325                 330                 335

Ala Ile Ala Glu Lys His Pro Glu Ile Gly Asp Val Arg Gly Leu Gly
            340                 345                 350

Ala Met Ile Ala Ile Glu Leu Phe Glu Asp Gly Asp His Asn Lys Pro
        355                 360                 365

Asp Ala Lys Leu Thr Ala Glu Ile Val Ala Arg Ala Arg Asp Lys Gly
    370                 375                 380

Leu Ile Leu Leu Ser Cys Gly Pro Tyr Tyr Asn Val Leu Arg Ile Leu
385                 390                 395                 400

Val Pro Leu Thr Ile Glu Asp Ala Gln Ile Arg Gln Gly Leu Glu Ile
                405                 410                 415

Ile Ser Gln Cys Phe Asp Glu Ala Lys Gln
            420                 425

<210> 29
<211> 1266
<212> DNA
<213> Escherichia coli

<400> 29
atgagcaaca atgaattcca tcagcgtcgt ctttctgcca ctccgcgcgg ggttggcgtg 60

atgtgtaact tcttcgccca gtcggctgaa aacgccacgc tgaaggatgt tgagggcaac 120

gagtacatcg atttcgccgc aggcattgcg gtgctgaata ccggacatcg ccaccctgat 180

ctggtcgcgg cggtggagca gcaactgcaa cagtttaccc acaccgcgta tcagattgtg 240

ccgtatgaaa gctacgtcac cctggcggag aaaatcaacg cccttgcccc ggtgagcggg 300

caggccaaaa ccgcgttctt caccaccggt gcggaagcgg tggaaaacgc ggtgaaaatt 360

gctcgcgccc ataccggacg ccctggcgtg attgcgttta gcggcggctt tcacggtcgt 420

acgtatatga ccatggcgct gaccggaaaa gttgcgccgt acaaaatcgg cttcggcccg 480

ttccctggtt cggtgtatca cgtaccttat ccgtcagatt tacacggcat ttcaacacag 540

gactccctcg acgccatcga acgcttgttt aaatcagaca tcgaagcgaa gcaggtggcg 600

gcgattattt tcgaaccggt gcagggcgag ggcggtttca acgttgcgcc aaaagagctg 660

gttgccgcta ttcgccgcct gtgcgacgag cacggtattg tgatgattgc tgatgaagtg 720

caaagcggct ttgcgcgtac cggtaagctg tttgccatgg atcattacgc cgataagccg 780

gatttaatga cgatggcgaa aagcctcgcg ggcgggatgc cgctttcggg cgtggtcggt 840

aacgcgaata ttatggacgc acccgcgccg ggcgggcttg gcggcaccta cgccggtaac 900

ccgctggcgg tggctgccgc gcacgcggtg ctcaacatta tcgacaaaga atcactctgc 960

gaacgcgcga atcaactggg ccagcgtctc aaaaacacgt tgattgatgc caaagaaagc 1020

gttccggcca ttgctgcggt acgcggcctg gggtcgatga ttgcggtaga gtttaacgat 1080

ccgcaaacgg gcgagccgtc agcggcgatt gcacagaaaa tccagcaacg cgcgctggcg 1140

caggggctgc tcctgctgac ctgtggcgca tacggcaacg tgattcgctt cctgtatccg 1200

ctgaccatcc cggatgcgca attcgatgcg gcaatgaaaa ttttgcagga tgcgctgagc 1260

gattaa 1266

<210> 30
<211> 421
<212> PRT
<213> Escherichia coli

<400> 30

Met Ser Asn Asn Glu Phe His Gln Arg Arg Leu Ser Ala Thr Pro Arg
1               5                   10                  15

Gly Val Gly Val Met Cys Asn Phe Phe Ala Gln Ser Ala Glu Asn Ala
            20                  25                  30

Thr Leu Lys Asp Val Glu Gly Asn Glu Tyr Ile Asp Phe Ala Ala Gly
        35                  40                  45

Ile Ala Val Leu Asn Thr Gly His Arg His Pro Asp Leu Val Ala Ala
    50                  55                  60

Val Glu Gln Gln Leu Gln Gln Phe Thr His Thr Ala Tyr Gln Ile Val
65                  70                  75                  80

Pro Tyr Glu Ser Tyr Val Thr Leu Ala Glu Lys Ile Asn Ala Leu Ala
                85                  90                  95

Pro Val Ser Gly Gln Ala Lys Thr Ala Phe Phe Thr Thr Gly Ala Glu
            100                 105                 110

Ala Val Glu Asn Ala Val Lys Ile Ala Arg Ala His Thr Gly Arg Pro
        115                 120                 125

Gly Val Ile Ala Phe Ser Gly Gly Phe His Gly Arg Thr Tyr Met Thr
    130                 135                 140

Met Ala Leu Thr Gly Lys Val Ala Pro Tyr Lys Ile Gly Phe Gly Pro
145                 150                 155                 160

Phe Pro Gly Ser Val Tyr His Val Pro Tyr Pro Ser Asp Leu His Gly
                165                 170                 175

Ile Ser Thr Gln Asp Ser Leu Asp Ala Ile Glu Arg Leu Phe Lys Ser
            180                 185                 190

Asp Ile Glu Ala Lys Gln Val Ala Ala Ile Ile Phe Glu Pro Val Gln
        195                 200                 205

Gly Glu Gly Gly Phe Asn Val Ala Pro Lys Glu Leu Val Ala Ala Ile
    210                 215                 220

Arg Arg Leu Cys Asp Glu His Gly Ile Val Met Ile Ala Asp Glu Val
225                 230                 235                 240

Gln Ser Gly Phe Ala Arg Thr Gly Lys Leu Phe Ala Met Asp His Tyr
                245                 250                 255

Ala Asp Lys Pro Asp Leu Met Thr Met Ala Lys Ser Leu Ala Gly Gly
            260                 265                 270

Met Pro Leu Ser Gly Val Val Gly Asn Ala Asn Ile Met Asp Ala Pro
        275                 280                 285

Ala Pro Gly Gly Leu Gly Gly Thr Tyr Ala Gly Asn Pro Leu Ala Val
    290                 295                 300

Ala Ala Ala His Ala Val Leu Asn Ile Ile Asp Lys Glu Ser Leu Cys
305                 310                 315                 320

Glu Arg Ala Asn Gln Leu Gly Gln Arg Leu Lys Asn Thr Leu Ile Asp
                325                 330                 335

Ala Lys Glu Ser Val Pro Ala Ile Ala Ala Val Arg Gly Leu Gly Ser
            340                 345                 350

Met Ile Ala Val Glu Phe Asn Asp Pro Gln Thr Gly Glu Pro Ser Ala
        355                 360                 365

Ala Ile Ala Gln Lys Ile Gln Gln Arg Ala Leu Ala Gln Gly Leu Leu
    370                 375                 380

Leu Leu Thr Cys Gly Ala Tyr Gly Asn Val Ile Arg Phe Leu Tyr Pro
385                 390                 395                 400

Leu Thr Ile Pro Asp Ala Gln Phe Asp Ala Ala Met Lys Ile Leu Gln
                405                 410                 415

Asp Ala Leu Ser Asp
            420

<210> 31
<211> 1116
<212> DNA
<213> Clostridium kluyveri

<400> 31
atgaagttat taaaattggc acctgatgtt tataaatttg atactgcaga ggagtttatg 60

aaatacttta aggttggaaa aggtgacttt atacttacta atgaattttt atataaacct 120

ttccttgaga aattcaatga tggtgcagat gctgtatttc aggagaaata tggactcggt 180

gaaccttctg atgaaatgat aaacaatata attaaggata ttggagataa acaatataat 240

agaattattg ctgtaggggg aggatctgta atagatatag ccaaaatcct cagtcttaag 300

tatactgatg attcattgga tttgtttgag ggaaaagtac ctcttgtaaa aaacaaagaa 360

ttaattatag ttccaactac atgtggaaca ggttcagaag ttacaaatgt atcagttgca 420

gaattaaaga gaagacatac taaaaaagga attgcttcag acgaattata tgcaacttat 480

gcagtacttg taccagaatt tataaaagga cttccatata agttttttgt aaccagctcc 540

gtagatgcct taatacatgc aacagaagct tatgtatctc caaatgcaaa tccttatact 600

gatatgttta gtgtaaaagc tatggagtta attttaaatg gatacatgca aatggtagag 660

aaaggaaatg attacagagt tgaaataatt gaggattttg ttataggcag caattatgca 720

ggtatagctt ttggaaatgc aggagtggga gcggttcacg cactctcata tccaataggc 780

ggaaattatc atgtgcctca tggagaagca aattatctgt tttttacaga aatatttaaa 840

acttattatg agaaaaatcc aaatggcaag attaaagatg taaataaact attagcaggc 900

atactaaaat gtgatgaaag tgaagcttat gacagtttat cacaactttt agataaatta 960

ttgtcaagaa aaccattaag agaatatgga atgaaagagg aagaaattga aacttttgct 1020

gattcagtaa tagaaggaca gcagagactg ttggtaaaca attatgaacc tttttcaaga 1080

gaagacatag taaacacata taaaaagtta tattaa 1116

<210> 32
<211> 371
<212> PRT
<213> Clostridium kluyveri

<400> 32

Met Lys Leu Leu Lys Leu Ala Pro Asp Val Tyr Lys Phe Asp Thr Ala
1               5                   10                  15

Glu Glu Phe Met Lys Tyr Phe Lys Val Gly Lys Gly Asp Phe Ile Leu
            20                  25                  30

Thr Asn Glu Phe Leu Tyr Lys Pro Phe Leu Glu Lys Phe Asn Asp Gly
        35                  40                  45

Ala Asp Ala Val Phe Gln Glu Lys Tyr Gly Leu Gly Glu Pro Ser Asp
    50                  55                  60

Glu Met Ile Asn Asn Ile Ile Lys Asp Ile Gly Asp Lys Gln Tyr Asn
65                  70                  75                  80

Arg Ile Ile Ala Val Gly Gly Gly Ser Val Ile Asp Ile Ala Lys Ile
                85                  90                  95

Leu Ser Leu Lys Tyr Thr Asp Asp Ser Leu Asp Leu Phe Glu Gly Lys
            100                 105                 110

Val Pro Leu Val Lys Asn Lys Glu Leu Ile Ile Val Pro Thr Thr Cys
        115                 120                 125

Gly Thr Gly Ser Glu Val Thr Asn Val Ser Val Ala Glu Leu Lys Arg
    130                 135                 140

Arg His Thr Lys Lys Gly Ile Ala Ser Asp Glu Leu Tyr Ala Thr Tyr
145                 150                 155                 160

Ala Val Leu Val Pro Glu Phe Ile Lys Gly Leu Pro Tyr Lys Phe Phe
                165                 170                 175

Val Thr Ser Ser Val Asp Ala Leu Ile His Ala Thr Glu Ala Tyr Val
            180                 185                 190

Ser Pro Asn Ala Asn Pro Tyr Thr Asp Met Phe Ser Val Lys Ala Met
        195                 200                 205

Glu Leu Ile Leu Asn Gly Tyr Met Gln Met Val Glu Lys Gly Asn Asp
    210                 215                 220

Tyr Arg Val Glu Ile Ile Glu Asp Phe Val Ile Gly Ser Asn Tyr Ala
225                 230                 235                 240

Gly Ile Ala Phe Gly Asn Ala Gly Val Gly Ala Val His Ala Leu Ser
                245                 250                 255

Tyr Pro Ile Gly Gly Asn Tyr His Val Pro His Gly Glu Ala Asn Tyr
            260                 265                 270

Leu Phe Phe Thr Glu Ile Phe Lys Thr Tyr Tyr Glu Lys Asn Pro Asn
        275                 280                 285

Gly Lys Ile Lys Asp Val Asn Lys Leu Leu Ala Gly Ile Leu Lys Cys
    290                 295                 300

Asp Glu Ser Glu Ala Tyr Asp Ser Leu Ser Gln Leu Leu Asp Lys Leu
305                 310                 315                 320

Leu Ser Arg Lys Pro Leu Arg Glu Tyr Gly Met Lys Glu Glu Glu Ile
                325                 330                 335

Glu Thr Phe Ala Asp Ser Val Ile Glu Gly Gln Gln Arg Leu Leu Val
            340                 345                 350

Asn Asn Tyr Glu Pro Phe Ser Arg Glu Asp Ile Val Asn Thr Tyr Lys
        355                 360                 365

Lys Leu Tyr
    370

<210>  33
<211>  1617
<212>  DNA
<213>  Clostridium kluyveri

<400>  33
atgagtaaag ggataaagaa ttcacaattg aaaaaaaaga atgtaaaggc tagtaatgtg      60

gcagaaaaga ttgaagagaa agttgaaaaa acagataagg ttgttgaaaa ggcagctgag     120

gttacagaaa aacgaattag aaacttgaag cttcaggaaa aagttgtaac agcagatgtg     180

gcagctgata tgatagaaaa cggtatgatt gttgcaatta gcggatttac tccttccggg     240

tatcctaaag aagtacctaa agcattgact aaaaaagtta atgccttaga ggaagaattc     300

aaggtaacac tttatacagg ttcatctaca ggagccgata tagacggaga atgggcaaaa     360

gcaggaataa tagaaagaag aattccatat cagacaaatt ctgatatgag gaaaaaaata     420

aatgatggtt ctattaagta tgctgatatg catttaagcc atatggctca atatattaat     480

tattctgtaa ttcctaaagt agatatagct ataatagagg cagtagctat tacagaagaa     540

ggggatatta ttccttcaac aggaattgga aatacagcta cttttgtgga aaatgcagat     600

aaggtaatag tggaaattaa tgaggctcaa ccgcttgaat tggaaggtat ggcagatata     660

tatacattaa aaaaccctcc aagaagagag cccataccta tagttaatgc aggcaatagg     720

atagggacca catatgtgac ctgtggttct gaaaaaatat gcgctatagt gatgacaaat     780

acccaggata aaacaagacc tcttacagaa gtgtctcctg tatctcaggc tatatccgat     840

aatcttatag gatttttaaa taaagaggtt gaagagggaa aattacctaa gaacctgctt     900

cctatacagt caggagttgg aagtgtagca aatgcagttt tggccggact ttgtgaatca     960

aattttaaaa atttgagttg ttatacagaa gttatacagg attctatgct gaagcttata    1020

aaatgtggta aagcagatgt ggtgtcaggc acttccataa gtccttcacc ggagatgttg    1080

cctgagttca taaaggacat aaatttcttt agagaaaaga tagtattaag accacaggaa    1140

ataagtaata atccagagat agcaagaaga ataggagtta tatccataaa cactgctttg    1200

gaagtagata tatatggtaa tgtaaactcc actcatgtta tgggaagcaa aatgatgaat    1260

ggtataggcg gttctggaga ctttgccaga aatgcatatt tgactatatt cactacagag 1320

tctatcgcca aaaaaggaga tatatcatct atagttccta tggtatccca tgtggatcat 1380

acagaacatg atgtaatggt aattgttaca gaacagggag tagcagattt aagaggtctt 1440

tctcctaggg aaaaggccgt ggctataata gaaaattgtg ttcatcctga ttacaaggat 1500

atgcttatgg aatattttga agaggcttgt aagtcatcag gtggaaatac accacataat 1560

cttgaaaaag ctctttcctg gcatacaaaa tttataaaaa ctggtagtat gaaataa 1617

<210> 34
<211> 538
<212> PRT
<213> Clostridium kluyveri

<400> 34

Met Ser Lys Gly Ile Lys Asn Ser Gln Leu Lys Lys Lys Asn Val Lys
1               5                   10                  15

Ala Ser Asn Val Ala Glu Lys Ile Glu Glu Lys Val Glu Lys Thr Asp
            20                  25                  30

Lys Val Val Glu Lys Ala Ala Glu Val Thr Glu Lys Arg Ile Arg Asn
        35                  40                  45

Leu Lys Leu Gln Glu Lys Val Val Thr Ala Asp Val Ala Ala Asp Met
    50                  55                  60

Ile Glu Asn Gly Met Ile Val Ala Ile Ser Gly Phe Thr Pro Ser Gly
65                  70                  75                  80

Tyr Pro Lys Glu Val Pro Lys Ala Leu Thr Lys Lys Val Asn Ala Leu
                85                  90                  95

Glu Glu Glu Phe Lys Val Thr Leu Tyr Thr Gly Ser Ser Thr Gly Ala
            100                 105                 110

Asp Ile Asp Gly Glu Trp Ala Lys Ala Gly Ile Ile Glu Arg Arg Ile
        115                 120                 125

Pro Tyr Gln Thr Asn Ser Asp Met Arg Lys Lys Ile Asn Asp Gly Ser
    130                 135                 140

Ile Lys Tyr Ala Asp Met His Leu Ser His Met Ala Gln Tyr Ile Asn
145                 150                 155                 160

Tyr Ser Val Ile Pro Lys Val Asp Ile Ala Ile Ile Glu Ala Val Ala
                165                 170                 175

Ile Thr Glu Glu Gly Asp Ile Ile Pro Ser Thr Gly Ile Gly Asn Thr
            180                 185                 190

Ala Thr Phe Val Glu Asn Ala Asp Lys Val Ile Val Glu Ile Asn Glu
        195                 200                 205

Ala Gln Pro Leu Glu Leu Glu Gly Met Ala Asp Ile Tyr Thr Leu Lys
    210                 215                 220

Asn Pro Pro Arg Arg Glu Pro Ile Pro Ile Val Asn Ala Gly Asn Arg
225                 230                 235                 240

Ile Gly Thr Thr Tyr Val Thr Cys Gly Ser Glu Lys Ile Cys Ala Ile
                245                 250                 255

Val Met Thr Asn Thr Gln Asp Lys Thr Arg Pro Leu Thr Glu Val Ser
            260                 265                 270

Pro Val Ser Gln Ala Ile Ser Asp Asn Leu Ile Gly Phe Leu Asn Lys
        275                 280                 285

Glu Val Glu Glu Gly Lys Leu Pro Lys Asn Leu Leu Pro Ile Gln Ser
    290                 295                 300

Gly Val Gly Ser Val Ala Asn Ala Val Leu Ala Gly Leu Cys Glu Ser
305                 310                 315                 320

Asn Phe Lys Asn Leu Ser Cys Tyr Thr Glu Val Ile Gln Asp Ser Met
                325                 330                 335

Leu Lys Leu Ile Lys Cys Gly Lys Ala Asp Val Val Ser Gly Thr Ser
            340                 345                 350

Ile Ser Pro Ser Pro Glu Met Leu Pro Glu Phe Ile Lys Asp Ile Asn
        355                 360                 365

Phe Phe Arg Glu Lys Ile Val Leu Arg Pro Gln Glu Ile Ser Asn Asn
    370                 375                 380

Pro Glu Ile Ala Arg Arg Ile Gly Val Ile Ser Ile Asn Thr Ala Leu
385                 390                 395                 400

Glu Val Asp Ile Tyr Gly Asn Val Asn Ser Thr His Val Met Gly Ser
                405                 410                 415

Lys Met Met Asn Gly Ile Gly Gly Ser Gly Asp Phe Ala Arg Asn Ala
            420                 425                 430

Tyr Leu Thr Ile Phe Thr Thr Glu Ser Ile Ala Lys Lys Gly Asp Ile
        435                 440                 445

Ser Ser Ile Val Pro Met Val Ser His Val Asp His Thr Glu His Asp
    450                 455                 460

Val Met Val Ile Val Thr Glu Gln Gly Val Ala Asp Leu Arg Gly Leu
465                 470                 475                 480

Ser Pro Arg Glu Lys Ala Val Ala Ile Ile Glu Asn Cys Val His Pro
                485                 490                 495

Asp Tyr Lys Asp Met Leu Met Glu Tyr Phe Glu Glu Ala Cys Lys Ser
            500                 505                 510

Ser Gly Gly Asn Thr Pro His Asn Leu Glu Lys Ala Leu Ser Trp His
        515                 520                 525

Thr Lys Phe Ile Lys Thr Gly Ser Met Lys
    530                 535

<210>  35
<211>  1290
<212>  DNA
<213>  Clostridium kluyveri

<400>  35
atggagtggg aagagatata taaagagaaa ctggtaactg cagaaaaagc tgtttcaaaa      60

atagaaaacc atagcagggt agtttttgca catgcagtag gagaacccgt agatttagta     120

aatgcactag ttaaaaataa ggataattat ataggactag aaatagttca catggtagct     180

atgggcaaag gtgaatatac aaaagagggt atgcaaagac attttagaca taatgcttta     240

tttgtaggcg gatgtactag agatgcagta aattcaggaa gagcagatta tacaccttgt     300

tttttctatg aagtgccaag tttgtttaaa gaaaaacgtt tgcctgtaga tgtagcactt     360

attcaggtaa gtgagccaga taaatatggc tactgcagtt ttggagtttc caatgactat     420

accaagccag cagcagaaag tgctaagctt gtaattgcag aagtgaataa aaacatgcca     480

agaactcttg gagattcttt tatacatgta tcagatattg attatatagt ggaagcttca     540

cacccattgt tagaattgca gcctcctaaa ttgggagatg tagaaaaagc cataggagaa     600

aactgtgcat ctttaattga agatggagct actcttcagc ttggaatagg tgctatacca     660

gatgcggtac ttttattctt aaagaacaaa aagaatttag gaatacattc tgagatgata     720

tcagatggtg tgatggaact ggtgaaggca ggggttatca ataacaagaa aaagaccctc     780

catccaggca aaatagttgt aacattttta atgggaacaa aaaaattata tgattttgta     840

aacaataatc caatggtaga aacttattct gtagattatg taaataatcc actggtaatt     900

atgaaaaatg acaatatggt ttcaataaat tcttgtgttc aagtagactt aatgggacaa     960

gtatgttctg aaagtatagg attgaaacag ataagtggag tgggaggcca ggtagatttt    1020

attagaggag ctaatctatc aaagggtgga aaggctatta tagctatacc ttccacagct    1080

ggaaaaggaa aagtttcaag aataactcca cttctagata ctggtgctgc agttacaact    1140

tctagaaatg aagtagatta tgtagttact gaatatggtg ttgctcatct taagggcaaa    1200

actttaagaa atagggcaag agctctaata aatatcgctc atccaaaatt cagagaatca    1260

ttaatgaatg aatttaaaaa gagattttag                                          1290

<210>  36
<211>  429
<212>  PRT
<213>  Clostridium kluyveri

<400>  36

Met Glu Trp Glu Glu Ile Tyr Lys Glu Lys Leu Val Thr Ala Glu Lys
1               5                   10                  15

Ala Val Ser Lys Ile Glu Asn His Ser Arg Val Val Phe Ala His Ala
            20                  25                  30

Val Gly Glu Pro Val Asp Leu Val Asn Ala Leu Val Lys Asn Lys Asp
        35                  40                  45

Asn Tyr Ile Gly Leu Glu Ile Val His Met Val Ala Met Gly Lys Gly
    50                  55                  60

Val Tyr Thr Lys Glu Gly Met Gln Arg His Phe Arg His Asn Ala Leu
65                  70                  75                  80

Phe Val Gly Gly Ser Thr Arg Asp Ala Val Asn Ser Gly Arg Ala Val
                85                  90                  95

Tyr Thr Pro Cys Phe Phe Tyr Glu Val Pro Ser Leu Phe Lys Glu Lys
            100                 105                 110

Arg Leu Pro Val Asp Val Ala Leu Ile Gln Val Ser Glu Pro Asp Lys
        115                 120                 125

Tyr Gly Tyr Cys Ser Phe Gly Val Ser Asn Asp Tyr Thr Lys Pro Ala
    130                 135                 140

Ala Glu Ser Ala Lys Leu Val Ile Ala Glu Val Asn Lys Asn Met Pro
145                 150                 155                 160

Arg Thr Leu Gly Asp Ser Phe Ile His Val Ser Asp Ile Asp Tyr Ile
                165                 170                 175

Val Glu Ala Ser His Pro Leu Leu Glu Leu Gln Pro Pro Lys Leu Gly
            180                 185                 190

Asp Val Glu Lys Ala Ile Gly Glu Asn Cys Ala Ser Leu Ile Glu Asp
        195                 200                 205

Gly Ala Thr Leu Gln Leu Gly Ile Gly Ala Ile Pro Asp Ala Val Leu
    210                 215                 220

Leu Phe Leu Lys Asn Lys Lys Asn Leu Gly Ile His Ser Glu Met Ile

225                 230                 235                 240

Ser Asp Gly Val Met Glu Leu Val Lys Ala Gly Val Ile Asn Asn Lys
                245                 250                 255

Lys Lys Thr Leu His Pro Gly Lys Ile Val Val Thr Phe Leu Met Gly
            260                 265                 270

Thr Lys Lys Leu Tyr Asp Phe Val Asn Asn Asn Pro Met Val Glu Thr
        275                 280                 285

Tyr Ser Val Asp Tyr Val Asn Asn Pro Leu Val Ile Met Lys Asn Asp
    290                 295                 300

Asn Met Val Ser Ile Asn Ser Cys Val Gln Val Asp Leu Met Gly Gln
305                 310                 315                 320

Val Cys Ser Glu Ser Ile Gly Leu Lys Gln Ile Ser Gly Val Gly Gly
                325                 330                 335

Gln Val Asp Phe Ile Arg Gly Ala Asn Leu Ser Lys Gly Gly Lys Ala
            340                 345                 350

Ile Ile Ala Ile Pro Ser Thr Ala Gly Lys Gly Lys Val Ser Arg Ile
        355                 360                 365

Thr Pro Leu Leu Asp Thr Gly Ala Ala Val Thr Thr Ser Arg Asn Glu
    370                 375                 380

Val Asp Tyr Val Val Thr Glu Tyr Gly Val Ala His Leu Lys Gly Lys
385                 390                 395                 400

Thr Leu Arg Asn Arg Ala Arg Ala Leu Ile Asn Ile Ala His Pro Lys
                405                 410                 415

Phe Arg Glu Ser Leu Met Asn Glu Phe Lys Lys Arg Phe
            420                 425

<210>  37
<211>  1419
<212>  DNA
<213>  Metallosphaera sedula

<400>  37
atgacggttc tacaggaata tgaaaggatc cacgaggaac ttagaaggga aggttacatt      60

aacacccaat atcccttgaa tccctccgag acgctctgga acaagaagat aatgaccatg     120

aagagggagg agttggagaa ggtaaagtcc ttcaggctca agaggatagt taagtgggca     180

tgggacaacg tgccctttta caggaacttc tggaagtcca agggtttcga accagatcaa     240

atacgcgatt ggaaggatat cgtgaagata ccgattctaa ggaaggacga actcaggaag     300

gacctgtcag ctaaccctcc attcggttcc attatggttc ctgagctagc caagaggatt     360

agatttgtga gtgcaacctc tggttccaca ggtttaccca ccttccaagg ttggggtgcc 420
ctcgaacttg actacttcga ggaggcacaa gctaggtatc tctggacctt cgccggagtt 480
aaacctacga ctgtttacgc aaactacctt aacatgtcag gtttctacag ctggggtcct 540
ccagtggtcg agactgcaat gtggagatgc ggagcgacgg ctattgcggg gggaggtgag 600
acttacttct cgtggaaggc aaggcataac ctcatattca ggctctggaa agtagacgtc 660
ctggctacca ctccctggct tcacaggctc ataggagagg aagcaaaggc ggagggttgg 720
gagtccccgt tcaaggttct tctccttcac ggcggggccg cggccgagaa cacgaagaaa 780
aagttgttcc aagttcatcc caacgcgaag ctagcgatca gcgtgtgggg cacgactgac 840
ggacacatgg ccgttgaagt gcctggccta gatggccagc ttgtgatttg ggaggacatg 900
gagatatttg atatcgtcga tcccaagacc gacgagccag catcaccagg agagagggga 960
gagcttatcg ctacccttct caaccacttt accatgcccc taatcaggta cagtttaggc 1020
gattacgtga agaatgagtt taccacagat ccagatccca cgtatggaat aacccacgcc 1080
aggttcgtgg agccaattcc cggaagagtg gaatggatgt ttaaggtcaa gggcaagtta 1140
ttgctcccaa tatacgtgga ggatgcagtg aacgagatac cagacacaac cggaatgttt 1200
aacgtgataa tctacggtaa tgagatggat aaactgaaga taagggttga gacgaggaga 1260
aacatggtag attctaccta cgactctagg gcaagggaga tactcgcaga gagaataggg 1320
ctcaacaagg atgatgtgga gatagagtgg gtggaaccag gtaagactgc ctggacaggt 1380
tataagttgc aggtgtttct ggaccagaga aagaagtga 1419

<210> 38
<211> 472
<212> PRT
<213> Metallosphaera sedula

<400> 38

Met Thr Val Leu Gln Glu Tyr Glu Arg Ile His Glu Glu Leu Arg Arg
1               5                   10                  15

Glu Gly Tyr Ile Asn Thr Gln Tyr Pro Leu Asn Pro Ser Glu Thr Leu
            20                  25                  30

Trp Asn Lys Lys Ile Met Thr Met Lys Arg Glu Glu Leu Glu Lys Val
        35                  40                  45

Lys Ser Phe Arg Leu Lys Arg Ile Val Lys Trp Ala Trp Asp Asn Val
    50                  55                  60

Pro Phe Tyr Arg Asn Phe Trp Lys Ser Lys Gly Phe Glu Pro Asp Gln
65                  70                  75                  80

Ile Arg Asp Trp Lys Asp Ile Val Lys Ile Pro Ile Leu Arg Lys Asp
                85                  90                  95

Glu Leu Arg Lys Asp Leu Ser Ala Asn Pro Pro Phe Gly Ser Ile Met
            100                 105                 110

Val Pro Glu Leu Ala Lys Arg Ile Arg Phe Val Ser Ala Thr Ser Gly
        115                 120                 125

Ser Thr Gly Leu Pro Thr Phe Gln Gly Trp Gly Ala Leu Glu Leu Asp
    130                 135                 140

Tyr Phe Glu Glu Ala Gln Ala Arg Tyr Leu Trp Thr Phe Ala Gly Val
145                 150                 155                 160

Lys Pro Thr Thr Val Tyr Ala Asn Tyr Leu Asn Met Ser Gly Phe Tyr
                165                 170                 175

Ser Trp Gly Pro Pro Val Val Glu Thr Ala Met Trp Arg Cys Gly Ala
            180                 185                 190

Thr Ala Ile Ala Gly Gly Gly Glu Thr Tyr Phe Ser Trp Lys Ala Arg
        195                 200                 205

His Asn Leu Ile Phe Arg Leu Trp Lys Val Asp Val Leu Ala Thr Thr
    210                 215                 220

Pro Trp Leu His Arg Leu Ile Gly Glu Glu Ala Lys Ala Glu Gly Trp
225                 230                 235                 240

Glu Ser Pro Phe Lys Val Leu Leu Leu His Gly Gly Ala Ala Ala Glu
                245                 250                 255

Asn Thr Lys Lys Lys Leu Phe Gln Val His Pro Asn Ala Lys Leu Ala
            260                 265                 270

Ile Ser Val Trp Gly Thr Thr Asp Gly His Met Ala Val Glu Val Pro
        275                 280                 285

Gly Leu Asp Gly Gln Leu Val Ile Trp Glu Asp Met Glu Ile Phe Asp
    290                 295                 300

Ile Val Asp Pro Lys Thr Asp Glu Pro Ala Ser Pro Gly Glu Arg Gly
305                 310                 315                 320

Glu Leu Ile Ala Thr Leu Leu Asn His Phe Thr Met Pro Leu Ile Arg
                325                 330                 335

Tyr Ser Leu Gly Asp Tyr Val Lys Asn Glu Phe Thr Thr Asp Pro Asp
            340                 345                 350

Pro Thr Tyr Gly Ile Thr His Ala Arg Phe Val Glu Pro Ile Pro Gly
        355                 360                 365

Arg Val Glu Trp Met Phe Lys Val Lys Gly Lys Leu Leu Leu Pro Ile
    370                 375                 380

Tyr Val Glu Asp Ala Val Asn Glu Ile Pro Asp Thr Thr Gly Met Phe
385                 390                 395                 400

Asn Val Ile Ile Tyr Gly Asn Glu Met Asp Lys Leu Lys Ile Arg Val
                405                 410                 415

Glu Thr Arg Arg Asn Met Val Asp Ser Thr Tyr Asp Ser Arg Ala Arg
            420                 425                 430

Glu Ile Leu Ala Glu Arg Ile Gly Leu Asn Lys Asp Asp Val Glu Ile
        435                 440                 445

Glu Trp Val Glu Pro Gly Lys Thr Ala Trp Thr Gly Tyr Lys Leu Gln
    450                 455                 460

Val Phe Leu Asp Gln Arg Lys Lys
465                 470

<210>  39
<211>  1473
<212>  DNA
<213>  Clostridium aminobutyricum

<400>  39
atgttaatga cagcagaaca gtacattgag agtctaagaa agctaaacac aagagtttat      60

atgtttggtg aaaaaatcga gaattgggtg gatcatccaa tgatcagacc ttccatcaac     120

tgcgtagcaa tgacttatga attagctcag gatcctcagt acgctgactt aatgactaca     180

aagtcaaact taataggtaa aactatcaac agatttgcaa atctacacca gagcacagat     240

gaccttagaa aaaaggttaa gatgcagaga cttcttggac agaagaccgc atcatgcttc     300

cagagatgtg taggtatgga cgctttcaat gcagttttct caactacata tgaaatcgac     360

cagaaatatg gaacaaacta tcacaagaac tttactgaat acttaaagta tatacaggaa     420

aatgacctta ttgttgacgg tgcaatgact gaccctaagg gtgacagagg acttgctcca     480

tccgcacaga aggatccaga tcttttcttg agaatcgttg aaaaaagaga agatggtatc     540

gttgtaagag gagctaaggc tcaccagact ggttccatca actcccacga acacatcatc     600

atgcctacaa tcgctatgac agaagctgat aaggattatg cagtatcatt tgcttgtcct     660

tccgatgctg atggtctatt catgatctac ggcagacagt catgtgacac aagaaagatg     720

gaagaaggcg ctgacattga ccttggtaac aagcagttcg gcggacagga agctttagtc     780

gtattcgata acgtatttat tccaaatgac agaatcttcc tttgccaaga atatgatttc     840

gctggcatga tggtagaaag atttgctgga taccacagac agtcatacgg cggatgtaag     900

gttggagtag gcgacgttgt aatcggtgct gctgctttag ctgctgacta caatggagct     960

cagaaggctt ctcacgttaa agataagctt atcgaaatga ctcacttaaa tgaaacttta    1020

tattgctgcg gtattgcttg ttcagcagaa ggttatccaa ctgctgctgg taactatcag 1080

attgaccttc ttcttgcaaa tgtatgtaag cagaacatca ctagattccc ttacgaaatc 1140

gtaagactag ctgaagatat cgctggtgga ttaatggtta ctatgccttc agaagctgac 1200

tttaagtcag aaacagttgt tggtagagat ggcgaaacta ttggagattt ctgcaataag 1260

ttcttcgctg ctgctcctac ttgcacaaca gaagaaagaa tgagagttct tagattctta 1320

gaaaacatct gcttaggtgc atccgctgta ggttacagaa ctgaatccat gcatggtgca 1380

ggttcccctc aggctcagag aatcatgatc gctcgtcagg gcaacatcaa cgctaagaaa 1440

gaattagcta aggcaatcgc tggaattaaa taa 1473

<210> 40
<211> 490
<212> PRT
<213> Clostridium aminobutyricum

<400> 40

Met Leu Met Thr Ala Glu Gln Tyr Ile Glu Ser Leu Arg Lys Leu Asn
1               5                   10                  15

Thr Arg Val Tyr Met Phe Gly Glu Lys Ile Glu Asn Trp Val Asp His
            20                  25                  30

Pro Met Ile Arg Pro Ser Ile Asn Cys Val Ala Met Thr Tyr Glu Leu
        35                  40                  45

Ala Gln Asp Pro Gln Tyr Ala Asp Leu Met Thr Thr Lys Ser Asn Leu
    50                  55                  60

Ile Gly Lys Thr Ile Asn Arg Phe Ala Asn Leu His Gln Ser Thr Asp
65                  70                  75                  80

Asp Leu Arg Lys Lys Val Lys Met Gln Arg Leu Leu Gly Gln Lys Thr
                85                  90                  95

Ala Ser Cys Phe Gln Arg Cys Val Gly Met Asp Ala Phe Asn Ala Val
            100                 105                 110

Phe Ser Thr Thr Tyr Glu Ile Asp Gln Lys Tyr Gly Thr Asn Tyr His
        115                 120                 125

Lys Asn Phe Thr Glu Tyr Leu Lys Tyr Ile Gln Glu Asn Asp Leu Ile
    130                 135                 140

Val Asp Gly Ala Met Thr Asp Pro Lys Gly Asp Arg Gly Leu Ala Pro
145                 150                 155                 160

Ser Ala Gln Lys Asp Pro Asp Leu Phe Leu Arg Ile Val Glu Lys Arg
                165                 170                 175

Glu Asp Gly Ile Val Val Arg Gly Ala Lys Ala His Gln Thr Gly Ser
            180                 185                 190

Ile Asn Ser His Glu His Ile Ile Met Pro Thr Ile Ala Met Thr Glu
        195                 200                 205

Ala Asp Lys Asp Tyr Ala Val Ser Phe Ala Cys Pro Ser Asp Ala Asp
    210                 215                 220

Gly Leu Phe Met Ile Tyr Gly Arg Gln Ser Cys Asp Thr Arg Lys Met
225                 230                 235                 240

Glu Glu Gly Ala Asp Ile Asp Leu Gly Asn Lys Gln Phe Gly Gly Gln
                245                 250                 255

Glu Ala Leu Val Val Phe Asp Asn Val Phe Ile Pro Asn Asp Arg Ile
            260                 265                 270

Phe Leu Cys Gln Glu Tyr Asp Phe Ala Gly Met Met Val Glu Arg Phe
        275                 280                 285

Ala Gly Tyr His Arg Gln Ser Tyr Gly Gly Cys Lys Val Gly Val Gly
    290                 295                 300

Asp Val Val Ile Gly Ala Ala Ala Leu Ala Ala Asp Tyr Asn Gly Ala
305                 310                 315                 320

Gln Lys Ala Ser His Val Lys Asp Lys Leu Ile Glu Met Thr His Leu
                325                 330                 335

Asn Glu Thr Leu Tyr Cys Cys Gly Ile Ala Cys Ser Ala Glu Gly Tyr
            340                 345                 350

Pro Thr Ala Ala Gly Asn Tyr Gln Ile Asp Leu Leu Leu Ala Asn Val
        355                 360                 365

Cys Lys Gln Asn Ile Thr Arg Phe Pro Tyr Glu Ile Val Arg Leu Ala
    370                 375                 380

Glu Asp Ile Ala Gly Gly Leu Met Val Thr Met Pro Ser Glu Ala Asp
385                 390                 395                 400

Phe Lys Ser Glu Thr Val Val Gly Arg Asp Gly Glu Thr Ile Gly Asp
                405                 410                 415

Phe Cys Asn Lys Phe Phe Ala Ala Ala Pro Thr Cys Thr Thr Glu Glu
            420                 425                 430

Arg Met Arg Val Leu Arg Phe Leu Glu Asn Ile Cys Leu Gly Ala Ser
        435                 440                 445

Ala Val Gly Tyr Arg Thr Glu Ser Met His Gly Ala Gly Ser Pro Gln
    450                 455                 460

Ala Gln Arg Ile Met Ile Ala Arg Gln Gly Asn Ile Asn Ala Lys Lys
465                 470                 475                 480

Glu Leu Ala Lys Ala Ile Ala Gly Ile Lys
                485                 490

<210>  41
<211>  663
<212>  DNA
<213>  Escherichia coli

<400>  41
atgaaaacaa aattgatgac attacaagac gccaccggct tctttcgtga cggcatgacc      60

atcatggtgg gcggatttat ggggattggc actccatccc gcctggttga agcattactg     120

gaatctggtg ttcgcgacct gacattgata gccaatgata ccgcgtttgt tgataccggc     180

atcggtccgc tcatcgtcaa tggtcgagtc cgcaaagtga ttgcttcaca tatcggcacc     240

aacccggaaa caggtcggcg catgatatct ggtgagatgg acgtcgttct ggtgccgcaa     300

ggtacgctaa tcgagcaaat tcgctgtggt ggagctggac ttggtggttt tctcacccca     360

acgggtgtcg gcaccgtcgt agaggaaggc aaacagacac tgacactcga cggtaaaacc     420

tggctgctcg aacgcccact gcgcgccgac ctggcgctaa ttcgcgctca tcgttgcgac     480

acacttggca acctgaccta tcaacttagc gcccgcaact ttaaccccct gatagccctt     540

gcggctgata tcacgctggt agagccagat gaactggtcg aaaccggcga gctgcaacct     600

gaccatattg tcacccctgg tgccgttatc gaccacatca tcgtttcaca ggagagcaaa     660

taa                                                                   663

<210>  42
<211>  220
<212>  PRT
<213>  Escherichia coli

<400>  42

Met Lys Thr Lys Leu Met Thr Leu Gln Asp Ala Thr Gly Phe Phe Arg
1               5                   10                  15

Asp Gly Met Thr Ile Met Val Gly Gly Phe Met Gly Ile Gly Thr Pro
            20                  25                  30

Ser Arg Leu Val Glu Ala Leu Leu Glu Ser Gly Val Arg Asp Leu Thr
        35                  40                  45

Leu Ile Ala Asn Asp Thr Ala Phe Val Asp Thr Gly Ile Gly Pro Leu
    50                  55                  60

Ile Val Asn Gly Arg Val Arg Lys Val Ile Ala Ser His Ile Gly Thr

65                  70                  75                  80

Asn Pro Glu Thr Gly Arg Arg Met Ile Ser Gly Glu Met Asp Val Val
                85                  90                  95

Leu Val Pro Gln Gly Thr Leu Ile Glu Gln Ile Arg Cys Gly Gly Ala
            100                 105                 110

Gly Leu Gly Gly Phe Leu Thr Pro Thr Gly Val Gly Thr Val Val Glu
        115                 120                 125

Glu Gly Lys Gln Thr Leu Thr Leu Asp Gly Lys Thr Trp Leu Leu Glu
    130                 135                 140

Arg Pro Leu Arg Ala Asp Leu Ala Leu Ile Arg Ala His Arg Cys Asp
145                 150                 155                 160

Thr Leu Gly Asn Leu Thr Tyr Gln Leu Ser Ala Arg Asn Phe Asn Pro
                165                 170                 175

Leu Ile Ala Leu Ala Ala Asp Ile Thr Leu Val Glu Pro Asp Glu Leu
            180                 185                 190

Val Glu Thr Gly Glu Leu Gln Pro Asp His Ile Val Thr Pro Gly Ala
        195                 200                 205

Val Ile Asp His Ile Ile Val Ser Gln Glu Ser Lys
    210                 215                 220

<210>  43
<211>  651
<212>  DNA
<213>  Escherichia coli

<400>  43
atggatgcga aacaacgtat tgcgcgccgt gtggcgcaag agcttcgtga tggtgacatc      60

gttaacttag ggatcggttt acccacaatg gtcgccaatt atttaccgga gggtattcat     120

atcactctgc aatcggaaaa cggcttcctc ggtttaggcc cggtcacgac agcgcatcca     180

gatctggtga acgctggcgg gcaaccgtgc ggtgttttac ccggtgcagc catgtttgat     240

agcgccatgt catttgcgct aatccgtggc ggtcatattg atgcctgcgt gctcggcggt     300

ttgcaagtag acgaagaagc aaacctcgcg aactgggtag tgcctgggaa aatggtgccc     360

ggtatgggtg gcgcgatgga tctggtgacc gggtcgcgca aagtgatcat cgccatggaa     420

cattgcgcca aagatggttc agcaaaaatt ttgcgccgct gcaccatgcc actcactgcg     480

caacatgcgg tgcatatgct ggttactgaa ctggctgtct ttcgttttat tgacggcaaa     540

atgtggctca ccgaaattgc cgacgggtgt gatttagcca ccgtgcgtgc caaaacagaa     600

gctcggtttg aagtcgccgc cgatctgaat acgcaacggg gtgatttatg a              651

<210> 44
<211> 216
<212> PRT
<213> Escherichia coli

<400> 44

Met Asp Ala Lys Gln Arg Ile Ala Arg Arg Val Ala Gln Glu Leu Arg
1               5                   10                  15

Asp Gly Asp Ile Val Asn Leu Gly Ile Gly Leu Pro Thr Met Val Ala
            20                  25                  30

Asn Tyr Leu Pro Glu Gly Ile His Ile Thr Leu Gln Ser Glu Asn Gly
        35                  40                  45

Phe Leu Gly Leu Gly Pro Val Thr Thr Ala His Pro Asp Leu Val Asn
    50                  55                  60

Ala Gly Gly Gln Pro Cys Gly Val Leu Pro Gly Ala Ala Met Phe Asp
65                  70                  75                  80

Ser Ala Met Ser Phe Ala Leu Ile Arg Gly Gly His Ile Asp Ala Cys
                85                  90                  95

Val Leu Gly Gly Leu Gln Val Asp Glu Glu Ala Asn Leu Ala Asn Trp
            100                 105                 110

Val Val Pro Gly Lys Met Val Pro Gly Met Gly Gly Ala Met Asp Leu
        115                 120                 125

Val Thr Gly Ser Arg Lys Val Ile Ile Ala Met Glu His Cys Ala Lys
    130                 135                 140

Asp Gly Ser Ala Lys Ile Leu Arg Arg Cys Thr Met Pro Leu Thr Ala
145                 150                 155                 160

Gln His Ala Val His Met Leu Val Thr Glu Leu Ala Val Phe Arg Phe
                165                 170                 175

Ile Asp Gly Lys Met Trp Leu Thr Glu Ile Ala Asp Gly Cys Asp Leu
            180                 185                 190

Ala Thr Val Arg Ala Lys Thr Glu Ala Arg Phe Glu Val Ala Ala Asp
        195                 200                 205

Leu Asn Thr Gln Arg Gly Asp Leu
    210                 215


<210> 45
<211> 1197
<212> DNA
<213> Clostridium acetobutylicum

<400> 45
atgatagtaa aagcaaagtt tgtaaaagga tttatcagag atgtacatcc ttatggttgc 60

agaagggaag tactaaatca aatagattat tgtaagaagg ctattgggtt taggggacca 120

aagaaggttt taattgttgg agcctcatct gggtttggtc ttgctactag aatttcagtt 180

gcatttggag gtccagaagc tcacacaatt ggagtatcct atgaaacagg agctacagat 240

agaagaatag gaacagcggg atggtataat aacatatttt ttaaagaatt tgctaaaaaa 300

aaaggattag ttgcaaaaaa cttcattgag gatgcctttt ctaatgaaac caaagataaa 360

gttattaagt atataaagga tgaatttggt aaaatagatt tatttgttta tagtttagct 420

gcgcctagga gaaaggacta taaaactgga aatgtttata cttcaagaat aaaaacaatt 480

ttaggagatt ttgagggacc gactattgat gttgaaagag acgagattac tttaaaaaag 540

gttagtagtg ctagcattga agaaattgaa gaaactagaa aggtaatggg tggagaggat 600

tggcaagagt ggtgtgaaga gctgctttat gaagattgtt tttcggataa agcaactacc 660

atagcatact cgtatatagg atccccaaga acctacaaga tatatagaga aggtactata 720

ggaatagcta aaaaggatct tgaagataag gctaagctta taaatgaaaa acttaacaga 780

gttataggtg gtagagcctt tgtgtctgtg aataaagcat tagttacaaa agcaagtgca 840

tatattccaa cttttcctct ttatgcagct attttatata aggtcatgaa agaaaaaaat 900

attcatgaaa attgtattat gcaaattgag agaatgtttt ctgaaaaaat atattcaaat 960

gaaaaaatac aatttgatga caagggaaga ttaaggatgg acgatttaga gcttagaaaa 1020

gacgttcaag acgaagttga tagaatatgg agtaatatta ctcctgaaaa ttttaaggaa 1080

ttatctgatt ataagggata caaaaaagaa ttcatgaact taaacggttt tgatctagat 1140

ggggttgatt atagtaaaga cctggatata gaattattaa gaaaattaga accttaa 1197

<210> 46
<211> 398
<212> PRT
<213> Clostridium acetobutylicum

<400> 46

Met Ile Val Lys Ala Lys Phe Val Lys Gly Phe Ile Arg Asp Val His
1               5                   10                  15

Pro Tyr Gly Cys Arg Arg Glu Val Leu Asn Gln Ile Asp Tyr Cys Lys
            20                  25                  30

Lys Ala Ile Gly Phe Arg Gly Pro Lys Lys Val Leu Ile Val Gly Ala
        35                  40                  45

Ser Ser Gly Phe Gly Leu Ala Thr Arg Ile Ser Val Ala Phe Gly Gly
    50                  55                  60

Pro Glu Ala His Thr Ile Gly Val Ser Tyr Glu Thr Gly Ala Thr Asp
65                  70                  75                  80

Arg Arg Ile Gly Thr Ala Gly Trp Tyr Asn Asn Ile Phe Phe Lys Glu
                85                  90                  95

Phe Ala Lys Lys Lys Gly Leu Val Ala Lys Asn Phe Ile Glu Asp Ala
            100                 105                 110

Phe Ser Asn Glu Thr Lys Asp Lys Val Ile Lys Tyr Ile Lys Asp Glu
        115                 120                 125

Phe Gly Lys Ile Asp Leu Phe Val Tyr Ser Leu Ala Ala Pro Arg Arg
    130                 135                 140

Lys Asp Tyr Lys Thr Gly Asn Val Tyr Thr Ser Arg Ile Lys Thr Ile
145                 150                 155                 160

Leu Gly Asp Phe Glu Gly Pro Thr Ile Asp Val Glu Arg Asp Glu Ile
                165                 170                 175

Thr Leu Lys Lys Val Ser Ser Ala Ser Ile Glu Glu Ile Glu Glu Thr
            180                 185                 190

Arg Lys Val Met Gly Gly Glu Asp Trp Gln Glu Trp Cys Glu Glu Leu
        195                 200                 205

Leu Tyr Glu Asp Cys Phe Ser Asp Lys Ala Thr Thr Ile Ala Tyr Ser
    210                 215                 220

Tyr Ile Gly Ser Pro Arg Thr Tyr Lys Ile Tyr Arg Glu Gly Thr Ile
225                 230                 235                 240

Gly Ile Ala Lys Lys Asp Leu Glu Asp Lys Ala Lys Leu Ile Asn Glu
                245                 250                 255

Lys Leu Asn Arg Val Ile Gly Gly Arg Ala Phe Val Ser Val Asn Lys
            260                 265                 270

Ala Leu Val Thr Lys Ala Ser Ala Tyr Ile Pro Thr Phe Pro Leu Tyr
        275                 280                 285

Ala Ala Ile Leu Tyr Lys Val Met Lys Glu Lys Asn Ile His Glu Asn
    290                 295                 300

Cys Ile Met Gln Ile Glu Arg Met Phe Ser Glu Lys Ile Tyr Ser Asn
305                 310                 315                 320

Glu Lys Ile Gln Phe Asp Asp Lys Gly Arg Leu Arg Met Asp Asp Leu
                325                 330                 335

Glu Leu Arg Lys Asp Val Gln Asp Glu Val Asp Arg Ile Trp Ser Asn
            340                 345                 350

Ile Thr Pro Glu Asn Phe Lys Glu Leu Ser Asp Tyr Lys Gly Tyr Lys
        355                 360                 365

Lys Glu Phe Met Asn Leu Asn Gly Phe Asp Leu Asp Gly Val Asp Tyr
    370                 375                 380

Ser Lys Asp Leu Asp Ile Glu Leu Leu Arg Lys Leu Glu Pro
385                 390                 395

<210>  47
<211>  1509
<212>  DNA
<213>  Corynebacterium glutamicum

<400>  47
atgtctgatc gcattgcttc agaaaagctg cgctccaagc tcatgtccgc cgacgaggcg      60

gcacagtttg ttaaccacgg tgacaaggtt ggtttctccg gcttcaccgg cgctggctac     120

ccaaaggcac tgcctacggc aatcgctaac cgggctaaag aagcacacgg tgcaggcaac     180

gactacgcaa tcgacctgtt cactggcgca tcgaccgccc ctgactgcga tggcgtactt     240

gcagaagctg acgctatccg ctggcgcatg ccatacgcat ctgatccaat catgcgtaac     300

aagatcaact ccggctccat gggatactcc gatatccacc tgtcccactc cggccagcag     360

gttgaagagg gcttcttcgg ccagctcaac gtagctgtca ttgaaatcac ccgcatcact     420

gaagagggct acatcatccc ttcttcctcc gtgggtaaca acgttgagtg gctcaacgct     480

gcagagaagg tcatcctcga ggttaactct tggcagtctg aagacctcga aggtatgcac     540

gacatctggt ctgttcctgc cctgccaaac cgcattgccg tgccaatcaa caagccaggc     600

gaccgcatcg gtaagaccta catcgagttc gacaccgaca aggttgttgc tgttgttgag     660

accaacaccg cagaccgcaa cgcaccattc aagcctgtcg acgacatctc taagaagatc     720

gctggcaact tcctcgactt cctggaaagc gaagttgctg caggtcgcct gtcctacgac     780

ggctacatca tgcagtccgg cgtgggcaac gtgccaaacg cggtgatggc aggcctgctg     840

gaatccaagt ttgagaacat ccaggcctac accgaagtta tccaggacgg catggtggac     900

ctcatcgacg ccggcaagat gaccgttgca tccgcaactt ccttctccct gtctcctgag     960

tacgcagaga agatgaacaa cgaggctaag cgttaccgcg agtccattat cctgcgccca    1020

cagcagatct ctaaccaccc agaggtcatc cgccgcgttg gcctgatcgc caccaacggt    1080

ctcatcgagg ctgacattta cggcaacgtc aactccacca acgtttctgg ctcccgcgtc    1140

atgaacggca tcggcggctc cggcgacttc acccgtaacg gctacatctc cagcttcatc    1200

accccttcag aggcaaaggg cggcgcaatc tctgcgatcg ttcctttcgc atcccacatc    1260

gaccacaccg agcacgatgt catggttgtt atctctgagt acggttacgc agaccttcgt    1320

ggtctggctc cacgtgagcg cgttgccaag atgatcggcc tggctcaccc tgattaccgc    1380

ccactgctcg aggagtacta cgctcgcgca acctccggtg acaacaagta catgcagacc    1440

cctcatgatc ttgcaaccgc gtttgatttc cacatcaacc tggctaagaa cggctccatg    1500

aaggcataa                                                          1509

<210>  48
<211>  502
<212>  PRT
<213>  Corynebacterium glutamicum

<400>  48

Met Ser Asp Arg Ile Ala Ser Glu Lys Leu Arg Ser Lys Leu Met Ser
1               5                   10                  15

Ala Asp Glu Ala Ala Gln Phe Val Asn His Gly Asp Lys Val Gly Phe
            20                  25                  30

Ser Gly Phe Thr Gly Ala Gly Tyr Pro Lys Ala Leu Pro Thr Ala Ile
        35                  40                  45

Ala Asn Arg Ala Lys Glu Ala His Gly Ala Gly Asn Asp Tyr Ala Ile
    50                  55                  60

Asp Leu Phe Thr Gly Ala Ser Thr Ala Pro Asp Cys Asp Gly Val Leu
65                  70                  75                  80

Ala Glu Ala Asp Ala Ile Arg Trp Arg Met Pro Tyr Ala Ser Asp Pro
                85                  90                  95

Ile Met Arg Asn Lys Ile Asn Ser Gly Ser Met Gly Tyr Ser Asp Ile
            100                 105                 110

His Leu Ser His Ser Gly Gln Gln Val Glu Glu Gly Phe Phe Gly Gln
        115                 120                 125

Leu Asn Val Ala Val Ile Glu Ile Thr Arg Ile Thr Glu Glu Gly Tyr
    130                 135                 140

Ile Ile Pro Ser Ser Ser Val Gly Asn Asn Val Glu Trp Leu Asn Ala
145                 150                 155                 160

Ala Glu Lys Val Ile Leu Glu Val Asn Ser Trp Gln Ser Glu Asp Leu
                165                 170                 175

Glu Gly Met His Asp Ile Trp Ser Val Pro Ala Leu Pro Asn Arg Ile
            180                 185                 190

Ala Val Pro Ile Asn Lys Pro Gly Asp Arg Ile Gly Lys Thr Tyr Ile
        195                 200                 205

Glu Phe Asp Thr Asp Lys Val Val Ala Val Val Glu Thr Asn Thr Ala
    210                 215                 220

Asp Arg Asn Ala Pro Phe Lys Pro Val Asp Asp Ile Ser Lys Lys Ile

225                 230                 235                 240

Ala Gly Asn Phe Leu Asp Phe Leu Glu Ser Glu Val Ala Ala Gly Arg
                245                 250                 255

Leu Ser Tyr Asp Gly Tyr Ile Met Gln Ser Gly Val Gly Asn Val Pro
            260                 265                 270

Asn Ala Val Met Ala Gly Leu Leu Glu Ser Lys Phe Glu Asn Ile Gln
        275                 280                 285

Ala Tyr Thr Glu Val Ile Gln Asp Gly Met Val Asp Leu Ile Asp Ala
    290                 295                 300

Gly Lys Met Thr Val Ala Ser Ala Thr Ser Phe Ser Leu Ser Pro Glu
305                 310                 315                 320

Tyr Ala Glu Lys Met Asn Asn Glu Ala Lys Arg Tyr Arg Glu Ser Ile
                325                 330                 335

Ile Leu Arg Pro Gln Gln Ile Ser Asn His Pro Glu Val Ile Arg Arg
            340                 345                 350

Val Gly Leu Ile Ala Thr Asn Gly Leu Ile Glu Ala Asp Ile Tyr Gly
        355                 360                 365

Asn Val Asn Ser Thr Asn Val Ser Gly Ser Arg Val Met Asn Gly Ile
    370                 375                 380

Gly Gly Ser Gly Asp Phe Thr Arg Asn Gly Tyr Ile Ser Ser Phe Ile
385                 390                 395                 400

Thr Pro Ser Glu Ala Lys Gly Gly Ala Ile Ser Ala Ile Val Pro Phe
                405                 410                 415

Ala Ser His Ile Asp His Thr Glu His Asp Val Met Val Val Ile Ser
            420                 425                 430

Glu Tyr Gly Tyr Ala Asp Leu Arg Gly Leu Ala Pro Arg Glu Arg Val
        435                 440                 445

Ala Lys Met Ile Gly Leu Ala His Pro Asp Tyr Arg Pro Leu Leu Glu
    450                 455                 460

Glu Tyr Tyr Ala Arg Ala Thr Ser Gly Asp Asn Lys Tyr Met Gln Thr
465                 470                 475                 480

Pro His Asp Leu Ala Thr Ala Phe Asp Phe His Ile Asn Leu Ala Lys
                485                 490                 495

Asn Gly Ser Met Lys Ala

            500

<210> 49
<211> 2589
<212> DNA
<213> Clostridium acetobutylicum

<400> 49
atgaaagtca caacagtaaa ggaattagat gaaaaactca aggtaattaa agaagctcaa 60

aaaaaattct cttgttactc gcaagaaatg gttgatgaaa tctttagaaa tgcagcaatg 120

gcagcaatcg acgcaaggat agagctagca aaagcagctg ttttggaaac cggtatgggc 180

ttagttgaag acaaggttat aaaaaatcat tttgcaggcg aatacatcta taacaaatat 240

aaggatgaaa aaacctgcgg tataattgaa cgaaatgaac cctacggaat tacaaaaata 300

gcagaaccta taggagttgt agctgctata atccctgtaa caaaccccac atcaacaaca 360

atatttaaat ccttaatatc ccttaaaact agaaatggaa ttttcttttc gcctcaccca 420

agggcaaaaa aatccacaat actagcagct aaaacaatac ttgatgcagc cgttaagagt 480

ggtgccccgg aaaatataat aggttggata gatgaacctt caattgaact aactcaatat 540

ttaatgcaaa aagcagatat aacccttgca actggtggtc cctcactagt taaatctgct 600

tattcttccg gaaaaccagc aataggtgtt ggtccgggta acaccccagt aataattgat 660

gaatctgctc atataaaaat ggcagtaagt tcaattatat tatccaaaac ctatgataat 720

ggtgttatat gtgcttctga acaatctgta atagtcttaa aatccatata taacaaggta 780

aaagatgagt tccaagaaag aggagcttat ataataaaga aaaacgaatt ggataaagtc 840

cgtgaagtga tttttaaaga tggatccgta aaccctaaaa tagtcggaca gtcagcttat 900

actatagcag ctatggctgg cataaaagta cctaaaacca caagaatatt aataggagaa 960

gttacctcct taggtgaaga agaacctttt gcccacgaaa aactatctcc tgttttggct 1020

atgtatgagg ctgacaattt tgatgatgct ttaaaaaaag cagtaactct aataaactta 1080

ggaggcctcg gccatacctc aggaatatat gcagatgaaa taaaagcacg agataaaata 1140

gatagattta gtagtgccat gaaaaccgta agaacctttg taaatatccc aacctcacaa 1200

ggtgcaagtg gagatctata taattttaga ataccacctt ctttcacgct tggctgcgga 1260

ttttggggag gaaattctgt ttccgagaat gttggtccaa aacatctttt gaatattaaa 1320

accgtagctg aaaggagaga aaacatgctt tggtttagag ttccacataa agtatatttt 1380

aagttcggtt gtcttcaatt tgctttaaaa gatttaaaag atctaaagaa aaaaagagcc 1440

tttatagtta ctgatagtga cccctataat ttaaactatg ttgattcaat aataaaaata 1500

cttgagcacc tagatattga ttttaaagta tttaataagg ttggaagaga agctgatctt 1560

aaaaccataa aaaaagcaac tgaagaaatg tcctccttta tgccagacac tataatagct 1620

ttaggtggta cccctgaaat gagctctgca aagctaatgt gggtactata tgaacatcca 1680

gaagtaaaat ttgaagatct tgcaataaaa tttatggaca taagaaagag aatatatact 1740

ttcccaaaac tcggtaaaaa ggctatgtta gttgcaatta caacttctgc tggttccggt 1800

tctgaggtta ctccttttgc tttagtaact gacaataaca ctggaaataa gtacatgtta 1860

gcagattatg aaatgacacc aaatatggca attgtagatg cagaacttat gatgaaaatg 1920

ccaaagggat taaccgctta ttcaggtata gatgcactag taaatagtat agaagcatac 1980

acatccgtat atgcttcaga atacacaaac ggactagcac tagaggcaat acgattaata 2040

tttaaatatt tgcctgaggc ttacaaaaac ggaagaacca atgaaaaagc aagagagaaa 2100

atggctcacg cttcaactat ggcaggtatg gcatccgcta atgcatttct aggtctatgt 2160

cattccatgg caataaaatt aagttcagaa cacaatattc ctagtggcat tgccaatgca 2220

ttactaatag aagaagtaat aaaatttaac gcagttgata atcctgtaaa acaagcccct 2280

tgcccacaat ataagtatcc aaacaccata tttagatatg ctcgaattgc agattatata 2340

aagcttggag gaaatactga tgaggaaaag gtagatctct taattaacaa aatacatgaa 2400

ctaaaaaaag ctttaaatat accaacttca ataaaggatg caggtgtttt ggaggaaaac 2460

ttctattcct cccttgatag aatatctgaa cttgcactag atgatcaatg cacaggcgct 2520

aatcctagat ttcctcttac aagtgagata aaagaaatgt atataaattg ttttaaaaaa 2580

caaccttaa 2589

<210> 50
<211> 862
<212> PRT
<213> Clostridium acetobutylicum

<400> 50

Met Lys Val Thr Thr Val Lys Glu Leu Asp Glu Lys Leu Lys Val Ile
1               5                   10                  15

Lys Glu Ala Gln Lys Lys Phe Ser Cys Tyr Ser Gln Glu Met Val Asp
            20                  25                  30

Glu Ile Phe Arg Asn Ala Ala Met Ala Ala Ile Asp Ala Arg Ile Glu
        35                  40                  45

Leu Ala Lys Ala Ala Val Leu Glu Thr Gly Met Gly Leu Val Glu Asp
    50                  55                  60

Lys Val Ile Lys Asn His Phe Ala Gly Glu Tyr Ile Tyr Asn Lys Tyr
65                  70                  75                  80

Lys Asp Glu Lys Thr Cys Gly Ile Ile Glu Arg Asn Glu Pro Tyr Gly
                85                  90                  95

Ile Thr Lys Ile Ala Glu Pro Ile Gly Val Val Ala Ala Ile Ile Pro
            100                 105                 110

Val Thr Asn Pro Thr Ser Thr Thr Ile Phe Lys Ser Leu Ile Ser Leu
        115                 120                 125

Lys Thr Arg Asn Gly Ile Phe Phe Ser Pro His Pro Arg Ala Lys Lys
    130                 135                 140

Ser Thr Ile Leu Ala Ala Lys Thr Ile Leu Asp Ala Ala Val Lys Ser
145                 150                 155                 160

Gly Ala Pro Glu Asn Ile Ile Gly Trp Ile Asp Glu Pro Ser Ile Glu
                165                 170                 175

Leu Thr Gln Tyr Leu Met Gln Lys Ala Asp Ile Thr Leu Ala Thr Gly
            180                 185                 190

Gly Pro Ser Leu Val Lys Ser Ala Tyr Ser Ser Gly Lys Pro Ala Ile
        195                 200                 205

Gly Val Gly Pro Gly Asn Thr Pro Val Ile Ile Asp Glu Ser Ala His
    210                 215                 220

Ile Lys Met Ala Val Ser Ser Ile Ile Leu Ser Lys Thr Tyr Asp Asn
225                 230                 235                 240

Gly Val Ile Cys Ala Ser Glu Gln Ser Val Ile Val Leu Lys Ser Ile
                245                 250                 255

Tyr Asn Lys Val Lys Asp Glu Phe Gln Glu Arg Gly Ala Tyr Ile Ile
            260                 265                 270

Lys Lys Asn Glu Leu Asp Lys Val Arg Glu Val Ile Phe Lys Asp Gly
        275                 280                 285

Ser Val Asn Pro Lys Ile Val Gly Gln Ser Ala Tyr Thr Ile Ala Ala
    290                 295                 300

Met Ala Gly Ile Lys Val Pro Lys Thr Thr Arg Ile Leu Ile Gly Glu
305                 310                 315                 320

Val Thr Ser Leu Gly Glu Glu Glu Pro Phe Ala His Glu Lys Leu Ser
                325                 330                 335

Pro Val Leu Ala Met Tyr Glu Ala Asp Asn Phe Asp Asp Ala Leu Lys
            340                 345                 350

Lys Ala Val Thr Leu Ile Asn Leu Gly Gly Leu Gly His Thr Ser Gly
        355                 360                 365

Ile Tyr Ala Asp Glu Ile Lys Ala Arg Asp Lys Ile Asp Arg Phe Ser
    370                 375                 380

Ser Ala Met Lys Thr Val Arg Thr Phe Val Asn Ile Pro Thr Ser Gln
385                 390                 395                 400

Gly Ala Ser Gly Asp Leu Tyr Asn Phe Arg Ile Pro Pro Ser Phe Thr
                405                 410                 415

Leu Gly Cys Gly Phe Trp Gly Gly Asn Ser Val Ser Glu Asn Val Gly
            420                 425                 430

Pro Lys His Leu Leu Asn Ile Lys Thr Val Ala Glu Arg Arg Glu Asn
        435                 440                 445

Met Leu Trp Phe Arg Val Pro His Lys Val Tyr Phe Lys Phe Gly Cys
    450                 455                 460

Leu Gln Phe Ala Leu Lys Asp Leu Lys Asp Leu Lys Lys Lys Arg Ala
465                 470                 475                 480

Phe Ile Val Thr Asp Ser Asp Pro Tyr Asn Leu Asn Tyr Val Asp Ser
                485                 490                 495

Ile Ile Lys Ile Leu Glu His Leu Asp Ile Asp Phe Lys Val Phe Asn
            500                 505                 510

Lys Val Gly Arg Glu Ala Asp Leu Lys Thr Ile Lys Lys Ala Thr Glu
        515                 520                 525

Glu Met Ser Ser Phe Met Pro Asp Thr Ile Ile Ala Leu Gly Gly Thr
    530                 535                 540

Pro Glu Met Ser Ser Ala Lys Leu Met Trp Val Leu Tyr Glu His Pro
545                 550                 555                 560

Glu Val Lys Phe Glu Asp Leu Ala Ile Lys Phe Met Asp Ile Arg Lys
                565                 570                 575

Arg Ile Tyr Thr Phe Pro Lys Leu Gly Lys Lys Ala Met Leu Val Ala
            580                 585                 590

Ile Thr Thr Ser Ala Gly Ser Gly Ser Glu Val Thr Pro Phe Ala Leu
        595                 600                 605

Val Thr Asp Asn Asn Thr Gly Asn Lys Tyr Met Leu Ala Asp Tyr Glu
    610                 615                 620

Met Thr Pro Asn Met Ala Ile Val Asp Ala Glu Leu Met Met Lys Met
625                 630                 635                 640

Pro Lys Gly Leu Thr Ala Tyr Ser Gly Ile Asp Ala Leu Val Asn Ser
                645                 650                 655

Ile Glu Ala Tyr Thr Ser Val Tyr Ala Ser Glu Tyr Thr Asn Gly Leu
            660                 665                 670

Ala Leu Glu Ala Ile Arg Leu Ile Phe Lys Tyr Leu Pro Glu Ala Tyr
        675                 680                 685

Lys Asn Gly Arg Thr Asn Glu Lys Ala Arg Glu Lys Met Ala His Ala
    690                 695                 700

Ser Thr Met Ala Gly Met Ala Ser Ala Asn Ala Phe Leu Gly Leu Cys
705                 710                 715                 720

His Ser Met Ala Ile Lys Leu Ser Ser Glu His Asn Ile Pro Ser Gly
                725                 730                 735

Ile Ala Asn Ala Leu Leu Ile Glu Glu Val Ile Lys Phe Asn Ala Val
            740                 745                 750

Asp Asn Pro Val Lys Gln Ala Pro Cys Pro Gln Tyr Lys Tyr Pro Asn
        755                 760                 765

Thr Ile Phe Arg Tyr Ala Arg Ile Ala Asp Tyr Ile Lys Leu Gly Gly
    770                 775                 780

Asn Thr Asp Glu Glu Lys Val Asp Leu Leu Ile Asn Lys Ile His Glu
785                 790                 795                 800

Leu Lys Lys Ala Leu Asn Ile Pro Thr Ser Ile Lys Asp Ala Gly Val
                805                 810                 815

Leu Glu Glu Asn Phe Tyr Ser Ser Leu Asp Arg Ile Ser Glu Leu Ala
            820                 825                 830

Leu Asp Asp Gln Cys Thr Gly Ala Asn Pro Arg Phe Pro Leu Thr Ser
        835                 840                 845

Glu Ile Lys Glu Met Tyr Ile Asn Cys Phe Lys Lys Gln Pro
    850                 855                 860

<210> 51
<211> 2577
<212> DNA
<213> Clostridium acetobutylicum

<400> 51
atgaaagtta caaatcaaaa agaactaaaa caaaagctaa atgaattgag agaagcgcaa 60

aagaagtttg caacctatac tcaagagcaa gttgataaaa tttttaaaca atgtgccata 120

gccgcagcta aagaaagaat aaacttagct aaattagcag tagaagaaac aggaataggt 180

cttgtagaag ataaaattat aaaaaatcat tttgcagcag aatatatata caataaatat 240

aaaaatgaaa aaacttgtgg cataatagac catgacgatt ctttaggcat aacaaaggtt 300

gctgaaccaa ttggaattgt tgcagccata gttcctacta ctaatccaac ttccacagca 360

attttcaaat cattaatttc tttaaaaaca agaaacgcaa tattcttttc accacatcca 420

cgtgcaaaaa aatctacaat tgctgcagca aaattaattt tagatgcagc tgttaaagca 480
ggagcaccta aaaatataat aggctggata gatgagccat caatagaact ttctcaagat 540
ttgatgagtg aagctgatat aatattagca acaggaggtc cttcaatggt taaagcggcc 600
tattcatctg gaaaacctgc aattggtgtt ggagcaggaa atacaccagc aataatagat 660
gagagtgcag atatagatat ggcagtaagc tccataattt tatcaaagac ttatgacaat 720
ggagtaatat gcgcttctga acaatcaata ttagttatga attcaatata cgaaaaagtt 780
aaagaggaat ttgtaaaacg aggatcatat atactcaatc aaaatgaaat agctaaaata 840
aaagaaacta tgtttaaaaa tggagctatt aatgctgaca tagttggaaa atctgcttat 900
ataattgcta aaatggcagg aattgaagtt cctcaaacta caaagatact tataggcgaa 960
gtacaatctg ttgaaaaaag cgagctgttc tcacatgaaa aactatcacc agtacttgca 1020
atgtataaag ttaaggattt tgatgaagct ctaaaaaagg cacaaaggct aatagaatta 1080
ggtggaagtg gacacacgtc atctttatat atagattcac aaaacaataa ggataaagtt 1140
aaagaatttg gattagcaat gaaaacttca aggacattta ttaacatgcc ttcttcacag 1200
ggagcaagcg gagatttata caattttgcg atagcaccat catttactct tggatgcggc 1260
acttggggag gaaactctgt atcgcaaaat gtagagccta aacatttatt aaatattaaa 1320
agtgttgctg aaagaaggga aaatatgctt tggtttaaag tgccacaaaa aatatatttt 1380
aaatatggat gtcttagatt tgcattaaaa gaattaaaag atatgaataa gaaaagagcc 1440
tttatagtaa cagataaaga tctttttaaa cttggatatg ttaataaaat aacaaaggta 1500
ctagatgaga tagatattaa atacagtata tttacagata ttaaatctga tccaactatt 1560
gattcagtaa aaaaaggtgc taaagaaatg cttaactttg aacctgatac tataatctct 1620
attggtggtg gatcgccaat ggatgcagca aaggttatgc acttgttata tgaatatcca 1680
gaagcagaaa ttgaaaatct agctataaac tttatggata taagaaagag aatatgcaat 1740
ttccctaaat taggtacaaa ggcgatttca gtagctattc ctacaactgc tggtaccggt 1800
tcagaggcaa caccttttgc agttataact aatgatgaaa caggaatgaa atacccttta 1860
acttcttatg aattgacccc aaacatggca ataatagata ctgaattaat gttaaatatg 1920
cctagaaaat taacagcagc aactggaata gatgcattag ttcatgctat agaagcatat 1980
gtttcggtta tggctacgga ttatactgat gaattagcct taagagcaat aaaaatgata 2040
tttaaatatt tgcctagagc ctataaaaat gggactaacg acattgaagc aagagaaaaa 2100
atggcacatg cctctaatat tgcggggatg gcatttgcaa atgctttctt aggtgtatgc 2160
cattcaatgg ctcataaact tggggcaatg catcacgttc cacatggaat tgcttgtgct 2220
gtattaatag aagaagttat taaatataac gctacagact gtccaacaaa gcaaacagca 2280
ttccctcaat ataaatctcc taatgctaag agaaaatatg ctgaaattgc agagtatttg 2340
aatttaaagg gtactagcga taccgaaaag gtaacagcct taatagaagc tatttcaaag 2400
ttaaagatag atttgagtat tccacaaaat ataagtgccg ctggaataaa taaaaaagat 2460

ttttataata cgctagataa aatgtcagag cttgcttttg atgaccaatg tacaacagct 2520

aatcctaggt atccacttat aagtgaactt aaggatatct atataaaatc attttaa 2577

<210> 52
<211> 858
<212> PRT
<213> Clostridium acetobutylicum

<400> 52

Met Lys Val Thr Asn Gln Lys Glu Leu Lys Gln Lys Leu Asn Glu Leu
1               5                   10                  15

Arg Glu Ala Gln Lys Lys Phe Ala Thr Tyr Thr Gln Glu Gln Val Asp
            20                  25                  30

Lys Ile Phe Lys Gln Cys Ala Ile Ala Ala Ala Lys Glu Arg Ile Asn
        35                  40                  45

Leu Ala Lys Leu Ala Val Glu Glu Thr Gly Ile Gly Leu Val Glu Asp
    50                  55                  60

Lys Ile Ile Lys Asn His Phe Ala Ala Glu Tyr Ile Tyr Asn Lys Tyr
65                  70                  75                  80

Lys Asn Glu Lys Thr Cys Gly Ile Ile Asp His Asp Asp Ser Leu Gly
                85                  90                  95

Ile Thr Lys Val Ala Glu Pro Ile Gly Ile Val Ala Ala Ile Val Pro
            100                 105                 110

Thr Thr Asn Pro Thr Ser Thr Ala Ile Phe Lys Ser Leu Ile Ser Leu
        115                 120                 125

Lys Thr Arg Asn Ala Ile Phe Phe Ser Pro His Pro Arg Ala Lys Lys
    130                 135                 140

Ser Thr Ile Ala Ala Ala Lys Leu Ile Leu Asp Ala Ala Val Lys Ala
145                 150                 155                 160

Gly Ala Pro Lys Asn Ile Ile Gly Trp Ile Asp Glu Pro Ser Ile Glu
                165                 170                 175

Leu Ser Gln Asp Leu Met Ser Glu Ala Asp Ile Ile Leu Ala Thr Gly
            180                 185                 190

Gly Pro Ser Met Val Lys Ala Ala Tyr Ser Ser Gly Lys Pro Ala Ile
        195                 200                 205

Gly Val Gly Ala Gly Asn Thr Pro Ala Ile Ile Asp Glu Ser Ala Asp
    210                 215                 220

Ile Asp Met Ala Val Ser Ser Ile Ile Leu Ser Lys Thr Tyr Asp Asn
225                 230                 235                 240

Gly Val Ile Cys Ala Ser Glu Gln Ser Ile Leu Val Met Asn Ser Ile
                245                 250                 255

Tyr Glu Lys Val Lys Glu Glu Phe Val Lys Arg Gly Ser Tyr Ile Leu
            260                 265                 270

Asn Gln Asn Glu Ile Ala Lys Ile Lys Glu Thr Met Phe Lys Asn Gly
        275                 280                 285

Ala Ile Asn Ala Asp Ile Val Gly Lys Ser Ala Tyr Ile Ile Ala Lys
    290                 295                 300

Met Ala Gly Ile Glu Val Pro Gln Thr Thr Lys Ile Leu Ile Gly Glu
305                 310                 315                 320

Val Gln Ser Val Glu Lys Ser Glu Leu Phe Ser His Glu Lys Leu Ser
                325                 330                 335

Pro Val Leu Ala Met Tyr Lys Val Lys Asp Phe Asp Glu Ala Leu Lys
            340                 345                 350

Lys Ala Gln Arg Leu Ile Glu Leu Gly Gly Ser Gly His Thr Ser Ser
        355                 360                 365

Leu Tyr Ile Asp Ser Gln Asn Asn Lys Asp Lys Val Lys Glu Phe Gly
    370                 375                 380

Leu Ala Met Lys Thr Ser Arg Thr Phe Ile Asn Met Pro Ser Ser Gln
385                 390                 395                 400

Gly Ala Ser Gly Asp Leu Tyr Asn Phe Ala Ile Ala Pro Ser Phe Thr
                405                 410                 415

Leu Gly Cys Gly Thr Trp Gly Gly Asn Ser Val Ser Gln Asn Val Glu
            420                 425                 430

Pro Lys His Leu Leu Asn Ile Lys Ser Val Ala Glu Arg Arg Glu Asn
        435                 440                 445

Met Leu Trp Phe Lys Val Pro Gln Lys Ile Tyr Phe Lys Tyr Gly Cys
    450                 455                 460

Leu Arg Phe Ala Leu Lys Glu Leu Lys Asp Met Asn Lys Lys Arg Ala
465                 470                 475                 480

Phe Ile Val Thr Asp Lys Asp Leu Phe Lys Leu Gly Tyr Val Asn Lys
                485                 490                 495

Ile Thr Lys Val Leu Asp Glu Ile Asp Ile Lys Tyr Ser Ile Phe Thr
            500                 505                 510

Asp Ile Lys Ser Asp Pro Thr Ile Asp Ser Val Lys Lys Gly Ala Lys
        515                 520                 525

Glu Met Leu Asn Phe Glu Pro Asp Thr Ile Ile Ser Ile Gly Gly Gly
    530                 535                 540

Ser Pro Met Asp Ala Ala Lys Val Met His Leu Leu Tyr Glu Tyr Pro
545                 550                 555                 560

Glu Ala Glu Ile Glu Asn Leu Ala Ile Asn Phe Met Asp Ile Arg Lys
                565                 570                 575

Arg Ile Cys Asn Phe Pro Lys Leu Gly Thr Lys Ala Ile Ser Val Ala
            580                 585                 590

Ile Pro Thr Thr Ala Gly Thr Gly Ser Glu Ala Thr Pro Phe Ala Val
        595                 600                 605

Ile Thr Asn Asp Glu Thr Gly Met Lys Tyr Pro Leu Thr Ser Tyr Glu
    610                 615                 620

Leu Thr Pro Asn Met Ala Ile Ile Asp Thr Glu Leu Met Leu Asn Met
625                 630                 635                 640

Pro Arg Lys Leu Thr Ala Ala Thr Gly Ile Asp Ala Leu Val His Ala
                645                 650                 655

Ile Glu Ala Tyr Val Ser Val Met Ala Thr Asp Tyr Thr Asp Glu Leu
            660                 665                 670

Ala Leu Arg Ala Ile Lys Met Ile Phe Lys Tyr Leu Pro Arg Ala Tyr
        675                 680                 685

Lys Asn Gly Thr Asn Asp Ile Glu Ala Arg Glu Lys Met Ala His Ala
    690                 695                 700

Ser Asn Ile Ala Gly Met Ala Phe Ala Asn Ala Phe Leu Gly Val Cys
705                 710                 715                 720

His Ser Met Ala His Lys Leu Gly Ala Met His His Val Pro His Gly
                725                 730                 735

Ile Ala Cys Ala Val Leu Ile Glu Glu Val Ile Lys Tyr Asn Ala Thr
            740                 745                 750

Asp Cys Pro Thr Lys Gln Thr Ala Phe Pro Gln Tyr Lys Ser Pro Asn
        755                 760                 765

Ala Lys Arg Lys Tyr Ala Glu Ile Ala Glu Tyr Leu Asn Leu Lys Gly
    770                 775                 780

Thr Ser Asp Thr Glu Lys Val Thr Ala Leu Ile Glu Ala Ile Ser Lys
785                 790                 795                 800

Leu Lys Ile Asp Leu Ser Ile Pro Gln Asn Ile Ser Ala Ala Gly Ile
                805                 810                 815

Asn Lys Lys Asp Phe Tyr Asn Thr Leu Asp Lys Met Ser Glu Leu Ala
            820                 825                 830

Phe Asp Asp Gln Cys Thr Thr Ala Asn Pro Arg Tyr Pro Leu Ile Ser
        835                 840                 845

Glu Leu Lys Asp Ile Tyr Ile Lys Ser Phe
    850                 855

<210>  53
<211>  1407
<212>  DNA
<213>  Clostridium saccharoperbutylacetonicum

<400>  53
atgattaaag acacgctagt ttctataaca aaagatttaa aattaaaaac aaatgttgaa      60

aatgccaatc taaagaacta caaggatgat tcttcatgtt tcggagtttt cgaaaatgtt     120

gaaaatgcta taagcaatgc cgtacacgca caaaagatat tatcccttca ttatacaaaa     180

gaacaaagag aaaaaatcat aactgagata agaaaggccg cattagaaaa taaagagatt     240

ctagctacaa tgattcttga agaaacacat atgggaagat atgaagataa aatattaaag     300

catgaattag tagctaaata cactcctggg acagaagatt taactactac tgcttggtca     360

ggagataacg ggcttacagt tgtagaaatg tctccatatg gcgttatagg tgcaataact     420

ccttctacga atccaactga aactgtaata tgtaatagta taggcatgat agctgctgga     480

aatactgtgg tatttaacgg acatccaggc gctaaaaaat gtgttgcttt tgctgtcgaa     540

atgataaata aagctattat ttcatgtggt ggtcctgaga atttagtaac aactataaaa     600

aatccaacta tggactctct agatgcaatt attaagcacc cttcaataaa actactttgc     660

ggaactggag ggccaggaat ggtaaaaacc ctcttaaatt ctggtaagaa agctataggt     720

gctggtgctg gaaatccacc agttattgta gatgatactg ctgatataga aaaggctggt     780

aagagtatca ttgaaggctg ttcttttgat aataatttac cttgtattgc agaaaaagaa     840

gtatttgttt ttgagaacgt tgcagatgat ttaatatcta acatgctaaa aaataatgct     900

gtaattataa atgaagatca agtatcaaag ttaatagatt tagtattaca aaaaaataat     960

gaaactcaag aatactctat aaataagaaa tgggtcggaa aagatgcaaa attattctta    1020

gatgaaatag atgttgagtc tccttcaagt gttaaatgca taatctgcga agtaagtgca    1080

aggcatccat ttgttatgac agaactcatg atgccaatat taccaattgt aagagttaaa    1140

gatatagatg aagctattga atatgcaaaa atagcagaac aaaatagaaa acatagtgcc     1200

tatatttatt caaaaaatat agacaaccta aataggtttg aaagagaaat cgatactact     1260

atctttgtaa agaatgctaa atcttttgcc ggtgttggtt atgaagcaga aggctttaca     1320

actttcacta ttgctggatc cactggtgaa ggaataactt ctgcaagaaa ttttacaaga     1380

caaagaagat gtgtactcgc cggttaa                                         1407

<210>  54
<211>  468
<212>  PRT
<213>  Clostridium saccharoperbutylacetonicum

<400>  54

Met Ile Lys Asp Thr Leu Val Ser Ile Thr Lys Asp Leu Lys Leu Lys
1               5                   10                  15

Thr Asn Val Glu Asn Ala Asn Leu Lys Asn Tyr Lys Asp Asp Ser Ser
            20                  25                  30

Cys Phe Gly Val Phe Glu Asn Val Glu Asn Ala Ile Ser Asn Ala Val
        35                  40                  45

His Ala Gln Lys Ile Leu Ser Leu His Tyr Thr Lys Glu Gln Arg Glu
    50                  55                  60

Lys Ile Ile Thr Glu Ile Arg Lys Ala Ala Leu Glu Asn Lys Glu Ile
65                  70                  75                  80

Leu Ala Thr Met Ile Leu Glu Glu Thr His Met Gly Arg Tyr Glu Asp
                85                  90                  95

Lys Ile Leu Lys His Glu Leu Val Ala Lys Tyr Thr Pro Gly Thr Glu
            100                 105                 110

Asp Leu Thr Thr Thr Ala Trp Ser Gly Asp Asn Gly Leu Thr Val Val
        115                 120                 125

Glu Met Ser Pro Tyr Gly Val Ile Gly Ala Ile Thr Pro Ser Thr Asn
    130                 135                 140

Pro Thr Glu Thr Val Ile Cys Asn Ser Ile Gly Met Ile Ala Ala Gly
145                 150                 155                 160

Asn Thr Val Val Phe Asn Gly His Pro Gly Ala Lys Lys Cys Val Ala
                165                 170                 175

Phe Ala Val Glu Met Ile Asn Lys Ala Ile Ile Ser Cys Gly Gly Pro
            180                 185                 190

Glu Asn Leu Val Thr Thr Ile Lys Asn Pro Thr Met Asp Ser Leu Asp

195                 200                 205

Ala Ile Ile Lys His Pro Ser Ile Lys Leu Leu Cys Gly Thr Gly Gly
    210                 215                 220

Pro Gly Met Val Lys Thr Leu Leu Asn Ser Gly Lys Lys Ala Ile Gly
225                 230                 235                 240

Ala Gly Ala Gly Asn Pro Pro Val Ile Val Asp Asp Thr Ala Asp Ile
                245                 250                 255

Glu Lys Ala Gly Lys Ser Ile Ile Glu Gly Cys Ser Phe Asp Asn Asn
            260                 265                 270

Leu Pro Cys Ile Ala Glu Lys Glu Val Phe Val Phe Glu Asn Val Ala
        275                 280                 285

Asp Asp Leu Ile Ser Asn Met Leu Lys Asn Asn Ala Val Ile Ile Asn
    290                 295                 300

Glu Asp Gln Val Ser Lys Leu Ile Asp Leu Val Leu Gln Lys Asn Asn
305                 310                 315                 320

Glu Thr Gln Glu Tyr Ser Ile Asn Lys Lys Trp Val Gly Lys Asp Ala
                325                 330                 335

Lys Leu Phe Leu Asp Glu Ile Asp Val Glu Ser Pro Ser Ser Val Lys
            340                 345                 350

Cys Ile Ile Cys Glu Val Ser Ala Arg His Pro Phe Val Met Thr Glu
        355                 360                 365

Leu Met Met Pro Ile Leu Pro Ile Val Arg Val Lys Asp Ile Asp Glu
    370                 375                 380

Ala Ile Glu Tyr Ala Lys Ile Ala Glu Gln Asn Arg Lys His Ser Ala
385                 390                 395                 400

Tyr Ile Tyr Ser Lys Asn Ile Asp Asn Leu Asn Arg Phe Glu Arg Glu
                405                 410                 415

Ile Asp Thr Thr Ile Phe Val Lys Asn Ala Lys Ser Phe Ala Gly Val
            420                 425                 430

Gly Tyr Glu Ala Glu Gly Phe Thr Thr Phe Thr Ile Ala Gly Ser Thr
        435                 440                 445

Gly Glu Gly Ile Thr Ser Ala Arg Asn Phe Thr Arg Gln Arg Arg Cys
    450                 455                 460

Val Leu Ala Gly

465

<210> 55
<211> 1170
<212> DNA
<213> Clostridium acetobutylicum

<400> 55
atgctaagtt ttgattattc aataccaact aaagtttttt ttggaaaagg aaaaatagac 60

gtaattggag aagaaattaa gaaatatggc tcaagagtgc ttatagttta tggcggagga 120

agtataaaaa ggaacggtat atatgataga gcaacagcta tattaaaaga aaacaatata 180

gctttctatg aactttcagg agtagagcca aatcctagga taacaacagt aaaaaaaggc 240

atagaaatat gtagagaaaa taatgtggat ttagtattag caataggggg aggaagtgca 300

atagactgtt ctaaggtaat tgcagctgga gtttattatg atggcgatac atgggacatg 360

gttaaagatc catctaaaat aactaaagtt cttccaattg caagtatact tactctttca 420

gcaacagggt ctgaaatgga tcaaattgca gtaatttcaa atatggagac taatgaaaag 480

cttggagtag gacatgatga tatgagacct aaattttcag tgttagatcc tacatatact 540

tttacagtac ctaaaaatca aacagcagcg ggaacagctg acattatgag tcacaccttt 600

gaatcttact ttagtggtgt tgaaggtgct tatgtgcagg acggtatagc agaagcaatc 660

ttaagaacat gtataaagta tggaaaaata gcaatggaga agactgatga ttacgaggct 720

agagctaatt tgatgtgggc ttcaagttta gctataaatg gtctattatc acttggtaag 780

gatagaaaat ggagttgtca tcctatggaa cacgagttaa gtgcatatta tgatataaca 840

catggtgtag gacttgcaat tttaacacct aattggatgg aatatattct aaatgacgat 900

acacttcata aatttgtttc ttatggaata aatgtttggg gaatagacaa gaacaaagat 960

aactatgaaa tagcacgaga ggctattaaa aatacgagag aatactttaa ttcattgggt 1020

attccttcaa agcttagaga agttggaata ggaaaagata aactagaact aatggcaaag 1080

caagctgtta gaaattctgg aggaacaata ggaagtttaa gaccaataaa tgcagaggat 1140

gttcttgaga tatttaaaaa atcttattaa 1170

<210> 56
<211> 389
<212> PRT
<213> Clostridium acetobutylicum

<400> 56

Met Leu Ser Phe Asp Tyr Ser Ile Pro Thr Lys Val Phe Phe Gly Lys
1               5                   10                  15

Gly Lys Ile Asp Val Ile Gly Glu Glu Ile Lys Lys Tyr Gly Ser Arg
            20                  25                  30

Val Leu Ile Val Tyr Gly Gly Gly Ser Ile Lys Arg Asn Gly Ile Tyr
        35                  40                  45

Asp Arg Ala Thr Ala Ile Leu Lys Glu Asn Asn Ile Ala Phe Tyr Glu
    50                  55                  60

Leu Ser Gly Val Glu Pro Asn Pro Arg Ile Thr Thr Val Lys Lys Gly
65                  70                  75                  80

Ile Glu Ile Cys Arg Glu Asn Asn Val Asp Leu Val Leu Ala Ile Gly
                85                  90                  95

Gly Gly Ser Ala Ile Asp Cys Ser Lys Val Ile Ala Ala Gly Val Tyr
            100                 105                 110

Tyr Asp Gly Asp Thr Trp Asp Met Val Lys Asp Pro Ser Lys Ile Thr
        115                 120                 125

Lys Val Leu Pro Ile Ala Ser Ile Leu Thr Leu Ser Ala Thr Gly Ser
    130                 135                 140

Glu Met Asp Gln Ile Ala Val Ile Ser Asn Met Glu Thr Asn Glu Lys
145                 150                 155                 160

Leu Gly Val Gly His Asp Asp Met Arg Pro Lys Phe Ser Val Leu Asp
                165                 170                 175

Pro Thr Tyr Thr Phe Thr Val Pro Lys Asn Gln Thr Ala Ala Gly Thr
            180                 185                 190

Ala Asp Ile Met Ser His Thr Phe Glu Ser Tyr Phe Ser Gly Val Glu
        195                 200                 205

Gly Ala Tyr Val Gln Asp Gly Ile Ala Glu Ala Ile Leu Arg Thr Cys
    210                 215                 220

Ile Lys Tyr Gly Lys Ile Ala Met Glu Lys Thr Asp Asp Tyr Glu Ala
225                 230                 235                 240

Arg Ala Asn Leu Met Trp Ala Ser Ser Leu Ala Ile Asn Gly Leu Leu
                245                 250                 255

Ser Leu Gly Lys Asp Arg Lys Trp Ser Cys His Pro Met Glu His Glu
            260                 265                 270

Leu Ser Ala Tyr Tyr Asp Ile Thr His Gly Val Gly Leu Ala Ile Leu
        275                 280                 285

Thr Pro Asn Trp Met Glu Tyr Ile Leu Asn Asp Asp Thr Leu His Lys
    290                 295                 300

Phe Val Ser Tyr Gly Ile Asn Val Trp Gly Ile Asp Lys Asn Lys Asp
305                 310                 315                 320

Asn Tyr Glu Ile Ala Arg Glu Ala Ile Lys Asn Thr Arg Glu Tyr Phe
                325                 330                 335

Asn Ser Leu Gly Ile Pro Ser Lys Leu Arg Glu Val Gly Ile Gly Lys
            340                 345                 350

Asp Lys Leu Glu Leu Met Ala Lys Gln Ala Val Arg Asn Ser Gly Gly
        355                 360                 365

Thr Ile Gly Ser Leu Arg Pro Ile Asn Ala Glu Asp Val Leu Glu Ile
    370                 375                 380

Phe Lys Lys Ser Tyr
385

<210>  57
<211>  1173
<212>  DNA
<213>  Clostridium acetobutylicum

<400>  57
gtggttgatt tcgaatattc aataccaact agaatttttt tcggtaaaga taagataaat      60

gtacttggaa gagagcttaa aaaatatggt tctaaagtgc ttatagttta tggtggagga     120

agtataaaga gaaatggaat atatgataaa gctgtaagta tacttgaaaa aaacagtatt     180

aaattttatg aacttgcagg agtagagcca aatccaagag taactacagt tgaaaaagga     240

gttaaaatat gtagagaaaa tggagttgaa gtagtactag ctataggtgg aggaagtgca     300

atagattgcg caaaggttat agcagcagca tgtgaatatg atggaaatcc atgggatatt     360

gtgttagatg gctcaaaaat aaaaagggtg cttcctatag ctagtatatt aaccattgct     420

gcaacaggat cagaaatgga tacgtgggca gtaataaata atatggatac aaacgaaaaa     480

ctaattgcgg cacatccaga tatggctcct aagttttcta tattagatcc aacgtatacg     540

tataccgtac ctaccaatca aacagcagca ggaacagctg atattatgag tcatatattt     600

gaggtgtatt ttagtaatac aaaaacagca tatttgcagg atagaatggc agaagcgtta     660

ttaagaactt gtattaaata tggaggaata gctcttgaga agccggatga ttatgaggca     720

agagccaatc taatgtgggc ttcaagtctt gcgataaatg gacttttaac atatggtaaa     780

gacactaatt ggagtgtaca cttaatggaa catgaattaa gtgcttatta cgacataaca     840

cacggcgtag ggcttgcaat tttaacacct aattggatgg agtatatttt aaataatgat     900

acagtgtaca agtttgttga atatggtgta aatgtttggg gaatagacaa agaaaaaaat     960

cactatgaca tagcacatca agcaatacaa aaaacaagag attactttgt aaatgtacta    1020

ggtttaccat ctagactgag agatgttgga attgaagaag aaaaattgga cataatggca    1080

aaggaatcag taaagcttac aggaggaacc ataggaaacc taagaccagt aaacgcctcc    1140

gaagtcctac aaatattcaa aaaatctgtg taa                                 1173

<210>  58
<211>  390
<212>  PRT
<213>  Clostridium acetobutylicum

<400>  58

Met Val Asp Phe Glu Tyr Ser Ile Pro Thr Arg Ile Phe Phe Gly Lys
1               5                   10                  15

Asp Lys Ile Asn Val Leu Gly Arg Glu Leu Lys Lys Tyr Gly Ser Lys
            20                  25                  30

Val Leu Ile Val Tyr Gly Gly Gly Ser Ile Lys Arg Asn Gly Ile Tyr
        35                  40                  45

Asp Lys Ala Val Ser Ile Leu Glu Lys Asn Ser Ile Lys Phe Tyr Glu
    50                  55                  60

Leu Ala Gly Val Glu Pro Asn Pro Arg Val Thr Thr Val Glu Lys Gly
65                  70                  75                  80

Val Lys Ile Cys Arg Glu Asn Gly Val Glu Val Val Leu Ala Ile Gly
                85                  90                  95

Gly Gly Ser Ala Ile Asp Cys Ala Lys Val Ile Ala Ala Ala Cys Glu
            100                 105                 110

Tyr Asp Gly Asn Pro Trp Asp Ile Val Leu Asp Gly Ser Lys Ile Lys
        115                 120                 125

Arg Val Leu Pro Ile Ala Ser Ile Leu Thr Ile Ala Ala Thr Gly Ser
    130                 135                 140

Glu Met Asp Thr Trp Ala Val Ile Asn Asn Met Asp Thr Asn Glu Lys
145                 150                 155                 160

Leu Ile Ala Ala His Pro Asp Met Ala Pro Lys Phe Ser Ile Leu Asp
                165                 170                 175

Pro Thr Tyr Thr Tyr Thr Val Pro Thr Asn Gln Thr Ala Ala Gly Thr
            180                 185                 190

Ala Asp Ile Met Ser His Ile Phe Glu Val Tyr Phe Ser Asn Thr Lys
        195                 200                 205

Thr Ala Tyr Leu Gln Asp Arg Met Ala Glu Ala Leu Leu Arg Thr Cys
    210                 215                 220

Ile Lys Tyr Gly Gly Ile Ala Leu Glu Lys Pro Asp Asp Tyr Glu Ala
225                 230                 235                 240

Arg Ala Asn Leu Met Trp Ala Ser Ser Leu Ala Ile Asn Gly Leu Leu

245                 250                 255

Thr Tyr Gly Lys Asp Thr Asn Trp Ser Val His Leu Met Glu His Glu
            260                 265                 270

Leu Ser Ala Tyr Tyr Asp Ile Thr His Gly Val Gly Leu Ala Ile Leu
        275                 280                 285

Thr Pro Asn Trp Met Glu Tyr Ile Leu Asn Asn Asp Thr Val Tyr Lys
    290                 295                 300

Phe Val Glu Tyr Gly Val Asn Val Trp Gly Ile Asp Lys Glu Lys Asn
305                 310                 315                 320

His Tyr Asp Ile Ala His Gln Ala Ile Gln Lys Thr Arg Asp Tyr Phe
                325                 330                 335

Val Asn Val Leu Gly Leu Pro Ser Arg Leu Arg Asp Val Gly Ile Glu
            340                 345                 350

Glu Glu Lys Leu Asp Ile Met Ala Lys Glu Ser Val Lys Leu Thr Gly
        355                 360                 365

Gly Thr Ile Gly Asn Leu Arg Pro Val Asn Ala Ser Glu Val Leu Gln
    370                 375                 380

Ile Phe Lys Lys Ser Val
385                 390

## Description

This invention relates to a recombinant host cell with elevated expression or activity of the following enzymes as compared to the parent host cell it is derived from: (a) 4-hydroxybutyryl-CoA dehydratase; and (b) (ba) crotonate CoA-transferase; and/or (bb) at least one, preferably all of the enzymes of at least one of the following groups (i) to (iv): (i) 2-ketoacid decarboxylase; (ii) 2-ketoglutarate dehydrogenase, succinyl-CoA synthetase and succinic semialdehyde dehydrogenase; (iii) 2-ketoglutarate dehydrogenase and succinyl-CoA reductase; and (iv) glutamate dehydrogenase, glutamate decarboxylase and γ-amino-N-butyrate transaminase.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Generation of bulk chemicals, such as crotonic acid, from renewable resources such as sugars instead of fossil resources, is of increasing economic and environmental importance due to the environmental burden associated with the use of fossil carbon resources, as well as increasing costs due to advancing depletion of known fossil fuels.

The bulk chemical crotonic acid can be used as building block for polymers such as polycrotonate. The precursor crotonyl-CoA is a key intermediate in several microbial pathways such as glutamate fermentation, or butanol production in the acetone-butanol-ethanol (ABE)-fermentation pathway of *Clostridium acetobutylicum.*

In the ABE-fermentation pathway of *Clostridium acetobutylicum,* a carbon source like glucose is degraded into two acetyl-CoA molecules, which are then reacted to form acetoacetyl-CoA and reduced in several steps via crotonyl-CoA to butanol. As described in WO2007/041269A2, this pathway may be utilized in a recombinant microorganism to produce butanol. However, a serious bottleneck of the pathway described in this document is the formation of acetoacetyl-CoA with a strong dependence on high acetyl-CoA concentrations for efficient enzymatic activity. Acetyl-CoA is a key intermediate for many pathways in the cell. Therefore, achieving and maintaining a high acetyl-CoA concentration is generally difficult.

In W02009/046828A1, crotonic acid production is achieved through a pathway known as the 2-hydroxyglutarate pathway. A weakness of the 2-hydroxyglutarate pathway is the decarboxylation step catalyzed by glutaconyl-CoA decarboxylase. Difficulties in the biotechnological application arise from the fact that this particular enzyme is a membrane bound hetero-tetramer which also acts as sodium ion pump, characteristics which hinder efficient heterologous activity.

WO2008/115840 describes compositions and methods for the synthesis of 1,4-butanediol. This document is silent about the metabolite crotonyl-CoA which, as further detailed below, is a key intermediate metabolite according to the present invention.

DE10 2007 059 248 describes a cyclic metabolic pathway which provides for the production of acetoacetyl-CoA from acetyl-CoA and two equivalents hydrogen carbonate. This document is silent about starting materlials or metabolites comprising five carbon atoms, let alone their use in the production of crotonyl-CoA or crotonic acid.

It is therefore apparent that there is an unmet need for means and methods of producing crotonyl-CoA and downstream products thereof such as crotonic acid, wherein said production utilizes 2-ketoacids or glutamate as a starting material.

Accordingly, in a first aspect this invention relates to a recombinant host cell with elevated expression or activity of the following enzymes as compared to the parent host cell it is derived from: (a) 4-hydroxybutyryl-CoA dehydratase; and (b) (ba) crotonate CoA-transferase; and/or (bb) at least one, preferably all of the enzymes of at least one of the following groups (i) to (iv): (i) 2-ketoacid decarboxylase; (ii) 2-ketoglutarate dehydrogenase, succinyl-CoA synthetase and succinic semialdehyde dehydrogenase; (iii) 2-ketoglutarate dehydrogenase and succinyl-CoA reductase; and (iv) glutamate dehydrogenase, glutamate decarboxylase and γ-amino-N-butyrate transaminase.

The host cell may be any cell. Preferred host cells are further detailed below. It is understood that the host cell according to the invention is not within the context of an animal or human. It is preferred that the host cell is in vitro, in culture, and/or isolated. Also, the host cell may be a unicellular microorganism. The host cell is derived from a parent host cell. Preferred parent host cells are described further below. The host cell differs from the parent host cell at least in that, and preferably only in that, it has elevated expression or activity of the enzymes as required or recited in the main embodiment or in further embodiments detailed below. Said elevated expression or activity is achieved by, for example, engineering the parent host cell with nucleic acids encoding the recited enzymes, which engineering with nucleic acids is the subject of a further aspect of the present invention as described below. As an alternative to being engineered with nucleic acids encoding the recited enzymes, the host cell may be recombinant in other respects, for example, by being engineered with nucleic acids which enhance or encode polypeptides enhancing the expression or activity of the recited enzymes.

The term "recombinant" has its meaning as established in the art. It refers to the presence of genetic material in the host cell which is not present in the parent host cell. The genetic material absent from the parent host cell may be a nucleic acid encoding an enzyme according to the invention. Alternatively or in addition, it may be the sequence of the vector which renders the host cell different from the parent host cell. More specifically, a nucleic acid encoding an enzyme, the nucleic acid as such being present in the parent host cell, may be placed in a different context within a vector, the presence of such a vector rendering the host cell different from the parent host cell. Vectors according to the invention are discussed in more detail below.

It is understood that the term "elevated" refers to a statistically significant difference in expression or activity. In preferred embodiments, expression and/or activity are elevated at least 1.5-fold, 2-fold, 3-fold, 4-fold, 5-fold, 10-fold, 20-fold, 50-fold, 100-fold, 1000-fold higher or above as compared to the expression and/or activity of the enzyme under consideration in the parent host cell. Also the case where the parent host cell has no expression of one or more of the recited enzymes is embraced. In the latter case, it is preferred that the host cell has an expression or activity of the recited enzymes which provides for a production of crotonyl-CoA or crotonic acid, when provided with (a) carbon source(s) as further detailed below, of at least 100 mg/L culture.

The term "expression" is understood to refer to the expression level of the mRNA encoding the respective enzyme on the one hand and protein expression level of the enzyme on the other hand. Means and methods for determining the mRNA expression levels are well-known in the art as are means and methods for determining protein expression levels. Such means include Northern-blotting, quantitative PCR, hybridization to microarrays or DNA chips on the one hand and Western-blotting, immunoassays and protein chips on the other hand.

The enzymes according to parts (a) and (b) of the main embodiment are responsible for the last step directly leading to the generation of the key intermediate crotonyl-CoA (part (a)) and for the initial step of converting a ketoacid, glutamate, succinate or 4-amino butyrate into succinic semialdehyde (part (b)), respectively.

The main embodiment provides for a plurality of enzyme combinations. Each of these combinations, regardless of whether it is expressly mentioned herein or not, is readily conceivable for the skilled person in view of the teaching of this application and is deliberately envisaged as an embodiment of the invention. Such combinations of enzymes include the following: (1) 4-hydroxybutyryl-CoA dehydratase and crotonate CoA transferase (2) 4-hydroxybutyryl-CoA dehydratase and 2-ketoacid decarboxylase, (3) 4-hydroxybutyryl-CoA dehydratase and one, two or three enzymes selected from 2-ketoglutarate dehydrogenase, succinyl-CoA synthetase and succinic semialdehyde dehydrogenase, (4) 4-hydroxybutyryl-CoA dehydratase and one or both of 2-ketoglutarate dehydrogenase and succinyl-CoA reductase, and (5) 4-hydroxybutyryl-CoA dehydratase and one, two or three enzymes selected from glutamate dehydrogenase, glutamate decarboxylase and γ-amino-N-butyrate transaminase.

Furthermore, it is preferred that features (a), (ba) and (bb) are present. An example of this particular preferred embodiment is a recombinant host cell with elevated expression or activity of 4-hydroxybutyryl-CoA dehydratase, crotonate CoA transferase and 2-ketoacid decarboxylase. To explain further, any of the preferred embodiments (1) to (5) described in the preceding paragraph may be modified by furthermore requiring elevated expression or activity of crotonate CoA transferase, thereby obtaining further examples of said embodiment requiring features (a), (ba) and (bb).

An enzyme with 4-hydroxybutyryl-CoA dehydratase activity, like the 4-hydroxybutyryl-CoA dehydratase also described as 4-hydroxybutanoyl-CoA dehydratase (EC 4.2.1.120), catalyzes the dehydration of the activated carboxylic acid 4-hydroxybutyryl-CoA to crotonyl-CoA. 4-hydroxybutyryl-CoA dehydratase can also act on a further substrate which is vinylacetyl-CoA. In that case, 4-hydroxybutyryl-CoA dehydratase acts as an isomerase and converts vinylacetyl-CoA into crotonyl-CoA (EC 5.3.3.3).

An enzyme with crotonate CoA-transferase or crotonyl CoA: acetate CoA-transferase activity, like the acetate CoA transferase (EC 2.8.3.8), catalyzes the conversion of crotonyl-CoA to crotonic acid.

An enzyme with 2-ketoglutarate dehydrogenase activity, like the 2-ketoglutarate dehydrogenase complex (EC 1.2.4.2 + EC 2.3.1.61 + EC 1.8.1.4), catalyzes the oxidative decarboxylation of 2-ketoglutarate to succinyl-CoA.

An enzyme with succinyl-CoA synthetase activity, like the ADP forming succinyl-CoA synthetase (EC 6.2.1.5), catalyzes the conversion of succinyl-CoA to succinate.

An enzyme with succinic semialdehyde dehydrogenase activity, like the NAD(P)⁺ dependent succinic semialdehyde dehydrogenase (EC 1.2.1.16), catalyzes the conversion of succinate to succinic semialdehyde.

An enzyme with succinyl-CoA reductase activity, like Metallosphaera sedula succinyl-CoA reductase (EC 1.2.1.76), catalyzes the NAD(P)H dependent conversion of succinyl-CoA to succinic semialdehyde. An example is the enzyme encoded by the gene with the gene symbol Msed_0709 of Metallosphaera sedula DSM5348. The corresponding Gene ID as of April 14, 2011 is 5103747.

An enzyme with 2-ketoglutarate decarboxylase activity, like the 2-ketoglutarate decarboxylase (EC 4.1.1.71), catalyzes the decarboxylation of 2-ketoglutarate.

An enzyme with glutamate dehydrogenase activity, like the glutamate dehydrogenase (EC 1.4.1.4), catalyzes the NAD(P)H and NH₃ dependent conversion of 2-ketoglutarate to glutamate.

An enzyme with glutamate decarboxylase activity, like glutamate decarboxylase (EC 4.1.1.15), catalyzes the decarboxylation of glutamate to 4-aminobutyrate.

An enzyme with 4-amino-N-butyrate transaminase activity, like 4-amino-N-butyrate transaminase (EC 2.6.1.19), catalyzes the conversion of 4-aminobutyrate to succinic semialdehyde and a co-substrate like 2-ketoglutarate to glutamate.

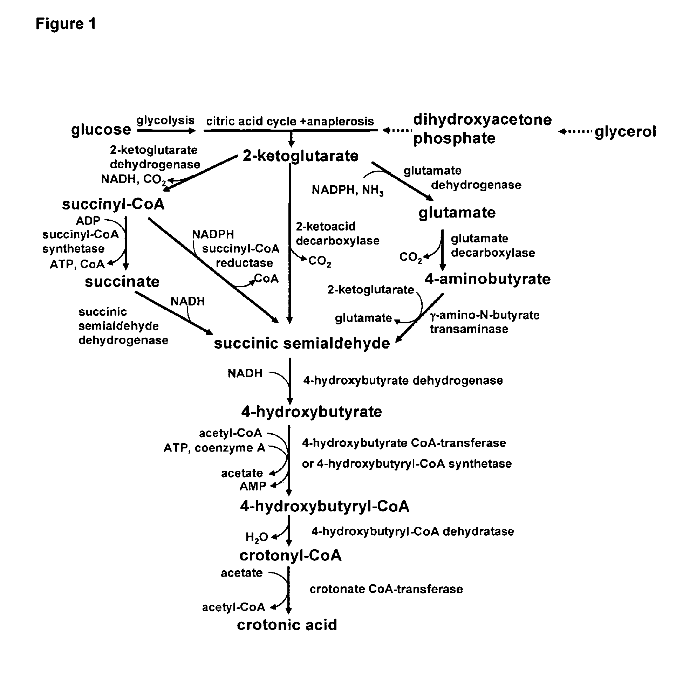

The attached Figure 1 provides a schematic overview of the metabolic pathway according to the invention which leads to crotonic acid.

A further key step of the pathway according to the present invention is the decarboxylation step. As discussed herein above, the prior art, for example, WO2009/046828 uses glutaconyl-CoA decarboxylase. The deficiencies of this approach are outlined herein above. In contrast, the present invention, for the purpose of decarboxylation, relies on different enzymatic activities, wherein the envisaged options are defined in part (b) of the main embodiment and depicted in the upper part of Figure 1. 2-ketoacid decarboxylases are a generally unproblematic class of enzymes which are highly efficient in biotechnological applications. Accordingly, option (i) of part (b), directed to a 2-ketoacid decarboxylase is preferred. Alternative or additional routes for the conversion of 2-ketoglutarate into succinic semialdehyde are the subject of options (ii), (iii) and (iv) of part (b). Moreover, options (ii), (iii) and (iv) of part (b) provide for other entry points such as glutamate, succinate and 4-amino butyrate, see Figure 1. It is preferred, though, that the host cell according to the invention comprises at least one enzyme with decarboxylating activity, said enzyme being selected from options (i) to (iv) of part (b).

As is well-known in the art, 2-ketoglutarate is a metabolite occurring in virtually all cells. It is available, for example, from glucose via glycolysis and parts of the citric acid cycle. Glycolysis and citric acid cycle are central elements of the ubiquitous carbohydrate catabolic pathway.

The present invention advantageously uses the ubiquitous intermediate metabolite ketoglutarate in order to produce the compound crotonyl-CoA. Most conceivable carbon sources, such as sugars, alcohols, lipids, waxes, alkanes or proteins, can be utilized as feed stock to allow a strong carbon flux to the intermediate 2-ketoglutarate, in contrast to DE10 2007 059 248, which relies on hydrogen carbonate. Furthermore, the carbon source glycerol can be used.

Option (ii), in addition to being energetically favourable through providing a GTP or ATP compared to the other presented options, utilizes enzymatic activities of the highly efficient citric acid cycle up to succinate.

In a second aspect of the invention, the present invention relates to a host cell engineered with (a) nucleic acid(s) encoding the following enzymes: (a) 4-hydroxybutyryl-CoA dehydratase; and (b) (ba) crotonate CoA-transferase; and/or (bb) at least one, preferably all of the enzymes of at least one of the following groups (i) to (iv): (i) 2-ketoacid decarboxylase; (ii) 2-ketoglutarate dehydrogenase, succinyl-CoA synthetase and succinic semialdehyde dehydrogenase; (iii) 2-ketoglutarate dehydrogenase and succinyl-CoA reductase; and (iv) glutamate dehydrogenase, glutamate decarboxylase and γ-amino-N-butyrate transaminase.

This embodiment stipulates specific means which render the host cell according to the invention an engineered or recombinant host cell. In particular, this embodiment requires that (a) nucleic acid(s) encoding the specific recited enzymes has/have been introduced into the host cell. In other words, the host cell is, for example, transformed, transduced or transfected with such nucleic acid(s).

The enzymes required for engineering the pathway according to the present invention may be provided by nucleic acid molecules initially inserted in a vector which can e.g. be synthesized by standard methods, or isolated from natural sources. Accordingly, provided are also one or more vectors, said vector(s) comprising nucleic acid(s) encoding the enzymes required by or recited in the main embodiment or embodiments described below.

Ligation of the coding sequences to transcriptional regulatory elements and/or to other amino acid encoding sequences comprised in the vector can be carried out using established methods. Transcriptional regulatory elements (parts of an expression cassette) ensuring expression in prokaryotes or eukaryotic cells are well known to those skilled in the art. These elements comprise regulatory sequences ensuring the initiation of transcription (e. g., translation initiation codon, promoters, such as naturally-associated or heterologous promoters and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers. Preferably, the polynucleotide(s) encoding the enzyme(s) of the invention is/are operatively linked to such expression control sequences allowing expression in prokaryotes or eukaryotic cells. The vector may further comprise nucleic acid sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

An expression cassette can be generated by fusing a suitable promoter with a suitable nucleotide sequence and a terminator signal or polyadenylation signal. The coding sequences can e.g. be synthesized by standard methods, or isolated from natural sources. For generating the expression cassette, customary recombination and cloning techniques are used as described, for example, by T. Maniatis, E. F. Fritsch and J. Sambrook, Molecular Cloning: A Laboratory Manual, Cold Spring Harbor Laboratory, Cold Spring Harbor, N.Y. (1989); by T. J. Silhavy, M. L. Berman and L. W. Enquist, Experiments with Gene Fusions, Cold Spring Harbor Laboratory, Cold Spring Harbor, N.Y. (1984) and by Ausubel, F. M. et al., Current Protocols in Molecular Biology, Greene Publishing Assoc.and Wiley Interscience (1987).

For expression in a suitable host organism, the recombinant nucleic acid construct or gene construct is advantageously inserted into a host-specific vector which allows optimal gene expression in said host. Vectors are well known to the skilled worker and can be found, for example, in "Cloning Vectors" (Pouwels P. H. et al., Ed., Elsevier, Amsterdam-N.Y.-Oxford, 1985). Vectors are to be understood as meaning not only plasmids, but all other vectors known to the skilled worker such as, for example, phages, viruses such as SV40, CMV, baculovirus and adenovirus, transposons, IS elements, phasmids, cosmids, and linear or circular DNA. These vectors can be replicated autonomously in the host organism or chromosomally.

Non-limiting examples of vectors include prokaryotic plasmid vectors, such as the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for *Pichia pastoris* comprise e.g. the plasmids pAO815, pPIC9K and pPIC3.5K (all Invitrogen). Examples of plasmid vectors suitable for C. *glutamicum* comprise e.g. the C. *glutamicum*/ *E. coli* shuttle vectors pEK-Ex1, pEC-XK99E, or pBL1 derivatives (pEK0).

Furthermore, it is preferred that the vector of the invention comprises a selectable marker. Examples of selectable markers include neomycin, gentamycin, ampicillin, zeocin, and hygromycin resistance and the like. Specifically-designed vectors allow the shuttling of DNA between different hosts, such as between bacteria and fungal cells or between bacteria and animal cells.

The vectors according to the invention allow the generation of recombinant host cells which can be transformed, for example, with at least one vector comprising sequences encoding the enzymes required for the pathway according to the invention. It is preferred to use cloning and transfection methods known to the skilled worker, for example co-precipitation, protoplast fusion, electroporation, retroviral transfection and the like, in order to achieve expression of the abovementioned nucleic acid(s) in the expression system in question. Suitable systems are described, for example, in Current Protocols in Molecular Biology, F. Ausubel et al., Ed., Wiley Interscience, New York 1997.

Alternatively, the nucleic acid(s) according to the above described second aspect is/are inserted into the suitable host cell's chromosome. Methods of stable chromosomal integration suitable for the respective host cell are known to persons skilled in the art. For example, genes can be integrated into the chromosome of the host cell in a targeted fashion by using a plasmid that does not replicate in the host cell. For instance, the vector pK18mobsacB, which replicates in E. *coli* but not in C. *glutamicum,* can be used to insert the , nucleic acid construct of choice into the chromosome of C. *glutamicum,* allowing for a stable chromosomal expression.

In a preferred embodiment, the invention provides the recombinant host cell according to the main embodiment, furthermore having an elevated expression or activity compared to the parent host cell it is derived from of 4-hydroxybutyrate dehydrogenase, 4-hydroxybutyrate CoA-transferase and/or 4-hydroxybutyryl-CoA synthetase or the engineered host cell according to a further aspect of the invention, further being transformed with (a) nucleic acid(s) encoding 4-hydroxybutyrate dehydrogenase, 4-hydroxybutyrate CoA-transferase and/or 4-hydroxybutyryl-CoA synthetase. Particularly preferred embodiments (1) to (3) relate to elevated expression or activity of or the host cell being engineered with (a) nucleic acid(s) encoding (1) 4-hydroxybutyryl-CoA dehydratase, 2-ketoacid decarboxylase and 4-hydroxybutyrate dehydrogenase, (2) 4-hydroxybutyryl-CoA dehydratase, 2-ketoacid decarboxylase and 4-hydroxybutyrate CoA-transferase, and (3) 4-hydroxybutyryl-CoA dehydratase, 2-ketoacid decarboxylase and 4-hydroxybutyryl-CoA synthetase.

As can be seen in Figure 1, the enzymes additionally recited in this preferred embodiment provide for (i) the conversion of succinic semialdehyde to 4-hydroxybutarate, and (ii) the conversion of 4-hydroxybutarate into 4-hydroxybutyryl-CoA. 4-hydroxybutyrate CoA-transferase and 4-hydroxybutarate synthetase are two alternative enzymes capable of performing the latter of the two steps. Accordingly, what is provided are host cells over-expressing or being engineered with (a) nucleic acid(s) encoding enzymes catalyzing three, four or all five steps of the pathway depicted in Figure 1. A particularly preferred recombinant or engineered host cell is a host cell which is characterized by elevated expression and/or activity of or being engineered with (a) nucleic acid(s) encoding, respectively, the following enzymes: 2-ketoacid decarboxylase, 4-hydroxybutyrate dehydrogenase, 4-hydroxybutyrate CoA-transferase, 4-hydroxybutyryl-CoA dehydratase, and crotonate CoA-transferase. In such preferred host cell, 4-hydroxybutyrate CoA-transferase may also be replaced with 4-hydroxybutarate-CoA synthetase. The latter particularly preferred embodiment is exemplified in Example 1 as enclosed herewith.

The crotonate CoA-transferase reaction can be replaced by a crotonyl-CoA synthetase reaction, converting crotonyl-CoA to crotonate under ATP formation, or a combined phosphotransacetylase and crotonate kinase reaction, converting crotonyl-CoA to crotonyl phosphate and then to crotonate.

A further preferred recombinant or engineered host cell is a host cell which is characterized by elevated expression and/or activity of or being engineered with (a) nucleic acid(s) encoding, respectively, the following enzymes: 2-ketoglutarate dehydrogenase, succinyl-CoA synthetase, succinic semialdehyde dehydrogenase, 4-hydroxybutyrate dehydrogenase, 4-hydroxybutyrate CoA-transferase and 4-hydroxybutyryl-CoA dehydratase.

By requiring the presence of three or more enzymes at elevated levels, a particular efficient conversion of 2-ketoglutarate into crotonyl-CoA is ensured. Furthermore, the chance of side product formation and accumulation of potentially toxic intermediates, such as aldehydes, is reduced. When referring to first step in this regard, it is understood that the first step may actually be a composite step as highlighted in the upper part of Figure 1 and furthermore evident from options (ii), (iii) and (iv) of part (b) of the main embodiment.

In a further preferred embodiment, said 2-ketoacid decarboxylase is 2-ketoglutarate decarboxylase.

In a further preferred embodiment, the present invention provides a recombinant host cell according to the invention, further having an elevated expression or activity as compared to the parent host cell it is derived from of at least one of the enzymes selected from the following group, or an engineered host cell according to the invention, further being transformed with (a) nucleic acid(s) encoding at least one enzyme of the following group, said group consisting of enzymes with 4-hydroxybut-2-enoate ammonia lyase and 4-hydroxybut-2-enoate reductase activity.

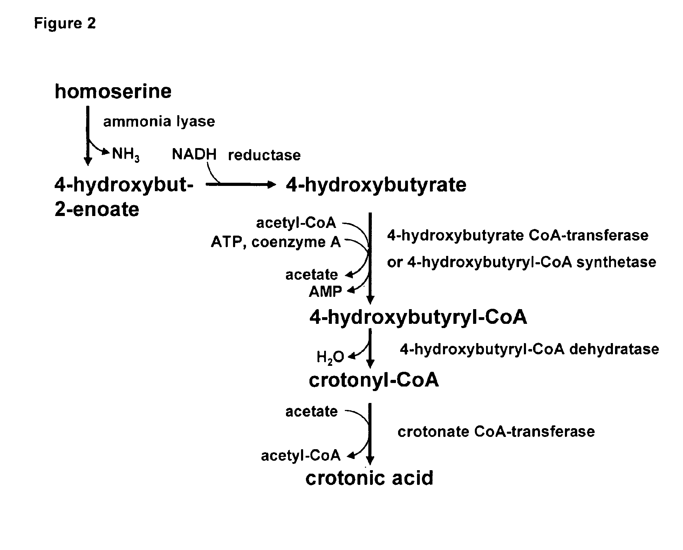

As can be seen from Figure 2, this embodiment provides for the utilization of an additional pathway leading to 4-hydroxybutyrate, 4-hydroxybutyrate in turn being an intermediate of the central pathway according to the invention as depicted in Figure 1.

In a further preferred embodiment, the present invention relates to a recombinant host cell according to any of the above described embodiments, further having an elevated expression or activity compared to the parent host cell it is derived from of at least one of the enzymes selected from the following group, or to an engineered host cell according to any one of the above described embodiments, further being transformed with (a) nucleic acid(s) encoding at least one enzyme of the following group, said group consisting of butyryl-CoA dehydrogenase, butyraldehyde dehydrogenase, butanol dehydrogenase, butyrate CoA-transferase, crotonate CoA-transferase, crotonyl-CoA synthetase and butyryl-CoA synthetase.

The butyrate CoA-transferase reaction can be replaced by a butyryl-CoA synthetase reaction, converting butyryl-CoA to butyrate under ATP formation, or a combined phosphotransacetylase and butyrate kinase reaction, converting butyrl-CoA to butyryl phosphate and then to butyrate.

Related thereto, provided is a recombinant or engineered host cell according to the invention, wherein in said recombinant host cell the expression or activity of at least one, preferably all of the enzymes of at least one of the following groups (a) and (b): (a) butyryl-CoA dehydrogenase, butyraldehyde dehydrogenase, and butanol dehydrogenase; and (b) butyryl-CoA dehydrogenase and butyrate CoA-transferase is elevated, as well as an engineered host cell which is transformed with (a) nucleic acid(s) encoding at least one, preferably all of the enzymes of at least one of said groups.

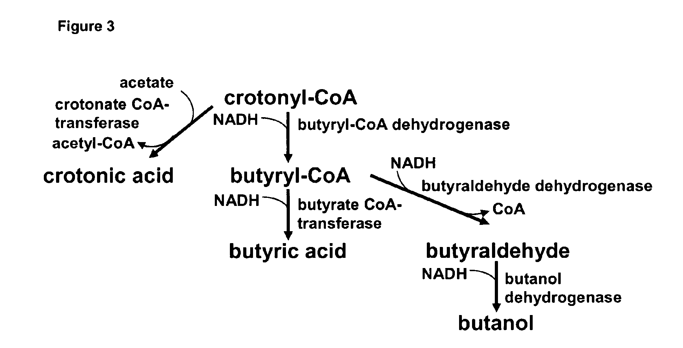

These embodiments relate to the further options for the downstream processing of crotonyl-CoA, namely to give rise to products such as butyric acid, butyraldehyde and butanol. A corresponding pathway scheme is comprised in Figure 3.

In a further preferred embodiment of the recombinant host cell according to the invention, said enzyme(s) is/are expressed from (a) nucleic acid(s) introduced into said host cell. This embodiment effectively provides for a limitation of the recombinant host cell according to the invention to a host cell which is engineered with nucleic acids encoding the recited enzymes.

In a further preferred embodiment, the above recited nucleic acid(s) comprise(s) at least one nucleic acid selected from SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, 55, and 57 or at least one nucleic acid encoding an enzyme with the same activity and having at least 40% sequence identity to the amino acid sequence of SEQ ID NOs: 2, 4, 6, 8, 10, 12, 14, 16, 18, 20, 22, 24, 26, 28, 30, 32, 34, 36, 40, 42, 44, 46, 48, 50, 52, 54, 56, and 58.

Preferably, said sequence identity is, independently for each sequence, at least 50%, at least 60%, at least 70%, at least 80%, at least 90%, at least 91%, at least 92%, at least 93%, at least 94%, at least 95%, at least 96%, at least 97%, at least 98%, or at least 99%.

It is understood that the recited nucleic acids, to the extent they exhibit less than 100% sequence identity to the specific SEQ ID NOs., exhibit the enzymatic activity of a protein encoded by the respective SEQ ID NO. Presence and absence of the respective enzymatic activity can be assayed by the skilled person using tests known in the art without further ado.

In a further preferred embodiment, the host cell is selected from a microorganism, a plant cell and an animal cell, preferably from the kingdom of bacteria, archea, yeast and fungi, more preferably from the genus of *Clostridium* and *Escherichia, Pseudomonas, Bacillus, Saccharomyces, Pichia, Clostridium, Corynebacterium* and *Aspergillus,* yet more preferably *E. coli.*

It is understood that the term "host cell" refers to the parent host cell, the recombinant host cell and the engineered host cell according to the invention. If, for example, an E. *coli* cell is used as a parent host cell, it follows that also the recombinant or engineered host cell is considered as an E. *coli* cell. To the extent human host cells are envisaged, it is preferred that such human host cell is not obtained from a human embryo, in particular not via methods entailing destruction of a human embryo. On the other hand, human embryonic stem cell lines are at the skilled person's disposal. Accordingly, the present invention may be worked with human embryonic stem cells without any need to use or destroy human embryos.

In a further preferred embodiment, said host cell is a microorganism which is strictly or facultatively anaerob. To explain further, 4-hydroxybutyryl-CoA dehydratase is an enzyme which is typically oxygen sensitive. Typically, 4-hydroxybutyryl-CoA dehydratases use a radical mechanism. In the presence of oxygen, the radical form of the enzyme might become inactivated to a certain degree, in particular in case no protective means are used.

On the other hand it is understood that also an aerob microorganism may be used. In such a case, an oxygen insensitive enzyme variant with 4-hydroxybutyryl-CoA dehydratase activity may be used. Such an enzyme can be obtained through adaptation of a 4-hydroxybutyryl-CoA dehydratase to oxygen, or through mutation or change of an oxygen insensitive enzyme, which enzyme initially exhibits a different enzymatic activity, to 4-hydroxybutyryl-CoA dehydratase activity. For instance, an enzyme utilizing cobalamine (vitamin B₁₂) as co-factor for the radical mechanism is an oxygen tolerant dehydratase which may serve as starting point for such a mutational approach to the design of an oxygen insensitive 4-hydroxybutyryl-CoA dehydratase. Oxygen tolerant glycerol dehydratases utilizing the co-factor vitamin B₁₂ as oxygen stable radical donor are known, such as encoded by dhaCBE in *Citrobacter freundii* (Seyfried et al., 1996, J Bacteriol 178: 5793-5796).

In a further preferred embodiment, one or more competing enzymes or pathways are down regulated or knocked out. A competing enzyme or pathway is an enzyme or pathway causing depletion of any of the starting materials or metabolites of the pathway of the present invention. An example of a competing pathway is any pathway utilizing glutamate in a manner which is different from the utilization of glutamate as depicted in Figure 1.

In a further preferred embodiment, at least one enzyme capable of degrading a precursor or derivative of the crotonyl-CoA biosynthetic pathway is down-regulated or knocked out, said enzyme preferably being selected from crotonase, crotonyl-CoA hydratase and polyhydroxyalkanoate synthetase.

In yet another preferred embodiment, at least one, preferably all enzymes of one, two or three groups of groups (i) to (iv) as defined in the main embodiment and the second aspect of the invention are down-regulated or knocked out. In a particular preferred embodiment, the competing pathway is a pathway which synthesizes glutamate from 2-ketoglutarate or a 2-ketoglutarate degrading pathway and/or wherein the competing enzyme is glutamate synthase.

In a further preferred embodiment, the host cell produces 2-ketoglutarate. As stated above, the production of 2-ketoglutarate in the course of carbohydrate catabolism is ubiquitous. For the sake of completeness, it is noted that the production of 2-ketoglutarate can be assayed by growing the host cell in defined minimal media with no glutamate and no glutamine being present. If the host cell is capable of growing, it follows that the host cell provides 2-ketoglutarate.

In a further preferred embodiment, the host cell shows natural and/or enhanced recombinant activity of 3-hydroxypropionate/4-hydroxybutyrate cycle or parts thereof. The 3-hydroxypropionate/4-hydroxybutyrate cycle is shown in Figure 1 of DE10 2007 059 248. This document is briefly discussed in the background section herein above. 3-hydroxypropionate/4-hydroxybutyrate cycle activity in addition to the activity of the invented pathways can enhance productivity.

The present invention furthermore provides a method of producing a compound selected from the group of crotonic acid, crotonyl-CoA, butyryl-CoA, butyric acid, butyraldehyde and butanol, said method comprising culturing a host cell to produce the said compound, wherein said host cell uses glycerol, polysaccharides, oligosaccharides, sugar(s), alcohols, polyalcohols, fatty acids and/or lipid(s) as carbon source(s), and wherein said host cell is selected from (a) the recombinant or engineered host cell according to the invention; (b) a host cell with elevated expression or activity of at least one enzyme as defined in the embodiment relating to the host cells herein above as compared to the parent host cell it is derived from; and (c) a host cell engineered with (a) nucleic acid(s) encoding at least one enzyme as defined in the embodiment relating to the host cells herein above. A preferred enzyme according to (b) or (c) is 4-hydroxybutyryl-CoA dehydratase. Preferably, the host cell according to (b) and (c) has an expression or activity of the enzymes required by the host cell of the invention which provides for a production of crotonyl-CoA, when provided with said carbon source(s), of at least 100 mg/L culture.

This embodiment provides a method which, in one option utilizes the host cell according to the invention. Alternatively, options (b) or (c) according this embodiment may be used as cells, provided that the cell is selected such that the absence of recombinant means to ensure elevated enzyme expression or activity is complemented by a naturally occurring expression or activity at sufficient levels. Since the pathway of the invention does not occur in nature, options (b) and (c) require elevated activity/expression of and/or the cell being engineered with (a) nucleic acid(s) encoding at least one of the recited enzymes.

A particularly preferred product of the above described method of producing according to the invention is crotonic acid. Furthermore, a particularly preferred carbon source is glycerol.

Preferred sugars are hexoses and pentoses.

The host cell can be cultured and fermented by known methods. Bacteria can, for example, be multiplied in TB or LB medium at a temperature of 20 to 40°C, preferably 25 to 35°C, and more preferred at 30°C, and a pH of 6 to 9. Preferred pH-values are 7 and 8. Suitable culture conditions are described in detail for example by T. Maniatis, E. F. Fritsch and J. Sambrook, Molecular Cloning: A Laboratory Manual, Cold Spring Harbor Laboratory, Cold Spring Harbor, N.Y. (1989). Appropriate culture mediums and conditions for the above-described host cells are well known in the art.

Preferred enzymes according to aspects (b) and (c) of this embodiment are at least one enzyme selected from crotonate CoA-transferase, 4-hydroxybutyryl CoA-dehydratase, 4-hydroxybutyrate CoA-transferase, 4-hydroxybutyrate hydrogenase and 2-ketoacid decarboxylase.

In a preferred embodiment, said host cell is cultured under anaerob conditions.

In a further preferred embodiment, said host cell is grown in a biofermenter.

In a further aspect, the present invention relates to the use of a host cell for the production of a compound selected from the group of crotonic acid, crotonyl-CoA, butyryl-CoA, butyric acid, butyraldehyde and butanol, wherein said host cell uses glycerol, polysaccharides, oligosaccharides, sugar(s), alcohols, polyalcohols, fatty acids and/or lipid(s) as carbon source(s), and wherein said host cell is selected from (a) the recombinant or engineered host cell according to the invention; (b) a host cell with elevated expression or activity of at least one enzyme as defined in accordance with the host cell of the invention as compared to the parent host cell it is derived from; and (c) a host cell engineered with (a) nucleic acid(s) encoding at least one enzyme as defined in accordance with the host cell of the invention.

This use according to the invention essentially corresponds to the above disclosed method of producing a compound selected from crotonic acid, crotonyl-CoA, butyryl-CoA, butyric acid, butyraldehyde and butanol. Any considerations relating to this method apply *mutatis mutandis* to the use according to the present invention.

The present invention furthermore relates to the use of a recombinant or engineered host cell as disclosed above for the production of fuel. The term "fuel" in the context of the present invention refers to bio-fuel noting that the fuel is not obtained from fossil resources but instead via biotechnological production in host cells. Preferable bio-fuels that can be derived from the invented pathway are alcohols such as butanol or esters incorporating above mentioned acids or alcohols. The acid component(s) of said esters are preferably selected from the presented pathway, such as 2-ketoglutarate, 4-hydroxybutyrate, crotonate, butyrate, or common fermentation products such as lactate, acetate, or fatty acids such as laureate.

The present invention furthermore provides a kit containing (a) nucleic acid(s) encoding (a) 4-hydroxybutyryl-CoA dehydratase; and (b) (ba) crotonate CoA-transferase; and/or (bb) at least one, preferably all of the enzymes of at least one of the following groups (i) to (iv): (i) 2-ketoacid decarboxylase; (ii) 2-ketoglutarate dehydrogenase, succinyl-CoA synthetase and succinic semialdehyde dehydrogenase; (iii) 2-ketoglutarate dehydrogenase and succinyl-CoA reductase; and (iv) glutamate dehydrogenase, glutamate decarboxylase and γ-amino-N-butyrate transaminase.

The kit according to the invention provides for one or more nucleic acids encoding at least two enzymes according to the invention. The kit may furthermore comprise a manual containing instructions for preparing an engineered host cell according to the invention. Moreover, the manual may comprise instructions for performing the above defined methods according to the invention.

In a preferred embodiment, the kit according to the invention further comprises nucleic acid(s) encoding at least one, preferably all of the enzymes of at least one of the following groups (a) to (c): (a) 4-hydroxybutyrate dehydrogenase; (b) butyryl-CoA dehydrogenase, butyraldehyde dehydrogenase and butanol dehydrogenase; and (c) butyryl-CoA dehydrogenase and butyrate CoA-transferase.

In a preferred embodiment, said nucleic acid or each of said nucleic acids is/are comprised in a vector.

In preferred embodiments according to the invention, the kits, to the extent they comprise nucleic acids, comprise only the specifically recited nucleic acids.

The figures illustrate the invention:
**Figure 1****:**
   Scheme of the pathway according to the invention which provides 2-ketoglutarate based crotonate production. At the top of the figure it is indicated in grey how glucose is led into the pathway.
**Figure 2****:**
   Figure 2 shows a different entry into the pathway according to the invention wherein homo serine is used as starting material and converted into 4-hydroxybutyrate. The final product is crotonate as in Figure 1.
**Figure 3****:**
   Figure 3 provides an overview of preferred downstream processing pathways. More specifically, it is indicated how crotonyl-CoA is converted into crotonic acid or the potential alternative products butyryl-CoA, butyric acid, butyraldehyde and butanol.

The examples illustrate the invention:
**Example 1:**
Synthesis of crotonic acid in an engineered *Corynebacterium glutamicum* strain

The strain C. *glutamicum* ATCC13032 can utilize various carbon sources and is a natural producer of glutamate. Since glutamate is derived from 2-ketoglutarate, this strain has an increased flux to 2-ketoglutarate. To increase availability of the intermediate 2-ketoglutarate, the host's expression of the gene encoding glutamate dehydrogenase is reduced by methods known to a person skilled in the art, like mutation of the gene or its promoter. The resulting accumulation of 2-ketoglutarate increases the 4-hydroxybutyrate pathway productivity.

To engineer the 4-hydroxybutyrate pathway in such a C. *glutamicum* mutant, DNA containing the genes encoding enzymes with 2-ketoglutarate decarboxylase, 4-hydroxybutyrate dehydrogenase, 4-hydroxybutyrate CoA-transferase, 4-hydroxybutyryl-CoA dehydratase, and crotonate CoA-transferase functionality is cloned into a C. *glutamicum* suitable expression vector, like pEC-XT99A, via standard procedures.

Preferably, each gene is controlled by a separate promoter, preferably constitutive, and cloned together into one vector. Alternatively, the expression cassettes consisting of a gene and its promoter can be integrated into the C. *glutamicum* chromosome by standard methods known to a person skilled in the art such as targeted integration into specific sites via cloning into and use of an integrative vector with homologous flanking regions.

The engineered strain is anaerobically cultured in a fermenter at 30°C, with glucose as carbon source. Alternatively, growth can be achieved aerobically until a sufficient cell mass is acquired, then a switch to anaerob conditions suitable for product biosynthesis is made. The product crotonic acid accumulates in the culture and can be isolated by standard methods.

## Claims

1. A recombinant host cell with elevated expression or activity of the following enzymes as compared to the parent host cell it is derived from:
(a) 4-hydroxybutyryl-CoA dehydratase; and
(b) (ba) crotonate CoA-transferase; and/or
(bb) at least one, preferably all of the enzymes of at least one of the
following groups (i) to (iv):
(i) 2-ketoacid decarboxylase;
(ii) 2-ketoglutarate dehydrogenase, succinyl-CoA synthetase and succinic semialdehyde dehydrogenase;
(iii) 2-ketoglutarate dehydrogenase and succinyl-CoA reductase; and
(iv) glutamate dehydrogenase, glutamate decarboxylase and γ-amino-N-butyrate transaminase.

2. A host cell engineered with (a) nucleic acid(s) encoding the following enzymes:
(a) 4-hydroxybutyryl-CoA dehydratase; and
(b) (ba) crotonate CoA-transferase; and/or
(bb) at least one, preferably all of the enzymes of at least one of the
following groups (i) to (iv):
(i) 2-ketoacid decarboxylase;
(ii) 2-ketoglutarate dehydrogenase, succinyl-CoA synthetase and succinic semialdehyde dehydrogenase;
(iii) 2-ketoglutarate dehydrogenase and succinyl-CoA reductase; and
(iv) glutamate dehydrogenase, glutamate decarboxylase and γ-amino-N-butyrate transaminase.

3. The recombinant host cell according to claim 1, furthermore having an elevated expression or activity compared to the parent host cell it is derived from of 4-hydroxybutyrate dehydrogenase, 4-hydroxybutyrate CoA-transferase and/or 4-hydroxybutyryl-CoA synthetase or the engineered host cell of claim 2, further being transformed with (a) nucleic acid(s) encoding 4-hydroxybutyrate dehydrogenase, 4-hydroxybutyrate CoA-transferase and/or 4-hydroxybutyryl-CoA synthetase.

4. The recombinant host cell according to claim 1 or 3, further having an elevated expression or activity compared to the parent host cell it is derived from of at least one of the enzymes selected from the following group, or the engineered host cell according to claims 2 and 3, further being transformed with (a) nucleic acid(s) encoding at least one enzyme of the following group, said group consisting of enzymes with 4-hydroxybut-2-enoate ammonia lyase and 4-hydroxybut-2-enoate reductase activity.

5. The recombinant or engineered host cell according to any one of claims 1 to 4, wherein in said recombinant host cell the expression or activity of at least one, preferably all of the enzymes of at least one of the following groups (a) and (b):
(a) butyryl-CoA dehydrogenase, butyraldehyde dehydrogenase, and butanol dehydrogenase; and
(b) butyryl-CoA dehydrogenase and butyrate CoA-transferase is elevated or said engineered host cell is transformed with (a) nucleic acid(s) encoding at least one, preferably all of the enzymes of at least one of said groups.

6. The recombinant host cell according to any one of claims 1 or 3 to 5, wherein said enzyme(s) is/are expressed from (a) nucleic acid(s) introduced into said host cell.

7. The engineered host cell according to any one of claims 2 to 6, wherein at least one nucleic acid comprises at least one nucleic acid selected from SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, 55, and 57 or at least one nucleic acid encoding an enzyme with the same activity and having at least 40% sequence identity to the amino acid sequence of SEQ ID NOs: 2, 4, 6, 8, 10, 12, 14, 16, 18, 20, 22, 24, 26, 28, 30, 32, 34, 36, 40, 42, 44, 46, 48, 50, 52, 54, 56, and 58.

8. The recombinant or engineered host cell according to any preceding claims, wherein the host cell is selected from a microorganism, a plant cell and an animal cell, preferably from the kingdom of bacteria, archea, yeast and fungi, more preferably from the genus of *Clostridium* and *Escherichia, Pseudomonas, Bacillus, Saccharomyces, Pichia, Clostridium, Corynebacterium* and *Aspergillus,* yet more preferably E. *coli.*

9. The recombinant or engineered host cell according to any one of claims 1 to 8, wherein the host cell produces 2-ketoglutarate.

10. The recombinant host or host cell according to any one of claims 1 to 9, wherein the host cell shows natural and/or enhanced recombinant activity of 3-hydroxypropionate/4-hydroxybutyrate cycle or parts thereof.

11. A method of producing a compound selected from the group of crotonic acid, crotonyl-CoA, butyryl-CoA, butyric acid, butyraldehyde and butanol, said method comprising culturing a host cell to produce the said compound, wherein said host cell uses glycerol, polysaccharides, oligosaccharides, sugar(s), alcohols, polyalcohols, glycerol, fatty acids, proteins, amino acids and/or lipid(s) as carbon source(s), and wherein said host cell is selected from
(a) the recombinant or engineered host cell according to any one of claims 1 to 10;
(b) a host cell with elevated expression or activity of at least one enzyme as defined in any one of claims 1 to 5 as compared to the parent host cell it is derived from; and
(c) a host cell engineered with (a) nucleic acid(s) encoding at least one enzyme as defined in any one of claims 1 to 5.

12. A method according to claim 11, wherein said host cell is cultured under anaerob conditions.

13. Use of a host cell for the production of a compound selected from the group of crotonic acid, crotonyl-CoA, butyryl-CoA, butyric acid, butyraldehyde and butanol, wherein said host cell uses glycerol, polysaccharides, oligosaccharides, sugar(s), alcohols, polyalcohols, fatty acids, proteins, amino acids and/or lipid(s) as carbon source(s), and wherein said host cell is selected from
(a) the recombinant or engineered host cell according to any one of claims 1 to 10;
(b) a host cell with elevated expression or activity of at least one enzyme as defined in any one of claims 1 to 5 as compared to the parent host cell it is derived from; and
(c) a host cell engineered with (a) nucleic acid(s) encoding at least one enzyme as defined in any one of claims 1 to 5.

14. Use of a recombinant or engineered host cell according to any of claims 1 to 10 for the production of fuel.

15. A kit containing (a) nucleic acid(s) encoding
(a) 4-hydroxybutyryl-CoA dehydratase; and
(b) (ba) crotonate CoA-transferase; and/or
(bb) at least one, preferably all of the enzymes of at least one of the
following groups (i) to (iv):
(i) 2-ketoacid decarboxylase;
(ii) 2-ketoglutarate dehydrogenase, succinyl-CoA synthetase and succinic semialdehyde dehydrogenase;
(iii) 2-ketoglutarate dehydrogenase and succinyl-CoA reductase; and
(iv) glutamate dehydrogenase, glutamate decarboxylase and γ-amino-N-butyrate transaminase.
